(19) Europäisches Patentamt
European Patent Office
Office européen des brevets

(11) EP 2 436 364 B1

(12) **EUROPÄISCHE PATENTSCHRIFT**

(45) Veröffentlichungstag und Bekanntmachung des Hinweises auf die Patenterteilung:
**31.05.2017 Patentblatt 2017/22**

(51) Int Cl.:
***A61K 6/00*** *(2006.01)*

(21) Anmeldenummer: **11183342.2**

(22) Anmeldetag: **29.09.2011**

(54) **Lackzusammensetzung umfassend ein Monomer mit einem polyalicyclischen Strukturelement**

Varnish compound comprising a monomer with a polyalicyclic structure element

Composition de laque comprenant un monomère doté d'un élément structurel polyalicyclique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2010 DE 102010041789**

(43) Veröffentlichungstag der Anmeldung:
**04.04.2012 Patentblatt 2012/14**

(73) Patentinhaber: **VOCO GmbH**
**27472 Cuxhaven (DE)**

(72) Erfinder:
- **Blömker, Dr. Tobias**
  **27472 Cuxhaven (DE)**
- **Stepputtis, Dr. Manfred**
  **27449 Kutenholz (DE)**
- **Maletz, Dr. Reinhard**
  **27472 Cuxhaven (DE)**
- **Plaumann, Manfred Thomas**
  **27474 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**EP-A2- 0 023 685**
**EP-A2- 0 101 807**
**WO-A1-00/08108**
**JP-A- H04 360 809**
**US-A- 3 681 298**
**US-A- 4 744 827**
**US-A1- 2004 039 078**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 436 364 B1

## Beschreibung

Die vorliegende Erfindung ist in den Ansprüchen definiert. Sie betrifft eine, vorzugsweise transparente, Lackzusammensetzung zur Anwendung in einem therapeutischen Verfahren als Dentalmaterial, die als Schutz- und Glanzlack zur Oberflächenbeschichtung verwendbar ist. Offenbart werden außerdem neue polymerisierbare Monomere umfassend mindestens ein polyalicyclisches Strukturelement und bestimmte ethylenische Strukturelemente, die für den Einsatz in einer erfindungsgemäßen dentalen Lackzusammensetzung besonders geeignet sind, sowie die Verwendung der neuen Monomere in einer Lackzusammensetzung.

Kronen- und Brückenprovisorien aus Kunststoff sind ein wichtiger Bestandteil der prothetischen Behandlung. Eine wesentliche Anforderung an diese Materialien ist die Gewährleistung einer glatten Oberflächenbeschaffenheit des Provisoriums, da raue Oberflächen intraorale Plaqueanhaftungen begünstigen. Hat sich erst einmal ein Bakterienfilm gebildet, so kann dies schnell zu entzündlichen Reaktionen des Parodonts und der Gingiva führen. Eine glatte Oberfläche hilft auch ästhetisch problematischen Verfärbungen vorzubeugen, wie sie beispielsweise durch Kaffee, Tee, Wein oder Zigaretten hervorgerufen werden. Darüber hinaus führt die Verwendung eines Zahnlacks zu einer glänzenden Oberfläche auf andernfalls matt erscheinenden Provisorien.

Im Allgemeinen wird eine Oberflächenglättung von Provisorien entweder mit Hilfe von Polierern und Polierpasten oder unter Verwendung von ungefüllten oder nur sehr wenig gefüllten Schutzlacksystemen durchgeführt. In vielen Untersuchungen konnte gezeigt werden, dass lackierte Provisorien eine geringere Plaquebesiedelung aufwiesen als polierte Oberflächen. Dies liegt daran, dass bei Verwendung gängiger Polier- und Schleifkörper bei allen Produkten mehr oder weniger deutliche Werkspuren auftreten, die sich mit mechanischen Poliermitteln nicht vermeiden lassen. Aus diesem Grund wird der Einsatz von Schutzlacken auf diesen Substraten empfohlen.

Es muss bei Anwendung eines Schutz- und Glanzlacks zum Beschichten von Prothesen- und Provisorien darauf geachtet werden, dass das ausgehärtete System nach einer gewissen Verweilzeit auf der Prothese oder dem Provisorium nicht eine rauere Oberfläche aufweist als direkt nach Applikation des Lacks. Ein solcher Umstand kann dann auftreten, wenn sich der Lack vom Kunststoff teilweise löst und nur noch sogenannte "Inseln" hinterlässt mit einem stufigen Übergang zum Restkunststoff. An diesen Stellen können sich dann Keimzellen bakterieller Besiedelung bilden.

Schutz- und Glanzlacke sind aus der Literatur bekannt.

JP 4-29910 A beschreibt die Zusammensetzung eines dentalen farblosen und transparenten Beschichtungsmaterials, das eine ausgezeichnete Abriebfestigkeit aufweisen soll. Der Lack enthält einen polyfunktionellen Vernetzer, der durch Veresterung von mehr als drei Hydroxylgruppen von Pentaerythritol oder Dipentaerythritol mit Acrylsäure herstellbar ist (insbesondere Pentaerythritolhexaacrylat), eine flüchtige Methacrylatverbindung (insbesondere Methylmethacrylat) und ein Acylphosphinoxid als Polymerisationskatalysator.

US 7,081,485 B2 offenbart eine lichthärtbare Zusammensetzung, verwendbar als dentales Beschichtungsmaterial, umfassend eine Multiacrylatverbindung und einen Photoinitiator (Acylphosphinoxid), wobei die Multiacrylatverbindung wenigstens fünf Acrylatfunktionalitäten pro Molekül aufweist und die Zusammensetzung kein Methylmethacrylat enthält und wobei der Photoinitiator in einer Menge von wenigstens 6 Gew.-% in der Zusammensetzung vorhanden ist. Der dort bevorzugte Photoinitiator ist 2,4,6-Trimethylbenzoyldiphenylphosphinoxid. Der Lack soll schmierschichtfrei aushärten.

US 2005/0288387 A1 betrifft ebenfalls dentale Beschichtungszusammensetzungen, die schmierschichtfrei aushärten sollen. Diese Zusammensetzungen sollen geruchsarm lagerbar sein und umfassen eine Multiacrylatverbindung, einen Photoinitiator (Acylphosphinoxid) und einen Alkohol, wobei bevorzugt Ethanol eingesetzt wird.

US 2004/0034119 A1 offenbart ebenfalls ein Dentalmaterial, das schmierschichtfrei aushärten soll. Hierbei wird zunächst ein Lack am Zahn appliziert. In einem zweiten Schritt wird eine Abdeckschicht, beispielsweise eine geschlossene, kontinuierliche Schicht eines ätherischen Öls auf die Oberfläche des Lacks aufgetragen. Der Lack wird so von der umgebenden Atmosphäre abgeschirmt und kann dann schmierschichtfrei polymerisiert werden. Vorteilhaft an dieser Methode soll der Umstand sein, dass die Abdeckschicht je nach Auswahl seiner Zusammensetzung einen guten Geschmack und/oder einen guten Geruch in die Behandlung bringt. Optional kann die Abdeckschicht nach Polymerisation entfernt werden.

US 2006/0111465 A1 beansprucht dentale polymerisierbare Glasuren, die eine polymerisierbare, ethylenisch ungesättigte Harzzusammensetzung enthält sowie ein Härtungsmittel und einen Füllstoff, der im Wesentlichen ein polyedrisches oligomeres Silsesquioxan darstellt.

EP 1 532 958 B1 zielt auf ein dentales Beschichtungsmaterial, das nanoskalige Partikel mit mittleren Teilchengrößen von 1 bis 100 nm, die oberflächenmodifiziert sind, mindestens ein polyfunktionelles Methacrylatmonomer, das vier oder mehr Methacryloylgruppen in einem Molekül aufweist und einen Photopolymerisator enthält.

US 2007/0010597 A1 offenbart laserhärtbare Zusammensetzungen zum Schutz des Zahnhartgewebes. Die Zusammensetzungen sind einkomponentig und enthalten härtbare Monomere mit wenigstens zwei polymerisierbaren Doppelbindungen im Molekül sowie Benzoylperoxid als Initiator.

DE 10 2007 048 925 A1 beschreibt Beschichtungs- und Lackzusammensetzungen, die u.a. für Prothesen,

Unterfütterungsmaterialien oder Implantate eingesetzt werden können. Die dort beschriebenen Zusammensetzungen basieren auf Polysiloxanen.

Ein weiterer dentaler Lack wird in DE 10 2008 010 464 A1 offenbart. Für den Fall, dass, wie oben geschildert, die Lackschichten beispielsweise in abrasiv beanspruchten Bereichen der Bahnoberfläche im Laufe der Zeit durchlässig werden und partiell lackfreie, ungeschützte Bereiche der Zahnoberfläche freigelegt werden, so ist dort eine verstärkte Ausbildung von bakteriellen Belägen wahrscheinlich. Aus diesem Grund wurden Zahnlacke vorgeschlagen, die eine Anhaftung von Belägen auf dem Lack verhindern oder erschweren sollen. Dies soll gemäß DE 10 2008 010 464 A1 dadurch erreicht werden, indem ein Lack verwendet wird, der über Urethangruppen in den Dentalwerkstoff eingebaute perfluorierte Reste enthält. Die durch Umsetzung von Perfluoralkoholen mit Isocyanatgruppen in die Lackformulierung eingebauten perfluorierten Alkylgruppen dienen der Hydrophobierung der Oberfläche des ausgehärteten Systems. Derartige hydrophobe Oberflächen sollen die Anlagerung von Belägen erschweren oder verhindern.

DE 10 2009 011 537 A1 betrifft Dentaloberflächenbeschichtungsmaterialien, die nach der Polymerisation zu weniger Verfärbungen führen. Das Lacksystem enthält 2,4,6-Trimethylbenzoyldiphenylphosphinoxid als Photoinitiator sowie durch Ethylenoxideinheiten aufgebaute Dimethacrylatverbindungen, wobei die Anzahl der Ethylenoxidstrukturelemente zwischen 9 und 50 liegt. Weiterhin kann der Lack ein polyfunktionelles Monomer wie Dipentaerythrithexaacrylat enthalten.

In DE 10 2009 011 536 A1 wird berichtet, dass die Verwendung einer (Bis)acylphosphinoxidverbindung wie 2,4,6-Trimethylbenzoyldiphenylphosphinoxid in Kombination mit einer Diketonverbindung wie Campherchinon in Abwesenheit eines Amins in einem lichthärtbaren Monomersystem verschiedene Effekte bewirkt, z.B. die Verbesserung in der Härtbarkeit in einem breiten Wellenlängenbereich, Abnahme der Änderung in einem Farbton vor und nach dem Härten sowie die Verbesserung in der Dünnschichtoberflächenhärtbarkeit. Als bevorzugte organische Matrixmonomere werden Dipentaerythrithexaacrylat sowie durch Alkylenoxideinheiten aufgebaute Dimethacrylatverbindungen genannt, wobei die Anzahl der Alkylenoxidstrukturelemente zwischen 5 und 50 liegt.

DE 10 2008 039 129 A1 betrifft transparente, kratzfeste Beschichtungen, die auch für einen Einsatz im Dentalbereich vorgesehen sind. Das Beschichtungsmaterial umfasst eine siloxanhaltige Matrix sowie darin dispergierte, funktionalisierte einfach- oder mehrfachwandige Kohlenstoffnanoröhrchen,

US 6,617,413 B1 beschreibt durch Addition an Isocyanatgruppen als auch durch strahlungsinduzierte Addition an aktivierte C-C-Doppelbindungen härtbare Beschichtungsmittel. Die dort beschriebenen Verbindungen können unter anderem in Dentalmassen verwendet werden.

In der EP 1 307 173 B1 wird ein Fluoridlack und Fissurenversiegelungsmaterial auf Silikonbasis beschrieben.

Eine temporäre, transparente, dentale Lackzusammensetzung zur Beschichtung von Zahnoberflächen beschreibt die US 2006/0063853 A1. Hier wird die Transparenz durch den Zusatz von Glashohlkugeln zur Zusammensetzung bewirkt, wobei die Glashohlkugeln jedes ankommende Licht zurück zur Lichtquelle umleiten. Dieser Effekt wird durch eine im Inneren der Kugeln stattfindende Totalreflexion bewirkt, die immer dann erfolgt, wenn Licht in eine Glashohlkugel eintritt. Dieses Phänomen wird insbesondere bei Straßenmarkierungen genutzt. Um diesen Effekt hervorbringen zu können, muss der Brechungsindex des Bindemittels geringer sein als der der Glashohlkugeln.

JPH 04360809 (Tokuyama Soda CO LTD) offenbart ein "photo-setting surface smoothing agent" (englische Übersetzung des Titels) zur Verwendung im dentalen Bereich. Offenbarte Zusammensetzungen umfassen Monomere mit einem Tricyclo[3.3.1.1]decan-Rest.

EP 0023685 A2 (Bayer AG) offenbart Dentalzusammensetzungen, die Monomere mit einem Tricyclo[5.2.1.0]decan-Rest enthalten.

Als Standardmaterialien bei der Formulierung von Schutz- und Glanzlacken werden Kunststoffe auf Di(meth)acrylatbasis eingesetzt, die entweder nicht oder nur wenig gefüllt sind. Aufgrund des Fehlens von Füllstoffen oder ihrer nur ganz geringen Füllstoffbeladung weisen Zahnlacke im Vergleich zu Flow- und Füllungskompositen noch niedrigere Viskositäten auf. Sie sind in der Regel dünn- bis mitteldünnfließend und werden mit einem Mikroapplikationsinstrument, beispielsweise mit einem Bürstchen oder Pinselchen aus einer Flasche entnommen und dann direkt appliziert. Alternativ kann das Lacksystem auch aus einer Spritze mittels Kanüle angewendet werden.

Mitteldünnfließende oder auch zähfließende Materialien können bei Applikation besser kontrolliert werden, während dünnfließende Systeme einen dünneren Auftrag erlauben und auch in sehr kleine Hohlräume einfließen. Der Auftrag in nur einer dünnen Schicht ist deshalb wichtig, damit die Okklusion mit dem antagonistischen Zahn nicht nachhaltig gestört wird. Darüberhinaus erlaubt ein umso dünnerer Auftrag des transparenten Lacks eine umso genauere Wiedergabe der Farbe des lackierten Zahns und somit der natürlichen Zahnsituation.

Allerdings sind die Lacksysteme durch den reduzierten Füllstoffanteil oder ganz fehlenden Füllstoff weniger abrasions- und biegefest als mittel- und hochgefüllte Materialien.

Werden dentale, radikalisch härtbare Zusammensetzungen polymerisiert, so erscheint die obere Schicht des Polymerisats oft klebrig. Die Oberflächen sind nicht ganz ausgehärtet. Dieses Phänomen wird durch den Sauerstoff der Luft, der ein Triplett-Diradikal ist, ausgelöst, indem Sauerstoffmoleküle die radikalische Polymerisation aufgrund ihres radikalischen Charakters stören und inhibieren. Eine solche, feucht erscheinende Schicht muß mit Alkohol entfernt

werden. Gelingt es durch Auswahl der Monomeren und Auswahl und Einstellung des Initiatorsystems die Ausbildung einer Sauerstoffinhibitionsschicht zu verhindern, dann zeigen diese Lacksysteme bessere Glanzeigenschaften und größere Beständigkeit gegenüber den Einflüssen verfärbender Agentien auf.

Niedrig gefüllte oder ungefüllte dentale Lacksysteme sind für eine Anwendung als "flüssige Politurmaterialien", "Dentalglasur", "Beschichtungsmaterialien", "Schutzlacke", "Glanzlacke", etc. für eine Vielzahl intra- und extraoralen Situationen sowie für den Gebrauch im Zahnlabor geeignet. Besonders geeignet ist ihr klinischer Einsatz für

- Beschichtungen der Randbereiche von alten und neuen, direkten und indirekten Restaurationen aus Kunststoffen und kunststoffverstärkten Glasionomerzementen (GIZ),
- Restaurationen und Provisorien zur Verbesserung der Ästhetik und Vereinfachung von Ausarbeitung und Politur,
- die Therapie von Defekten der natürlichen Zahnhartsubstanz.

Die Anforderungen an Schutz- und Glanzlackmaterialien sind somit äußerst vielfältig. Zunächst sollten transparente Zusammensetzungen für lange Zeit auf der Zahnoberfläche bleiben, um ihren Einsatzzweck zu erfüllen. Hierbei sollten sie den regelmäßigen mechanischen Angriffen auf sie, beispielsweise durch Mundhygienemaßnahmen wie Zähneputzen, standhalten.

Da die Lackmaterialien im Mundraum permanent ausgesprochen intensiven hydrolytischen Angriffen, beispielsweise durch den Speichel, ausgesetzt sind, sollten die Polymerisate eine möglichst geringe Wasseraufnahme aufweisen. Bei der radikalischen Vernetzung von Methacrylat/Acrylatzusammensetzungen entsteht ein dreidimensional verknüpftes Netzwerk. Aufgrund der sehr geringen Größe des Wassermoleküls kann Wasser in die Maschen des Polymerisats eindiffundieren, wo es sich an bestimmten Stellen im Netzwerk anlagert und dort Wasserstoffbrücken, bzw. andere schwach polare Bindungen ausbildet. Je mehr polare Anteile in der Polymermatrix vorhanden sind, umso leichter kann eine weitere Wasseraufnahme erfolgen. Durch die Wasseraufnahme weitet sich das Polymerisat auf und es kommt zu einer Vergrößerung der Intermolekularabstände. Diese hygroskopische Expansion kann dann zu einer strukturellen Reorganisation der Polymerketten führen. Die Wasseraufnahme erfolgt über einen längeren Zeitraum nach der Aushärtung, so dass es bei einer übermäßigen Wasseraufnahme zu einem Expansionsstress und somit zu einem "Überquellen" des Materials kommt. Hierbei können sich Teile des Lacks von der Zahnoberfläche lösen und die oben erwähnten "Inseln" bilden, die dann Ausgangspunkt neuer bakterieller Besiedelungen werden.

Darüberhinaus können Wassermoleküle empfindliche Strukturelemente des Polymerisats, wie beispielsweise Estergruppen, angreifen und hydrolytisch spalten. Dieser Abbau kann zur völligen Disintegration des Netzwerks und somit zum vollständigen Verlust des Produktes führen.

Andererseits ist eine gewisse Polarität der Lacksysteme durchaus wünschenswert, da die Zahnhartsubstanz hydrophil ist und polare Zusammensetzungen eine gute Adaptation und Benetzbarkeit des Materials am Zahnsubstrat gewährleisten.

Primäre Aufgabe der Erfindung war es, eine Lackzusammensetzung zur Anwendung in einem therapeutischen Verfahren als Dentalmaterial, vorzugsweise eine transparente Lackzusammensetzung, zur Verfügung zu stellen, die sich zur Verwendung als Schutz- und Glanzlack eignet und eine geringe Wasseraufnahme sowie sehr gute mechanische Eigenschaften aufweist. Darüberhinaus sollte die Lackzusammensetzung sich vorzugsweise durch eine sehr gute Adaptation, d.h. sehr gutes Anfließverhalten an der entsprechende Oberflächen, insbesondere der Zahnhartstruktur, auszeichnen. Darüberhinaus sollte die, vorzugsweise dentale, Lackzusammensetzung vorzugsweise schmierschichtfrei aushärten.

Die primäre Aufgabe wird erfindungsgemäß gelöst durch eine, vorzugsweise transparente, Lackzusammensetzung, gemäß Patentanspruch 1.

Unter (Meth)acryl ist im Rahmen des vorliegenden Textes sowohl Acryl als auch Methacryl zu verstehen.

Sämtliche nachfolgenden Ausführungen betreffend Verbindungen der Struktur $Q(YZ_e)_b$ (wobei jedes Y unabhängig von etwaigen weiteren Strukturelementen Y gewählt ist oder entfällt) und die im Zusammenhang mit diesen Verbindungen angegebenen bevorzugten bzw. besonders bevorzugten Ausgestaltungen der vorliegenden Erfindung gelten entsprechend für die Verbindungen der Struktur $Q(Y_xZ_e)_b$ (wobei jeder Index x unabhängig von etwaigen weiteren Indizes x 0 oder 1 bedeutet), und umgekehrt.

Eine erfindungsgemäß einzusetzende Verbindung der Struktur $Q(Y_xZ_e)_b$ umfasst ein polyalicyclisches Strukturelement Q, das von einem entsprechenden polyalicyclischen Kohlenwasserstoff abgeleitet ist. Dies bedeutet im Rahmen des vorliegenden Textes, dass b Wasserstoffatome des Kohlenwasserstoffs durch Substituenten $Y_xZ_e$ ersetzt sind (wie oben beschrieben), und optional ein, zwei oder mehr der nicht durch Substituenten $Y_xZ_e$ substituierten Wasserstoffatome durch Alkylgruppen, Alkoxygruppen, Halogenatome oder Trifluormethylgruppen substituiert sind. Das polyalicyclische Strukturelement Q wird konstituiert durch Kohlenstoff-Ring-Atome. Kohlenstoffatome außerhalb der Ringe sind Bestandteil von Substituenten.

Selbstverständlich gilt für Substanzen, die aufgrund ihrer Struktur gleichzeitig unter die Definition verschiedener Komponenten einer erfindungsgemäßen Lackzusammensetzung fallen, dass diese Substanzen für die Zwecke quantitativer Betrachtungen jeweils sämtlichen dieser Komponenten zugeordnet werden.

Bei dem "polyalicyclischen" Strukturelement Q handelt es sich um einen tricyclischen Kohlenwasserstoffrest, wie oben definiert. Die Bezeichnung "tricyclisch" entspricht dabei der IUPAC-Nomenklatur.

Es bedeutet jedes Y ein Strukturelement, welches in der Struktur $Q(Y_xZ_e)_b$ mit x = 1 das polyalicyclische Strukturelement Q mit e Strukturelementen Z verbindet, wobei jedes Y unabhängig von etwaigen weiteren Strukturelementen Y gewählt ist.

Es wurde gefunden, dass die erfindungsgemäßen Lackzusammensetzungen dentale Schutz- und Glanzlacke sind, die im Vergleich zu Lacken aus dem Stand der Technik ein sehr viel besseres Anfließverhalten zeigen (insbesondere am trockenen Zahnschmelz), sehr viel weniger Wasser aufnehmen und gute mechanische Werte aufweisen (insbesondere Biegefestigkeit und E-Modul).

Die erfindungsgemäße Lackzusammensetzung ist vorzugsweise lichthärtbar.

Die Summe der Zahlenwerte des Index b und des Index e beträgt vorzugsweise 3, 4, 5, 6, 7 oder 8.

Überraschend hat sich in eigenen Untersuchungen gezeigt, dass eine erfindungsgemäße Lackzusammensetzung zur Anwendung in einem therapeutischen Verfahren als Dentalmaterial im ausgehärteten Zustand einen geringen Schrumpf, eine gute Haftung auf verschiedenen Untergründen, eine hohe Hydrolysebeständigkeit, eine geringe Wasseraufnahme, eine hohe mechanische Festigkeit und einen hohen Glanz aufweist. Die genannten Eigenschaften sind insbesondere im Bereich der Dentaltechnik wichtig.

Bevorzugt ist eine erfindungsgemäße Lackzusammensetzung zur Anwendung in einem therapeutischen Verfahren als Dentalmaterial, umfassend oder bestehend aus

(a) 38 bis 96 Gew.-% einer Monomerenkomponente umfassend

(a1) ein, zwei oder mehr Monomere der Struktur $Q(YZ_e)_b$ (wie oben definiert und wobei jedes Y unabhängig von etwaigen weiteren Strukturelementen Y gewählt ist oder entfällt) der Komponente (a1), wobei vorzugsweise Z eine Gruppe $-O-(C=O)-CH=CH_2$, $-O-(C=O)-C(CH_3)=CH_2$, $-(C=O)-CH=CH_2$ oder - $(C=O)-C(CH_3)=CH_2$ bedeutet,

(a2) ein, zwei oder mehr radikalisch polymerisierbare Monomere ausgewählt aus der Gruppe bestehend aus Methylmethacrylat, Ethylmethacrylat, Propylmethacrylat, Butylmethacrylat und Pentylmethacrylat,

(a3) ein, zwei oder mehr weitere radikalisch polymerisierbare Monomere mit drei oder mehr (Meth)acrylatgruppen, vorzugsweise mit drei bis sechs (Meth)acrylatgruppen,
sowie

(a4) optional ein, zwei oder mehr weitere radikalisch polymerisierbare Monomere aus der Gruppe bestehend aus Acrylaten und Methacrylaten, vorzugsweise der Gruppe der Methacrylate,

wobei das Verhältnis der Masse der Komponente (a1) zur der Gesamtmasse der Komponenten (a2), (a3) und (a4) vorzugsweise im Bereich von 4 : 1 bis 1 : 6 liegt,

(b) ein oder mehrere Initiatoren und/oder Katalysatoren,

(c) 0 bis 60 Gew.-% einer Füllstoffkomponente, umfassend einen, zwei oder mehrere Füllstoffe ausgewählt aus der Gruppe bestehend aus

(c1) 0 bis 60 Gew.-% nicht agglomerierte, vorzugsweise Oberflächenmodifizierte, Nanopartikel mit einer mittleren Partikelgröße kleiner 200 nm (vorzugsweise kleiner 100 nm, besonders bevorzugt kleiner 60 nm),

(c2) 0 bis 10 Gew.-% Mikropartikel mit einer mittleren Partikelgröße von 0,4 μm bis 10 μm, und

(c3) 0 bis 15 Gew.-% weitere Füllstoffe, vorzugsweise 0 bis 10 New.-%, bevorzugt 0 bis 5 Gew.-%, weiter bevorzugt 0 bis 2,5 Gew.-% weitere Füllstoffe,

und

(d) gegebenenfalls ein oder mehrere Additive,

wobei die Gewichtsprozentangaben jeweils auf die Gesamtmasse der Lackzusammensetzung bezogen sind.

Vorzugsweise beträgt die Gesamtmenge an Komponente (a1) und Komponente (a2) in einer erfindungsgemäßen Lackzusammensetzung zur Anwendung in einem therapeutischen Verfahren als Dentalmaterial zumindest 25 Gew.-%, bevorzugt 30 Gew.-% oder mehr, bevorzugt 35 Gew.-% oder mehr, jeweils bezogen auf die Gesamtmasse der Lackzusammensetzung.

Vorzugsweise beträgt die Gesamtmenge an Komponenten (a1), (a2) und (a3) in einer erfindungsgemäßen Lackzusammensetzung zur Anwendung in einem therapeutischen Verfahren als Dentalmaterial zumindest 38 Gew.-%, bevorzugt 40 Gew.-% oder mehr.

In bevorzugten Ausgestaltungen liegt die Gesamtmenge an Komponenten (a1), (a2) und (a3) in einer erfindungsgemäßen Lackzusammensetzung zur Anwendung in einem therapeutischen Verfahren als Dentalmaterial im Bereich von 40 - 96 Gew.-%, weiter bevorzugt im Bereich von 50 - 95 Gew.-%, weiter bevorzugt im Bereich von 60 - 95 Gew.-%, weiter bevorzugt im Bereich von 70 - 95 Gew.-%, jeweils bezogen auf die Gesamtmasse der Lackzusammensetzung.

Im nicht-ausgehärteten Zustand zeichnet sich eine erfindungsgemäße Lackzusammensetzung zur Anwendung in einem therapeutischen Verfahren als Dentalmaterial durch einen geringen Kontaktwinkel auf trockenem Zahnschmelz aus (vorzugsweise von weniger als 50°, bevorzugt weniger als 40°, besonders bevorzugt weniger als 30°, gemessen mit einem Kontaktwinkelmessgerät der Firma Krüss (DSA 100)). Vor Durchführung der Kontaktwinkel-Messung wurde hierzu ein humaner Zahn mit einem Zellstofftuch getrocknet ("trockener Zahnschmelz").

Ferner hat sich gezeigt, dass eine erfindungsgemäße Lackzusammensetzung zur Anwendung in einem therapeutischen Verfahren als Dentalmaterial im ausgehärteten Zustand eine sehr geringe Wasseraufnahme aufweist, und vorzugsweise weniger als 50 $\mu g/mn^3$ beträgt, vorzugsweise weniger als 45 $\mu g/mm^3$ und bevorzugt weniger als 40 $\mu g/mm^3$, besonders bevorzugt weniger als 35 $\mu g/mm^3$. Die Wasseraufnahme wurde dabei analog zu ISO 4049 bestimmt.

Die erfindungsgemäßen Lackzusammensetzungen zur Anwendung in einem therapeutischen Verfahren als Dentalmaterial sind vielseitig auf verschiedenen Substraten einsetzbar und werden vorzugsweise als Kavitäten- und Oberflächenlack im Dentalbereich verwendet. Die Lackzusammensetzungen werden bevorzugt auf Glasionomerfüllungen, provisorischen Kronen und Brücken aus Komposit sowie auf Kompositrestaurationen eingesetzt. Die adhäsiven "Interfaces" zwischen Komposit und Zahnhartsubstanz können durch die Lackzusammensetzungen geschützt werden.

Als Kavitätenlacke können die erfindungsgemäßen Lackzusammensetzungen zur Anwendung in einem therapeutischen Verfahren als Dentalmaterial als Unterfüllung für Zahnfüllmaterialien dienen und dabei die Pulpa vor schädigenden Monomeren abschirmen. Als Oberflächenlack können sie den Zahnschmelz vor äußeren Beeinträchtigungen und gegenüber den in der Mundhöhle und an der Zahnoberfläche vorhandenen Substanzen schützen und ein Auftreten von Karies verhindern.

Bei Anwendung auf Kompositoberflächen bei endgültigen Kompositrestaurationen dient eine erfindungsgemäße Lackzusammensetzung zur Anwendung in einem therapeutischen Verfahren als Dentalmaterial gegen eine vorzeitige Abnutzung der Restauration. Sie verbessert somit die Verschleissfestigkeit des darunter liegenden Füllungsmaterials. Die Lackzusammensetzung bewirkt eine dauerhafte Festigung restaurierter Oberflächen und schützt vor Abrasion und Verfärbung. Aufgrund ihrer äußerst geringen Viskositäten sind die Lackzusammensetzungen befähigt, in marginale Spalten zu fließen, diese aufzufüllen und so zu schließen. Gleiches Verhalten zeigen sie bei Mikrorissen auf restaurativen Oberflächen.

Überdies erfüllen die erfindungsgemäßen Lackzusammensetzungen zur Anwendung in einem therapeutischen Verfahren als Dentalmaterial anspruchsvolle ästhetische Anforderungen. Durch das Auftragen einer sehr dünnen Schicht einer solchen Lackzusammensetzung bildet sich eine lang anhaltende hochglänzende, extrem glatte Oberfläche. Die Lackzusammensetzung verleiht dem Zahn oder der Restauration somit einen natürlichen Glanz durch die Bildung ästhetischer Oberflächen. Durch die feine Oberflächenglätte reduziert sie das Risiko von Farbstoffablagerungen.

Die erfindungsgemäßen Lackzusammensetzungen zur Anwendung in einem therapeutischen Verfahren als Dentalmaterial können auch zur Oberflächenvergütung von provisorischen Kronen und Brücken verwendet werden.

Erfindungsgemäße dentale Lackzusammensetzungen zur Anwendung in einem therapeutischen Verfahren als Dentalmaterial sind vorzugsweise so ausgestaltet, dass sie als Kavitäten- und/oder Oberflächenlack eingesetzt werden können, als Unterfüllung für Zahnfüllmaterialien, zum Schutz des Zahnschmelzes, zur Verhinderung von Karies, zum Schutz einer Restauration (insbesondere gegen vorzeitige Abnutzung), zur Verbesserung der Verschleissfestigkeit eines dentalen Füllungsmaterials, zur Festigung restaurierter Oberflächen, zum Schutz vor Abrasion und/oder Verfärbung eines Zahns oder einer Restauration, zum Schließen marginaler Spalten und/oder von Mikrorissen, zur Glättung von restaurativen Oberflächen, zum Verleihen von natürlichem Glanz auf einem Zahn oder einer Restauration und zum Reduzierung von Farbstoffablagerungen auf einem Zahn oder einer Restauration. Entsprechende Verwendungen einer erfindungsgemäßen Lackzusammensetzung sind bevorzugt.

Bestandteil (a): Monomerenkomponente

Innerhalb einer erfindungsgemäßen Lackzusammensetzung zur Anwendung in einem therapeutischen Verfahren als Dentalmaterial besteht die Funktion der Monomerenkomponente (a) darin, eine Matrix zu bilden. Diese Matrix wird gebildet durch Polymerisation, insbesondere radikalische Polymerisation, von ein, zwei oder mehr Monomeren der Struktur $Q(YZ_e)_b$ (wie oben definiert und wobei jedes Y unabhängig von etwaigen weiteren Strukturelementen Y gewählt ist oder entfällt) der Komponente (a1) zusammen mit einem oder mehreren weiteren Monomeren der Komponenten (a2), (a3) und (a4). Die Monomerenkomponente (a) ist eine Monomerenmischung, die Komponente (a1) und Komponente (a2) sowie Komponente (a3) sowie gegebenenfalls Komponente (a4) umfasst oder aus diesen besteht.

Dabei liegt das Verhältnis der Masse der Komponente (a1) zur der Gesamtmasse der Komponenten (a2), (a3) und (a4) vorzugsweise im Bereich von 4 : 1 bis 1 : 6, vorzugsweise im Bereich von 3: 1 bis 1 : 3.

Die Gesamtmenge der Komponente (a1) beträgt vorzugsweise zumindest 10 Gew.-%, bevorzugt zumindest 15 Gew.-%, jeweils bezogen auf die Gesamtmasse der Lackzusammensetzung.

Die Gesamtmenge der Komponente (a1) liegt vorzugsweise im Bereich von 10 bis 80 Gew.-%, bevorzugt im Bereich von 15 bis 75 Gew.-%, jeweils bezogen auf die Gesamtmasse der Lackzusammensetzung.

Das polyalicyclische Strukturelement Q der Komponente (a1) sorgt dabei für ein sterisch starres und hydrophobes Rückgrat, die Methacrylat-Monomere der Komponenten (a2) sorgen für eine rasche Polymerisationskinetik und die Monomere der Komponenten (a3) und/oder (a4) für eine ausreichende Vernetzung. Letzteres gilt insbesondere auch bezüglich oberflächenmodifizierter Füllstoffe, sofern vorhanden.

Die Kombination von Komponente (a2) und Komponente (a3) erlaubt ein Feineinstellen der mechanischen und rheologischen Eigenschaften einer erfindungsgemäßen Lackzusammensetzung. Insbesondere wird durch die Kombination hochfunktionalisierter Acrylatverbindungen der Komponente (a3) mit den äußerst mobilen monofunktionalisierten Monomeren niedrigen Molekulargewichts der Komponente (a2) durch Diffusionsprozesse eine schnelle Polymerisationskinetik erzeugt und so eine schnellhärtende Lackzusammensetzung geschaffen.

Komponente (a1): ein, zwei oder mehr Monomere der Struktur $Q(YZ_e)_b$ mit mindestens einem polyalicyclischen Strukturelement

Komponente (a1) bilden ein, zwei oder mehr Monomere der oben definierten Struktur $Q(YZ_e)_b$, (wie oben definiert und wobei jedes Y unabhängig von etwaigen weiteren Strukturelementen Y gewählt ist oder entfällt) wobei Z vorzugsweise ein Strukturelement bedeutet, das unabhängig von etwaigen weiteren Strukturelementen Z ausgewählt ist aus der Gruppe bestehend aus $-O-(C=O)-CH=CH_2$, $-O-(C=O)-C(CH_3)=CH_2$, $-(C=O)-CH=CH_2$ oder $-(C=O)-C(CH_3)=CH_2$. Bevorzugt sind Verbindungen der Struktur $Q(YZ_e)_b$ (wie oben definiert und wobei jedes Y unabhängig von etwaigen weiteren Strukturelementen Y gewählt ist oder entfällt), wobei Z ausgewählt ist aus der Gruppe bestehend aus $-O-(C=O)-CH=CH_2$, $-O-(C=O)-C(CH_3)=CH_2$, d.h. solche Verbindungen der Struktur $Q(YZ_e)_b$, (wie oben definiert und wobei jedes Y unabhängig von etwaigen weiteren Strukturelementen Y gewählt ist oder entfällt) die ein, zwei oder mehr Acrylat- und/oder Methacrylatgruppen aufweisen, vorzugsweise zwei oder mehr Acrylat- und/oder Methacrylatgruppen.

Die mit den erfindungsgemäß zu verwendenden Monomeren der Komponente (a1) erhältlichen Polymere und Erzeugnisse weisen eine ausgeprägte Hydrophobie auf, die sich u.a. in einer sehr geringen Wasseraufnahme der Polymere und Erzeugnisse zeigt. Daneben zeichnen sich die unter Verwendung der erfindungsgemäßen bzw. erfindungsgemäß zu verwendenden Monomere der Komponente (a1) erhältlichen Polymere durch eine hohe mechanische Stabilität aus, die sich u.a. in einer hohen Biegefestigkeit der Polymere zeigt. Die erfindungsgemäß zu verwendenden Monomere der Komponente (a1), insbesondere gemäß den besonders bevorzugten Ausgestaltungen und Ausführungsformen, lassen sich zu Polymeren verarbeiten, die im ausgehärteten Zustand sowohl eine geringe Wasseraufnahme als auch eine hohe Biegefestigkeit aufweisen.

Es wurde ferner gefunden, dass die erfindungsgemäß zu verwendenden Monomere der Komponente (a1) anwendbare, vergleichsweise niedrige Viskositäten aufweisen. Diese Monomere der Komponente (a1) sind mit den weiteren Monomeren der Komponente (a2) sowie der Komponente (a3) sowie gegebenenfalls der Komponente (a4) copolymerisierbar, wobei die ausgehärteten Polymere bzw. Formstoffe einen geringen Schrumpf, eine gute Haftung auf verschiedenen Untergründen, eine hohe Hydrolysebeständigkeit, eine geringe Wasseraufnahme, eine hohe mechanische Festigkeit und einen hohen Glanz aufweisen. Die genannten Eigenschaften sind insbesondere im Bereich der Dentaltechnik wichtig.

Insbesondere die bevorzugten und besonders bevorzugten erfindungsgemäß zu verwendenden Verbindungen der Komponente (a1) ermöglichen einen hohen Vernetzungsgrad und sind ferner vorzugsweise radikalisch vernetzbar. Wegen ihrer hochfunktionalisierten Struktur weisen sie eine hohe Vernetzungs- und Polymerisationswahrscheinlichkeit auf.

Bevorzugte erfindungsgemäß einzusetzende Verbindungen sind solche, wobei Q ein polyalicyclisches Strukturelement bedeutet, vorzugsweise ein gesättigtes polyalicyclisches Strukturelement, das ausgewählt ist aus der Gruppe bestehend aus tricyclischen Kohlenwasserstoffresten, wobei vorzugsweise keines der nicht durch Substituenten $YZ_e$ (wie oben definiert und wobei jedes Y unabhängig von etwaigen weiteren Strukturelementen Y gewählt ist oder entfällt) substituierten Wasserstoffatome dieses polyalicyclischen Strukturelements Q substituiert ist.

Sofern eine erfindungsgemäße einzusetzende Verbindung zwei oder mehrere polyalicyclische Strukturelemente umfasst, können diese identisch oder verschieden sein.

Besonders bevorzugt sind erfindungsgemäß einzusetzende Monomere $Q(YZ_e)_b$ (wie oben definiert und wobei jedes Y unabhängig von etwaigen weiteren Strukturelementen Y gewählt ist oder entfällt), deren polyalicyclisches Strukturelement Q sich von einem der folgenden tricyclischen Kohlenwasserstoffe ableitet: Tricyclo[5.2.1.0$^{2,6}$]decan (TCD), Tricyclo[5.2.1.0$^{2,6}$]dec-3-en oder Tricyclo[3.3.1.1$^{3,7}$]decan (Adamantan), d.h. bevorzugt sind erfindungsgemäße Verbindungen, die ein TCD - Gerüst, ein Tricyclo[5.2.1.0$^{2,6}$]dec-3-en - Gerüst oder ein Adamantan-Gerüst aufweisen.

Bei den genannten besonders bevorzugten erfindungsgemäß einzusetzenden Verbindungen, in denen das Strukturelement Q einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet, handelt es sich vorzugsweise um solche mit einem Tricyclo[5.2.1.0$^{2,6}$]decan-Gerüst, einem Tricyclo[5.2.1.0$^{2,6}$]dec-3-en-Gerüst, einem Tricyclo[3.3.1.1$^{3,7}$]decan-Gerüst bzw. einem Bicyclo[2.2.1]heptan-Gerüst, bei dem jeweils keines der nicht durch Substituenten $YZ_e$ (wie oben definiert und wobei jedes Y unabhängig von etwaigen weiteren Strukturelementen Y gewählt ist oder entfällt) substituierten Wasserstoffatome dieses polyalicyclischen Strukturelements Q substituiert ist.

Besonders bevorzugte erfindungsgemäß einzusetzende Verbindungen sind solche, wobei das Strukturelement Q einen Tricyclo[5.2.1.0$^{2,6}$]decan-Rest, einen Tricyclo[5.2.1.0$^{2,6}$]dec-3-en-Rest, einen Tricyclo[3.3.1.1$^{3,7}$]decan-Rest oder einen Bicyclo[2.2.1]heptan-Rest bedeutet, ganz besonders bevorzugt bedeutet das Strukturelement Q einen Tricyclo[5.2.1.0$^{2,6}$]decan-Rest, einen Tricyclo[5.2.1.0$^{2,6}$]dec-3-en-Rest.

Bevorzugt eingesetzt werden Methacrylsäure- oder Acrylsäureester mit einem Tricyclo[5.2.1.0$^{2,6}$]-decan- oder Tricyclo[5.2.1.0$^{2,6}$]-decen-Strukturelement ausgewählt aus der Gruppe bestehend aus

- 8,9-Bis(acryloxymethyl)tricyclo[5.2.1.0$^{2,6}$]decan
- 8,9-Bis(methacryloyloxymethyl)tricyclo[5.2.1.0$^{2,6}$]decan
- 8,9-[Bis(2-vinyloxyethyl)oxymethyl]tricyclo[5.2.1.0$^{2,6}$]dec-3-en
- 8,9-[Bis(2-vinyloxyethyl)oxymethyl]tricyclo[5.2.1.0$^{2,6}$]decan
- 8-Hydroxymethyl-9-(2-vinyloxyethyl)oxymethyl]tricyclo[5.2.1.0$^{2,6}$]dec-3-en
- 9-Hydroxymethyl-8-(2-vinyloxyethyl)oxymethyl]tricyclo[5,2.1.0$^{2,6}$]dec-3-en
- 8,9-Bis(acryloyloxymethyl)tricyclo[5.2.1.0$^{2,6}$]dec-3-en
- 8,9-Bis(methacryloyloxymethyl)tricyclo[5.2.1.0$^{2,6}$]dec-3-en
- Diacrylsäure- oder Dimethacrylsäureester von Verbindungen ausgewählt aus der Gruppe bestehend aus:
- 3,8-Dihydroxymethyl- tricyclo[5.2.1.0$^{2,6}$]decan
- 3,9-Dihydroxymethyltricyclo- [5.2.1.0$^{2,6}$]decan
- 4,8-Dihydroxymethyltricyclo[5.2.1.0$^{2,6}$]decan
- 3,8-Dihydroxytricyclo[5.2.1.0$^{2,6}$]decan
- 3,9-Dihydroxytricyclo- [5.2.1.0$^{2,6}$]decan
- 4,8-Dihydroxytricyclo[5.2.1.0$^{2,6}$]decan
- Methacrylsäure- oder Acrylsäureester von Verbindungen aus der Gruppe bestehend aus:
- Poly(hydroxymethyl-tricyclo[5.2.1.0$^{2,6}$]decanyl-siloxanen

- oxyalkyliertes Bishydroxymethyltricyclo[5.2.1.0$^{2,6}$]decan
- oxyalkyliertes Bishydroxytricyclo[5.2.1.0$^{2,6}$]decan
- Urethan- oder Harnstoffgruppen enthaltende Methacrylsäure- oder Acrylsäureester von Verbindungen ausgewählt aus der Gruppe bestehend aus:
- 3,8-Dihydroxymethyl- tricyclo[5.2.1.0$^{2,6}$]decan
- 4.8-Dihydroxymethyl- tricyclo[5.2.1.0$^{2,6}$]decan
- 3,9-Dihydroxymethyl- tricyclo[5.2.1.0$^{2,6}$]decan
- 4,9-Dihydroxymethyl- tricyclo[5.2.1.0$^{2,6}$]decan

Hierbei kann in den genannten Verbindungen Wasserstoff am Tricyclo[5.2.1.0$^{2,6}$]-decan-oder Tricyclo[5.2.1.0$^{2,6}$]-decen-Rest durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sein.

Viele der vorstehend aufgelisteten radikalisch polymerisierbaren Methacrylsäure- oder Acrylsäureester mit einem TCD-Strukturelement sind aus dem Stand der Technik bekannt.

In eigenen Untersuchungen hat sich gezeigt, dass insbesondere mit den vorstehend oder nachstehend genannten Monomeren $Q(YZ_e)_b$ (wie oben definiert und wobei jedes Y unabhängig von etwaigen weiteren Strukturelementen Y gewählt ist oder entfällt) der Komponente (a1) mit einem Tricyclo[5.2.1.0$^{2,6}$]-decan-Strukturelement der Komponente (a1) Lackzusammensetzungen mit geringem Kontaktwinkel auf trockenem Zahnschmelz (vorzugsweise weniger als 40°, bevorzugt weniger als 30°) und geringer Wasseraufnahme (vorzugsweise weniger als 40 $\mu$g/mm$^3$, bevorzugt weniger als 35 $\mu$g/mm$^3$) erhältlich sind.

Y ist vorzugsweise ein Strukturelement, welches in der Struktur $O(Y_xZ_e)_b$ das polyalicyclische Strukturelement Q mit e Strukturelementen Z verknüpft und ein Strukturelement enthält oder aus diesem besteht, das ausgewählt ist aus der Gruppe bestehend aus

$-CH_2-$ ,

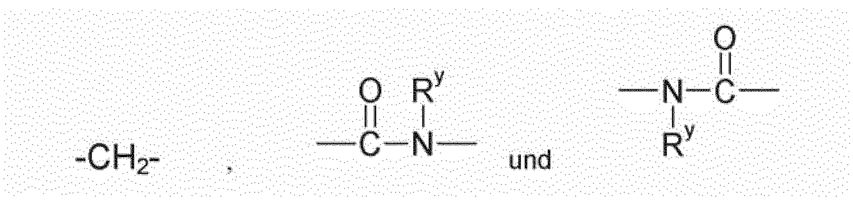

und

wobei $R^y$ einen sonstigen Rest der Verbindung bedeutet und
wobei die jeweils im Formelbild links angeordnete Bindung dem Strukturelement Q und die rechts angeordnete Bindung dem Strukturelement Z näher ist.

Der sonstige Rest $R^y$ einer erfindungsgemäß einzusetzenden Verbindung der Struktur $Q(Y_xZ_e)_b$ ist vorzugsweise ausgewählt aus der Gruppe bestehend aus Wasserstoff, linearen, verzweigten oder Ringe umfassenden Strukturelementen mit 1 bis 50 C-Atomen und 0 bis 12 Heteroatomen, wobei die gegebenenfalls vorhandenen Heteroatome vorzugsweise gewählt sind aus der Gruppe bestehend aus N und O.

Der sonstige Rest $R^y$ ist dabei bevorzugt ausgewählt aus der Gruppe bestehend aus Wasserstoff, linearen, verzweigten oder Ringe umfassenden Strukturelementen mit 1 bis 40 C-Atomen und 0 bis 10 Heteroatomen, wobei die gegebenenfalls vorhandenen Heteroatome vorzugsweise gewählt sind aus der Gruppe bestehend aus N und O.

Der sonstige Rest $R^y$ ist dabei besonders bevorzugt ausgewählt aus der Gruppe bestehend aus Wasserstoff, linearen, verzweigten oder Ringe umfassenden Strukturelementen mit 1 bis 35 C-Atomen und 1 bis 10 Heteroatomen, wobei die gegebenenfalls vorhandenen Heteroatome vorzugsweise gewählt sind aus der Gruppe bestehend aus N und O.

Y ist dabei bevorzugt ein Strukturelement, welches ein Strukturelement enthält oder aus diesem besteht, ausgewählt aus der Gruppe bestehend aus

$-CH_2-$

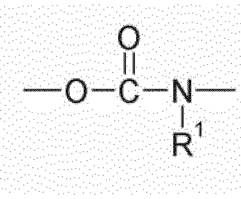

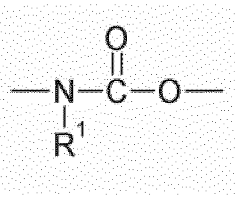

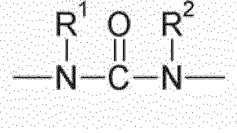

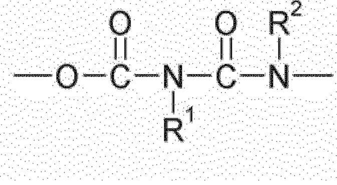

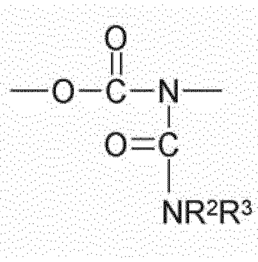

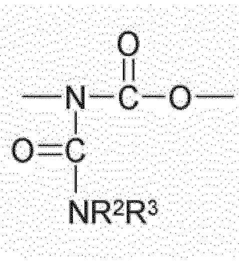

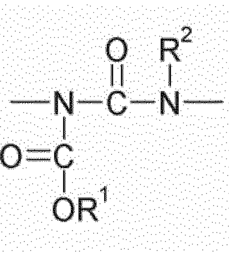

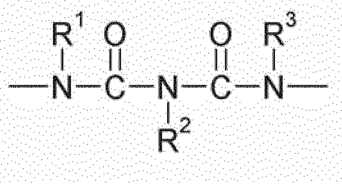

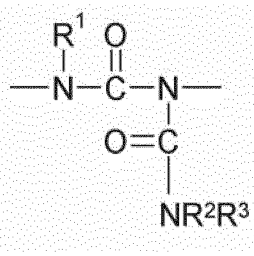

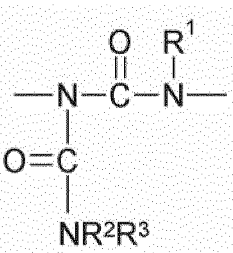

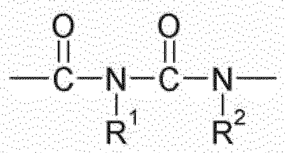

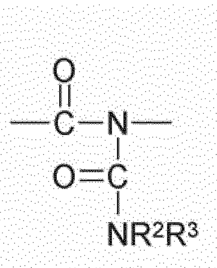

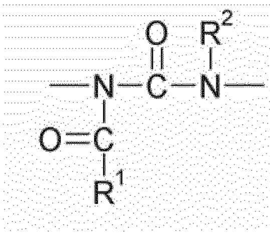

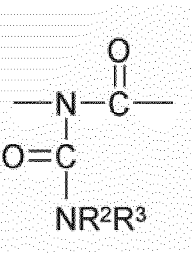

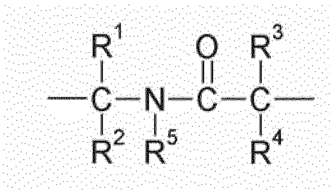

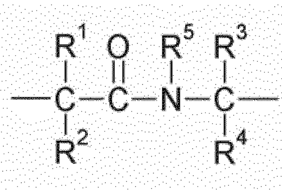

wobei $R^1$, $R^2$, $R^3$, $R^4$ und $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Die oben genannten sonstigen Reste $R^1$, $R^2$, $R^3$, $R^4$ bzw. $R^5$ einer erfindungsgemäß einzusetzenden Verbindung der Struktur $Q(Y_xZ_e)_b$ sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$, $R^3$, $R^4$ bzw. $R^5$ sind dabei, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$, $R^3$, $R^4$ bzw. $R^5$ sind dabei, jeweils unabhängig voneinander, besonders bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

In mit vergleichsweise geringem Aufwand synthetisierbaren erfindungsgemäß einzusetzenden Verbindungen der Struktur $Q(Y_xZ_e)_b$ ist Y ein Strukturelement, welches ein Strukturelement enthält oder aus diesem besteht, das ausgewählt ist aus der Gruppe bestehend aus $-CH_2-$

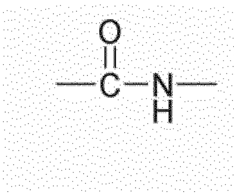
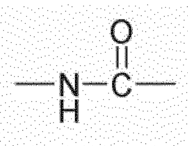

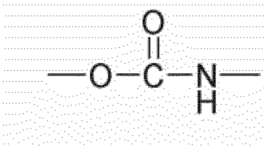
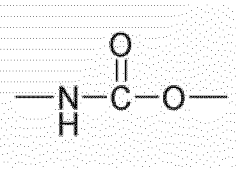
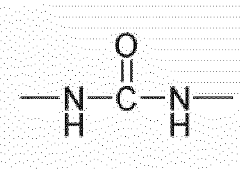

wobei die jeweils im Formelbild links angeordnete Bindung dem Strukturelement Q und die rechts angeordnete Bindung dem Strukturelement Z näher ist.

Die erfindungsgemäß einzusetzenden Verbindungen der Struktur $Q(Y_xZ_e)_b$ können gemäß dem Fachmann bekannten Herstellungsverfahren erhalten werden.

Erfindungsgemäß einzusetzende Verbindungen der Struktur $Q(Y_xZ_e)_b$ mit einem Amid-Strukturelement können beispielsweise durch Umsetzung von (i) einer Edukt-Verbindung mit einer Isocyanatgruppe und (ii) einer Edukt-Verbindung mit einer Carbonsäuregruppe erhalten werden.

Erfindungsgemäß einzusetzende Verbindungen der Struktur $Q(Y_xZ_e)_b$ mit einem Urethan-Strukturelement können beispielsweise durch Umsetzung von (i) einer Edukt-Verbindung mit einer Isocyanatgruppe und einer Edukt-Verbindung mit einer Alkoholgruppe erhalten werden.

Erfindungsgemäß einzusetzende Verbindungen der Struktur $Q(Y_xZ_e)_b$ mit einem Harnstoff-Strukturelement können beispielsweise durch Umsetzung von (i) einer Edukt-Verbindung mit einer Isocyanatgruppe und (ii) einer Edukt-Verbindung mit einer Aminogruppe erhalten werden.

Erfindungsgemäß einzusetzende Verbindungen der Struktur $Q(Y_xZ_e)_b$ mit einem Allophanat-Strukturelement können beispielsweise durch Umsetzung von (i) einer Edukt-Verbindung mit einer Urethangruppe und (ii) einer Edukt-Verbindung mit einer Isocyanatgruppe erhalten werden.

Erfindungsgemäß einzusetzende Verbindungen der Struktur $Q(Y_xZ_e)_b$ mit einem Biuret-Strukturelement können beispielsweise durch Umsetzung von (i) einer Edukt-Verbindung mit einer Harnstoffgruppe und (ii) einer Edukt-Verbindung mit einer Isocyanatgruppe erhalten werden.

Erfindungsgemäß einzusetzende Verbindungen der Struktur $Q(Y_xZ_e)_b$ mit einem *N*-Acylharnstoff-Strukturelement können beispielsweise durch Umsetzung von (i) einer Edukt- Verbindung mit einer Amidgruppe und (ii) einer Edukt-Verbindung mit einer Isocyanatgruppe erhalten werden.

In einer bevorzugten Ausgestaltung einer erfindungsgemäßen Lackzusammensetzung zur Anwendung in einem therapeutischen Verfahren als Dentalmaterial ist Komponente (a1) so gewählt, dass diese umfasst oder besteht

aus Bis(methacryloyloxymethyl)tricyclo[5.2.1.0$^{2,6}$]decan und/oder Bis(acryloyloxymethyl)tricyclo [5.2.1.0$^{2,6}$]decan.

In den erfindungsgemäßen Lackzusammensetzungen zur Anwendung in einem therapeutischen Verfahren als Dentalmaterial sind die Methacrylsäureester wegen ihrer höheren biologischen Verträglichkeit gegenüber den entsprechenden Acrylsäureestern bevorzugt, d.h. dass Z in Verbindungen der Struktur $Q(YZ_e)_b$ (wie oben definiert und wobei jedes Y unabhängig von etwaigen weiteren Strukturelementen Y gewählt ist oder entfällt) bevorzugt $-O-(C=O)-C(CH_3)=CH_2$ bedeutet.

Weiterhin bevorzugte Verbindungen (Monomere) der Struktur $Q(YZ_e)_b$ (wie oben definiert und wobei jedes Y unabhängig von etwaigen weiteren Strukturelementen Y gewählt ist oder entfällt) der Komponente (a1) sind solche mit ein, zwei, drei, vier oder mehr funktionellen Gruppen ausgewählt aus der Gruppe bestehend aus Urethan, Harnstoff, *N*-Acylharnstoff, Allophanat, Biuret und Amid, wobei die Amidfunktion nicht unmittelbar mit einem N-Atom, einem O-Atom oder einer Carbonylgruppe verknüpft ist.

EP 1 238 993 beschreibt ein Verfahren zur Herstellung von Acylharnstoff-Gruppen enthaltenden Polyisocyanaten sowie Gemische hiervon sowie deren Verwendung als Ausgangskomponente bei der Herstellung von Polyurethan-Kunststoffen.

EP 0 209 700 A2, DE 35 22 006 und DE 35 22 005 beschreiben (Meth)acrylsäureDerivate bestimmter Tricyclodecane mit zweibindigen Brückengliedern aus der Gruppe der Urethane bzw. Harnstoffe, welche im Dentalbereich eingesetzt werden können.

EP 0 000 194 A1 (entsprechend US 4,160,080) beschreibt Polyisocyanate, die Allophanatgruppen enthalten. Diese Allophanatpolyisocyanate können zur Herstellung von Polyurethan-Schaumstoffen, Elastomeren, Duromeren, Beschichtungen, Verklebungen und Lackierungen eingesetzt werden.

EP 0 682 012 B1 betrifft ein Verfahren zur Herstellung von hellfarbigen lichtechten Allophanatgruppen aufweisenden (cyclo)aliphatischen Polyisocyanaten, durch Umsetzung von Urethangruppen aufweisenden organischen Verbindungen mit organischen Polyisocyanaten mit (cyclo)aliphatisch gebundenen Isocyanatgruppen in Gegenwart von Zinn(II)-salzen. Die in EP 0 682 012 B1 beschriebenen Polyisocyanate können als Aufbaukomponente bei der Herstellung von Polyurethankunststoffen verwendet werden.

EP 1 727 846 B1 offenbart ein Verfahren zur Herstellung von Allophanatgruppen enthaltenden Bindemitteln, die unter Einwirkung aktinischer Strahlung mit ethylenisch ungesättigten Verbindungen unter Polymerisation reagierende Gruppen und gegebenenfalls NCO-reaktive Gruppen aufweisen.

EP 0 712 840 B1 betrifft ein Verfahren zur Herstellung von bestimmten Allophanatgruppen aufweisenden Polyisocyanaten durch Umsetzung unter Allophanatbildung von Urethangruppen aufweisenden Verbindungen. Die Verbindungen gemäß EP 0 712 840 B1 können als Bindemittel oder Bindemittelkomponente in Beschichtungsmitteln verwendet werden.

EP 0 867 457 B1 offenbart ein ethylenisch ungesättigtes Polyurethan, das im Wesentlichen frei von Isocyanatgruppen ist, welches das Reaktionsprodukt eines ethylenisch ungesättigten Polyisocyanats, das Allophanatgruppen und $\beta,\gamma$-ethylenisch ungesättigte Ethergruppen enthält, mit einer hydroxyfunktionellen, ethylenisch ungesättigten Verbindung ist, wobei das ethylenisch ungesättigte Polyisocyanat durch Allophanatierung des Urethangruppen-enthaltenden Reaktionsprodukts eines organischen Diisocyanats mit einem $\beta,\gamma$-ethylenisch ungesättigten Etheralkohol hergestellt wird, wobei der $\beta,\gamma$-ethylenisch ungesättigte Etheralkohol eine Verbindung umfasst, die aus der aus Glycerindiallylether, Trimethylolpropandiallylether und Pentaerythrittriallylether bestehenden Gruppe ausgewählt ist. Die in EP 0 867 457 B1 offenbarten ethylenisch ungesättigten Polyurethane mit Allophanatgruppen können als Bindemittel in Einkomponenten-Beschichtungszusammensetzungen verwendet werden.

DE 10 2007 040 240 A1 und EP 1 645 582 A1 beschreiben jeweils Verfahren zur Herstellung von strahlenhärtenden Allophanaten durch Umsetzung von isocyanatgruppenhaltigen Verbindungen und hydroxyfunktionellen Verbindungen, wobei das Verhältnis von NCO-Gruppen zu den OH-Gruppen 1,45 : 1,0 bis 1,1 : 1,0 beträgt. Gemäß DE 10 2007 040 239 A1 werden unter Einsatz von bestimmten Mischungen enthaltend Hydroxyethylacrylat und Hydroxypropylacrylat als hydroxyfunktionellen Verbindungen entsprechende strahlenhärtenden Allophanate erhalten. Die strahlenhärtenden Allophanate gemäß dieser drei Dokumente können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden.

DE 10 2004 060 285 A1 betrifft strahlungshärtbare Zusammensetzungen auf Basis eines Dicidolgemisches (enthaltend zwei oder drei isomere 3,8-, 4,8- und/oder 5,8-Bis(hydroxymethyl)tricyclo[5.2.1.0$^{2,6}$]decane) mit mindestens einer Verbindung, welche mindestens eine ethylenisch ungesättigte Gruppierung mit gleichzeitig mindestens einer gegenüber Dicidol reaktive Gruppierung aufweist, wobei diese Verbindung ein Umsetzungsprodukt aus Hydroxyalkyl(meth)acrylat und Diisocyanat sein kann. Die Zusammensetzungen gemäß DE 10 2004 060 285 A1 können verwendet werden als strahlungsinduziert härtende Beschichtungsstoffe, Klebstoffe, Laminierungen, Druckfarben und Tinten, Polituren, Lasuren, Pigmentpasten, Spachtelmassen, Kosmetikartikel, Verpackungsmaterialien und/oder Dicht- und/oder Dämmstoffe.

WO 2006/063891 A1 offenbart radikalisch polymerisierbare Verbindungen, im Wesentlichen enthaltend das

Umsetzungsprodukt eines Dicidolgemisches und mindestens einer Verbindung, welche mindestens eine ethylenisch ungesättigte Gruppierung mit gleichzeitig mindestens einer gegenüber Dicidol reaktive Gruppierung aufweist. Die Anwendungsgebiete entsprechen den in DE 10 2004 060 285 A1 genannten.

WO 03/035013 A1 und DE 602 16 951 T2 betreffen Dentalkleberzusammensetzungen zum Binden von Dental-Restaurierungsmitteln an Dentin und/oder Zahnschmelz. In diesen Dokumenten wird unter anderem die Herstellung von 3,(4),8,(9)-Bis(2-propenamidomethyl)tricyclo$[5.2.1.0]^{2,6}$-decan beschrieben.

US 6,670,499 B1 beschreibt vom Adamantan abgeleitet Diurethane. Die in US 6,670,499 beschriebenen Verbindungen eignen sich als Intermediate für den Einsatz im dentalen Bereich oder zur Herstellung von optischen Materialien (wie beispielsweise Linsen).

Auf dem Gebiet der Dentaltechnik besteht ein ständiger Bedarf an weiteren schrumpfungsarmen radikalisch polymerisierbaren Monomeren. Daher war es eine weitere Aufgabe radikalisch polymerisierbare Monomere bereitzustellen, die in einer erfindungsgemäßen Lackzusammensetzung, insbesondere in einer erfindungsgemäßen dentalen Lackzusammensetzung, als Bestandteil der Komponente (a1) eingesetzt werden können.

Offenbart wird entsprechend eine Verbindung der Struktur $Q(YZ_e)_b$ (wie oben definiert und wobei jedes Y unabhängig von etwaigen weiteren Strukturelementen Y gewählt ist) mit ein, zwei, drei, vier oder mehr funktionellen Gruppen ausgewählt aus der Gruppe bestehend aus *N*-Acylharnstoff, Allophanat, Biuret und Amid, wobei die Amidfunktion nicht unmittelbar mit einem N-Atom, einem O-Atom oder einer Carbonylgruppe verknüpft ist, wobei gilt:

- Q bedeutet ein gesättigtes oder olefinisch ungesättigtes polyalicyclisches Strukturelement ausgewählt aus der Gruppe bestehend aus bicyclischen, tricyclischen, tetracyclischen, pentacyclischen und hexacyclischen Kohlenwasserstoffresten, wobei optional ein, zwei oder mehr der nicht durch Substituenten $YZ_e$ substituierten Wasserstoffatome dieses polyalicyclischen Strukturelements Q durch Alkylgruppen (dabei vorzugsweise C1-C4-Alkyl), Alkoxygruppen (dabei vorzugsweise C1-C4-Alkoxy), Halogenatome (dabei vorzugsweise F) oder Trifluormethylgruppen substituiert sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$ und $-(C=O)-C(CH_3)=CH_2$,

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(YZ_e)_b$ das polyalicyclische Strukturelement Q mit e Strukturelementen Z verbindet;

wobei die Verbindung ein erstes Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,

wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;

- m ist eine natürliche Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,

oder
die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,
oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Aus dem oben Gesagten ergibt sich, dass solche Verbindungen, die eine Amidgruppe (wie definiert) enthalten, diese Amidgruppe nicht Bestandteil einer Urethangruppe ist.

Weiter bevorzugt sind offenbarte Verbindungen der Struktur $Q(YZ_e)b$ (wie oben definiert und wobei jedes Y unabhängig von etwaigen weiteren Strukturelementen Y gewählt ist) der Komponente (a1) mit ein, zwei, drei, vier oder mehr funktionellen Gruppen ausgewählt aus der Gruppe bestehend *N*-Acylharnstoff, Allophanat, Biuret und Amid, wobei die Amidfunktion nicht unmittelbar mit einem N-Atom, einem O-Atom oder einer Carbonylgruppe verknüpft ist, wobei das Amid wiederum vorzugsweise (Meth)acrylamid bedeutet.

In bevorzugten Verbindungen der Struktur $Q(YZ_e)_b$ (wie oben definiert und wobei jedes Y unabhängig von etwaigen weiteren Strukturelementen Y gewählt ist) erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

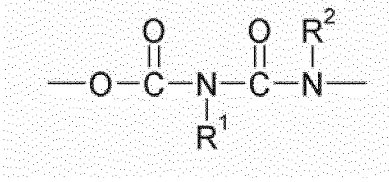
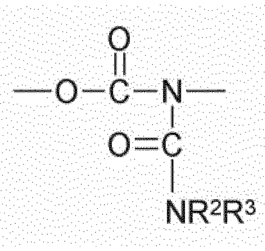
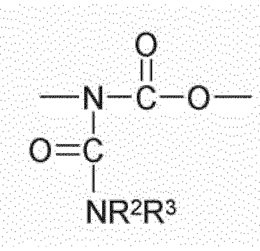

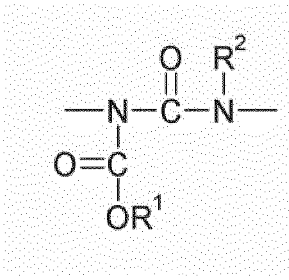
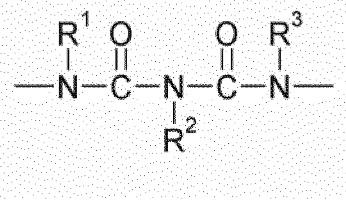
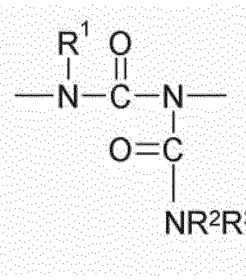

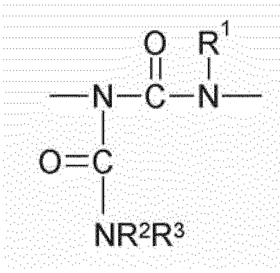
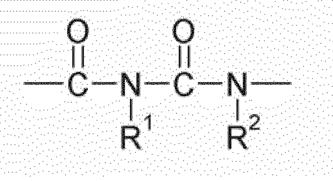
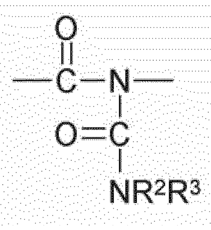

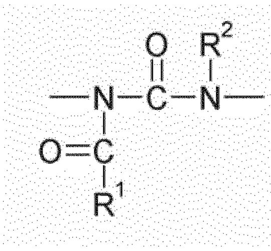
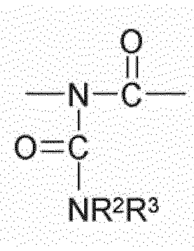

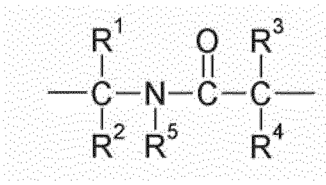
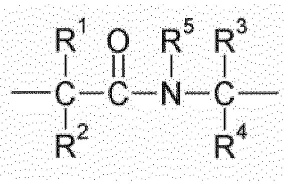

wobei $R^1$ $R^2$, $R^3$ $R^4$, $R^5$ sonstige Reste der Verbindung bedeuten, wobei die jeweils im Formelbild links angeordnete Bindung dem Strukturelement Q und die rechts angeordnete Bindung dem Strukturelement Z näher ist.

Offenbart werden auch Verbindungen der Struktur $Q(Y_xZ_e)_b$ mit x = 1 mit ein, zwei, drei, vier oder mehr funktionellen Gruppen, welche ausgewählt sind aus der Gruppe bestehend aus *N*-Acylharnstoff, Allophanat und Biuret, wobei gilt:

- Q bedeutet ein gesättigtes oder olefinisch ungesättigtes polyalicyclisches Strukturelement ausgewählt aus der Gruppe bestehend aus bicyclischen, tricyclischer, tetracyclischen, pentacyclischen und hexacyclischen Kohlenwasserstoffresten, wobei keines, eines, zwei oder mehr der nicht durch Substituenten $YZ_e$ substituierten Wasserstoffatome dieses polyalicyclischen Strukturelements Q durch Alkylgruppen, Alkoxygruppen, Halogenatome oder Trifluormethylgruppen substituiert ist bzw. sind;

- b ist eine natürliche Zahl ausgewählt aus der Gruppe der natürlichen Zahlen 2, 3, 4;

- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,

  $-(C=O)-CH=CH_2$, $-(C=O)-C(CH_3)=CH_2$,

  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4;

- jedes Y bedeutet ein Strukturelement, welches in der Struktur $Q(Y_xZ_e)_b$ mit x = 1 das polyalicyclische Strukturelement Q mit e Strukturelementen Z verknüpft und ein Strukturelement enthält oder aus diesem besteht, ausgewählt aus der Gruppe bestehend aus

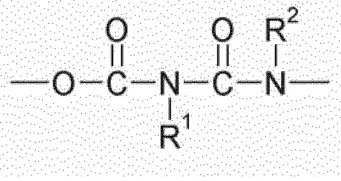
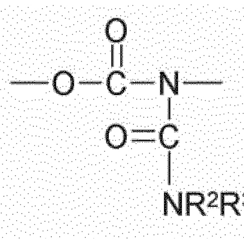
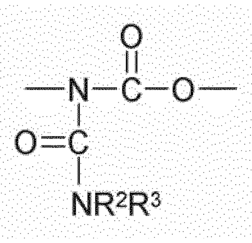

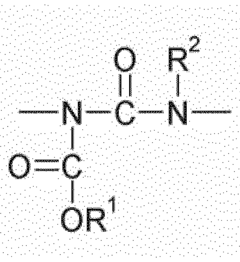 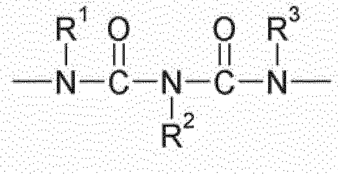 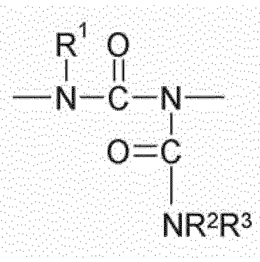

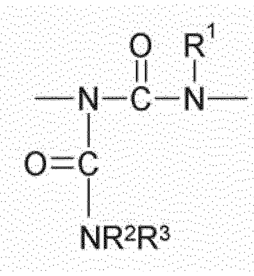 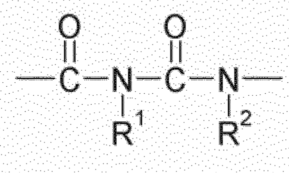 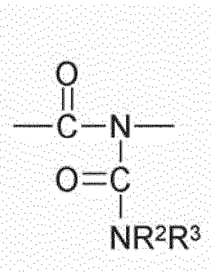

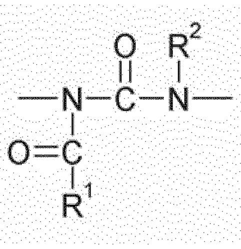 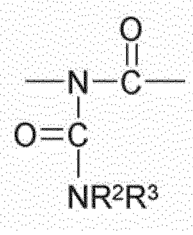

wobei $R^1$, $R^2$ und $R^3$ sonstigen Reste der Verbindung bedeuten, wobei die jeweils im Formelbild links angeordnete Bindung dem Strukturelement Q und die rechts angeordnete Bindung dem Strukturelement Z näher ist.

Diese Verbindungen sind in besonderer Weise als Monomere für den Einsatz in Lackzusammensetzungen zur Anwendung in einem therapeutischen Verfahren als Dentalmaterial geeignet.

Vorzugsweise umfasst eine solche Verbindung der Struktur $Q(Y_xZ_e)_b$ mit x = 1 zwei, drei, vier oder mehr funktionelle Gruppen ausgewählt aus der Gruppe bestehend aus *N*-Acylharnstoff, Allophanat und Biuret.

In einer bevorzugten Ausgestaltung bedeutet jeder Index e eine natürliche Zahl, die unabhängig von etwaigen weiteren Indizes e ausgewählt ist aus der Gruppe der natürlichen Zahlen 2, 3 und 4.

Die oben genannten sonstigen Reste $R^1$, $R^2$ bzw. $R^3$ einer erfindungsgemäß einzusetzenden Verbindung sind, jeweils unabhängig voneinander, vorzugsweise ausgewählt aus der Gruppe bestehend aus Wasserstoff, linearen, verzweigten oder Ringe umfassenden Strukturelementen mit 1 bis 30 C-Atomen und 0 bis 10 Heteroatomen, wobei die gegebenenfalls vorhandenen Heteroatome vorzugsweise gewählt sind aus der Gruppe bestehend aus N und O.

Die sonstigen Reste $R^1$, $R^2$ bzw. $R^3$ einer erfindungsgemäß einzusetzenden Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 25 C-Atomen und 0 bis 8 Heteroatomen, wobei die gegebenenfalls vorhandenen Heteroatome vorzugsweise ausgewählt sind aus der Gruppe bestehend aus N und O.

Die sonstigen Reste $R^1$, $R^2$, bzw. $R^3$ einer erfindungsgemäß einzusetzenden Verbindung sind, jeweils unabhängig voneinander, bevorzugt ausgewählt aus der Gruppe bestehend aus Wasserstoff, linearen, verzweigten oder Ringe umfassenden Strukturelementen mit 1 bis 20 C-Atomen und 0 bis 5 Heteroatomen, wobei die gegebenenfalls vorhandenen Heteroatome ausgewählt sind aus der Gruppe bestehend aus N und O.

Eine erfindungsgemäß einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ist vorzugsweise herstellbar durch Umsetzung eines ersten Umsetzungsproduktes, welches das Umsetzungsprodukt ist aus einer ersten Reaktion von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$ mit

B) zwei oder mehr gleichen oder verschiedenen Verbindungen $MZ_e$, wobei M ein Strukturelement bedeutet, das

jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH,

wobei gilt:

- R bedeutet jeweils unabhängig von etwaigen weiteren R ein Wasserstoffatom oder einen Alkylrest;
- m ist eine natürliche Zahl ausgewählt aus der Gruppe der natürlichen Zahlen von 0 bis 10,
- jeder Index n ist eine natürliche Zahl, die unabhängig von etwaigen weiteren Indizes n ausgewählt ist aus der Gruppe bestehend aus 0 und 1,

wobei die Verbindung ein zweites Umsetzungsprodukt ist aus einer Reaktion von dem obigen ersten Umsetzungsprodukt mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,

oder
die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion.

Eine erfindungsgemäß einzusetzende Verbindung gemäß einer bevorzugten Ausgestaltung ist ein zweites Umsetzungsprodukt aus einer Reaktion von dem obigen ersten Umsetzungsprodukt mit

C) einer weiteren Verbindung gemäß A) oder B), wobei jede weitere Verbindung gemäß A) bzw. B) dieselbe Bedeutung hat wie in der ersten Reaktion,

und/oder
wobei die Verbindung ein drittes Umsetzungsprodukt ist aus einer Reaktion von dem obigen zweiten Umsetzungsprodukt mit

D) einer weiteren Verbindung gemäß A) oder B), wobei jede weitere Verbindung gemäß A) bzw. B) dieselbe Bedeutung hat wie in der ersten und/oder der zweiten Reaktion, vorzugsweise wie in der ersten und der zweiten Reaktion.

In einer bevorzugten Ausführungsform ist m = 0. Dies gilt für sämtliche Aspekte der vorliegenden Erfindung.
In bevorzugten erfindungsgemäß einzusetzenden Verbindungen erfolgt die Verknüpfung zwischen Q und zumindest einem Strukturelement Z über eine Brücke, die ein zweibindiges Brückenglied enthält oder aus diesem besteht, ausgewählt aus der Gruppe bestehend aus

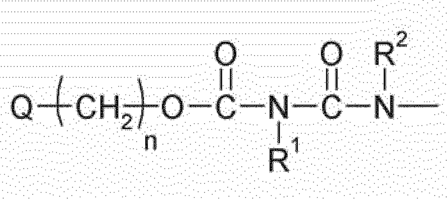

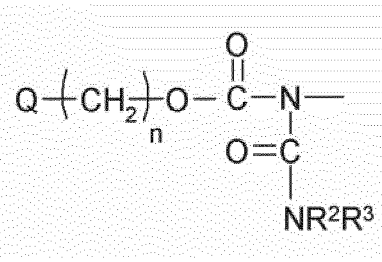

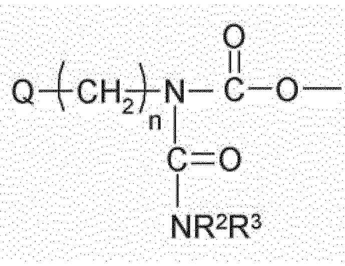

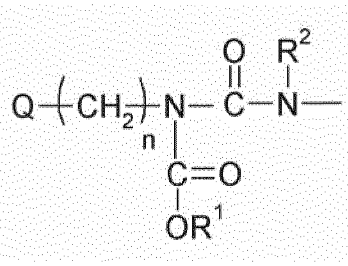

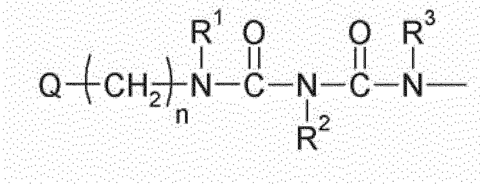

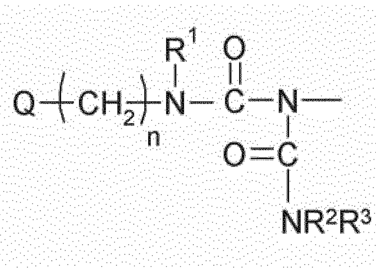

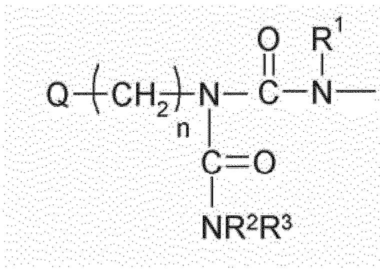

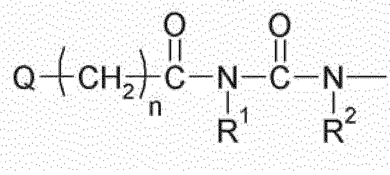

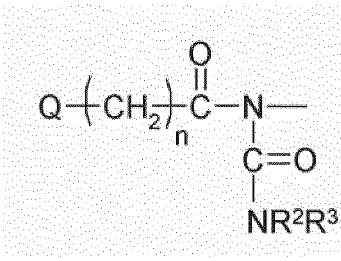

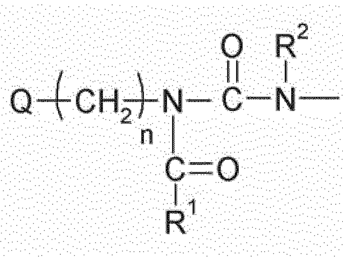

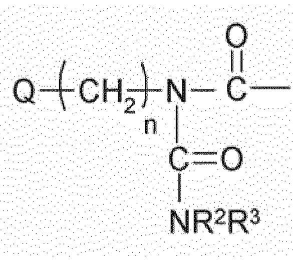

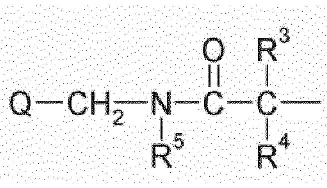

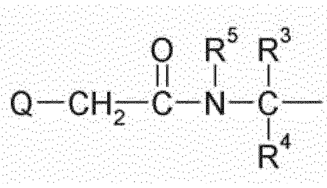

wobei $R^1$, $R^2$, $R^3$ $R^4$, $R^5$ sonstige Reste der Verbindung bedeuten und Q sowie n die oben angegebene Bedeutung haben.

Die jeweils im Formelbild rechts angeordnete Bindung ist dem Strukturelement Z näher.

In einer bevorzugten Ausgestaltung umfasst eine erfindungsgemäß einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, ein oder mehrere Strukturelemente ausgewählt aus der Gruppe bestehend aus

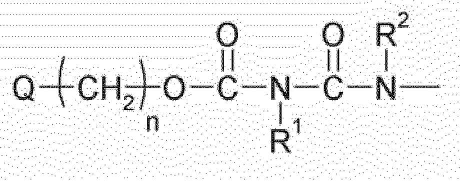

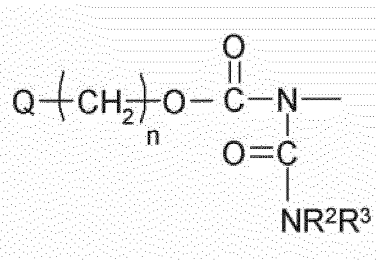

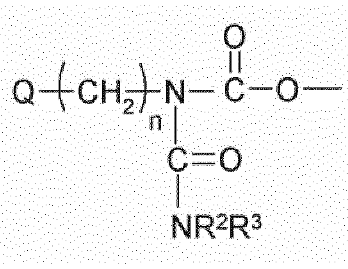

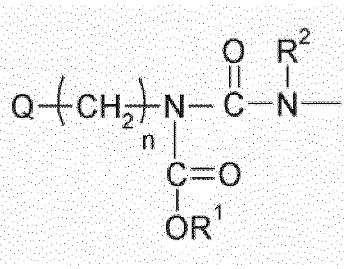

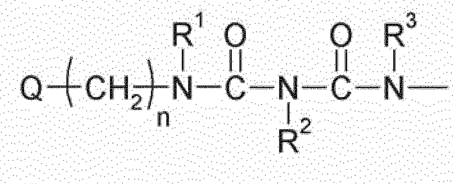

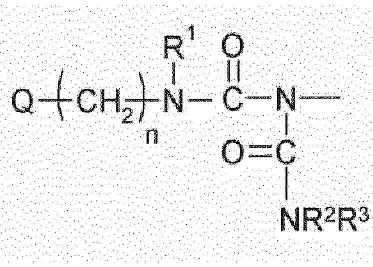

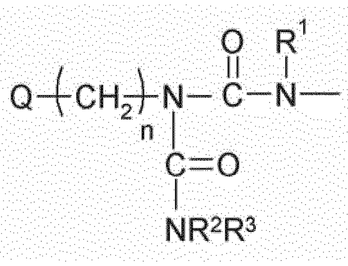

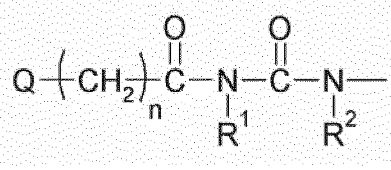

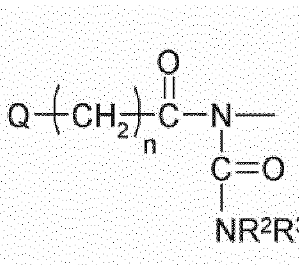

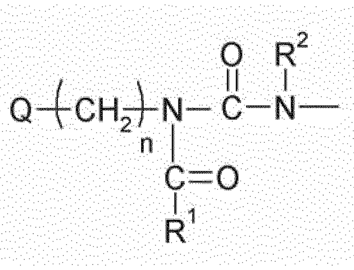

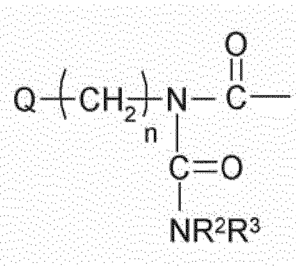

wobei $R^1$, $R^2$ und $R^3$ sonstige Reste der Verbindung bedeuten (und vorzugsweise die oben genannte bevorzugte Bedeutung haben) und Q die oben genannte Bedeutung hat und der Index n ausgewählt ist aus der Gruppe bestehend aus 0 und 1.

Wie oben bereits erwähnt sind bevorzugte erfindungsgemäß einzusetzende Verbindungen solche, wobei Q ein gesättigtes polyalicyclisches Strukturelement bedeutet, das ausgewählt ist aus der Gruppe bestehend aus bicyclischen und tricyclischen Kohlenwasserstoffresten, wobei vorzugsweise keines der nicht durch Substituenten $YZ_e$ (wobei jedes Y unabhängig von etwaigen weiteren Strukturelementen Y gewählt ist) substituierten Wasserstoffatome dieses polyalicyclischen Strukturelements Q substituiert ist.

Besonders bevorzugte erfindungsgemäß einzusetzende Verbindungen sind solche, wobei das Strukturelement Q einen Tricyclo[5.2.1.$0^{2,6}$]decan-Rest, einen Tricyclo[5.2.1.$0^{2,6}$]dec-3-enRest oder einen Tricyclo[3.3.1.$1^{3,7}$]decan-Rest bedeutet.

Bevorzugt sind erfindungsgemäße neue Verbindungen, in denen

(i) das Strukturelement Z = $-O-(C=O)-C(CH_3)=CH_2$ bedeutet, wobei die funktionellen Gruppen Allophanat-, Biuret- oder Acylharnstoff-Gruppen sind, da mit diesen Verbindungen besonders gute Ergebnisse erzielt wurden, und/oder

(ii) das Strukturelement Q einen Tricyclo[5.2.1.$0^{2,6}$]decan-Rest bedeutet.

Weiter bevorzugt sind erfindungsgemäß einzusetzende Verbindungen, in denen das Strukturelement Z = $-O-(C=O)-C(CH_3)=CH_2$ bedeutet, wobei die funktionellen Gruppen Allophanat-, Biuret-oder Acylharnstoff-Gruppen sind und das Strukturelement Q einen Tricyclo[5.2.1.$0^{2,6}$]decan-Rest bedeutet.

Bevorzugt sind erfindungsgemäß einzusetzende Verbindungen, in denen sämtliche vorhandenen lichthärtbaren Gruppen dem Strukturelement Z entsprechen.

Bevorzugt sind erfindungsgemäßeinzusetzende Verbindungen, in denen sämtliche vorhandenen endständigen polymerisierbaren Gruppen dem Strukturelement Z entsprechen.

Eine erfindungsgemäß einzusetzende Verbindung kann neben lichthärtbaren Gruppen des Strukturelements Z auch weitere polymerisierbare, vorzugsweise endständige polymerisierbare, Gruppen umfassen, die nicht lichthärtbar sind, insbesondere nicht unter den im Dentalbereich üblichen Bedingungen des Lichthärtens. Dies ist regelmäßig jedoch nicht bevorzugt, da solche Gruppen nicht zu den gewünschten Eigenschaften des nach der Polymerisation vorliegenden Produktes beitragen.

Weiter bevorzugte erfindungsgemäß einzusetzende Verbindungen sind solche, in denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

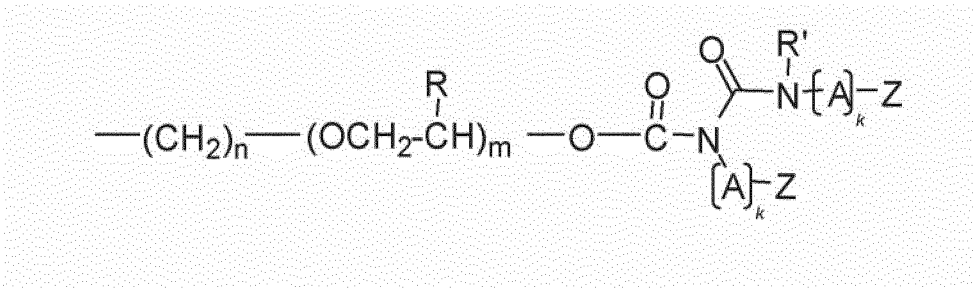

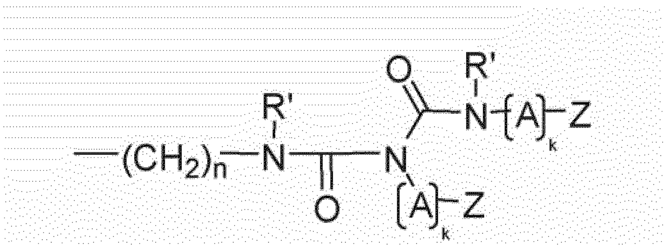

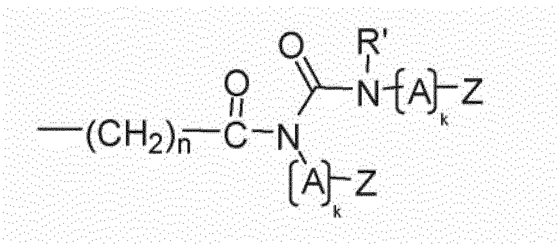

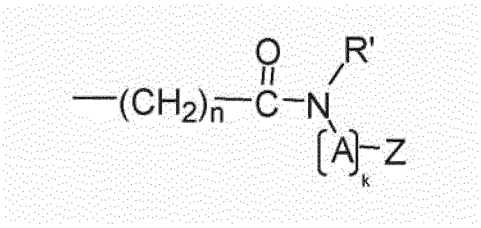

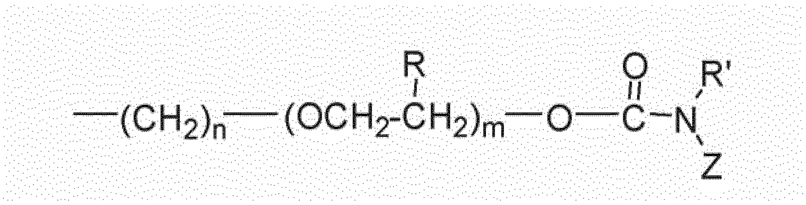

wobei Z, R, m sowie n die obige Bedeutung haben und worin ferner gilt:

- jedes A bedeutet ein zweibindiges organisches Brückenglied,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

In einer bevorzugten Ausführungsform ist m = 0.
Ebenfalls bevorzugte erfindungsgemaß einzusetzende Verbindungen sind solche, bei denen mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

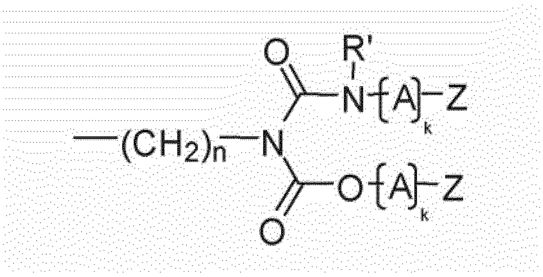

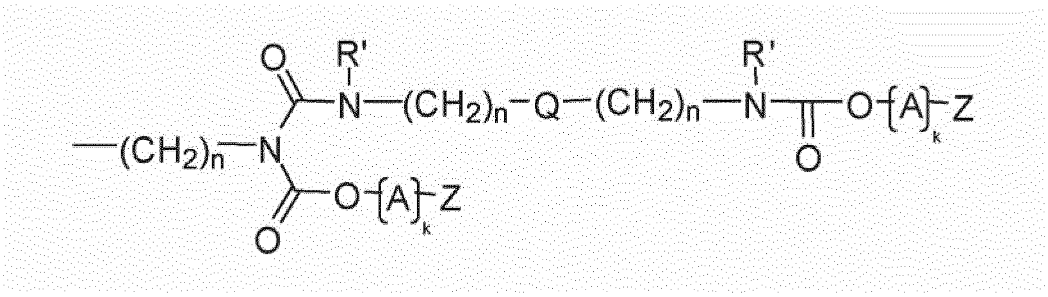

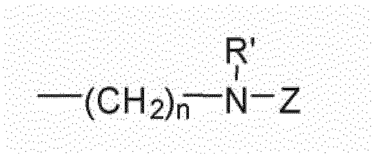

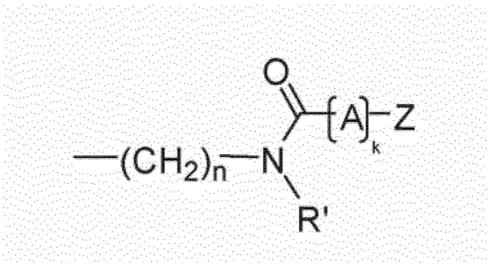

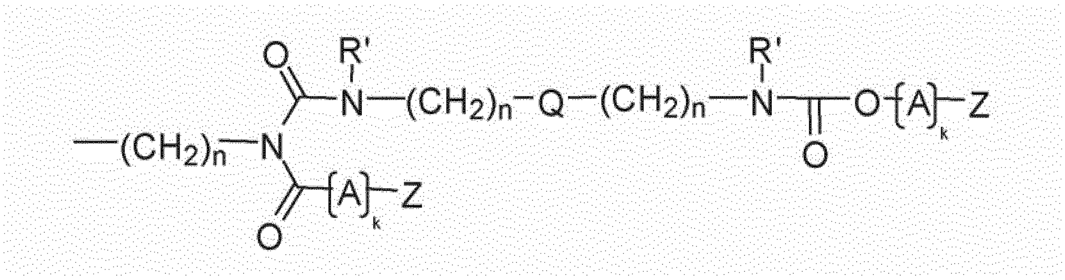

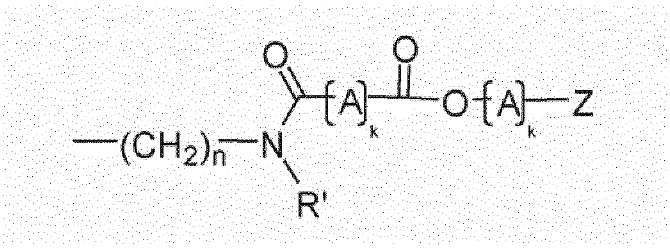

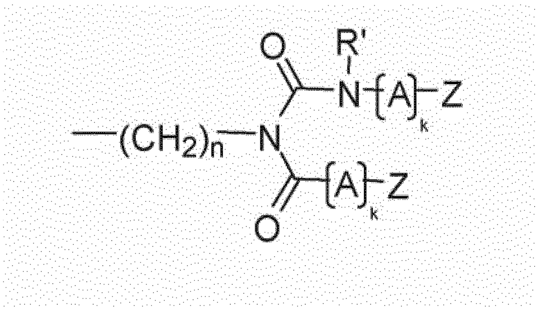

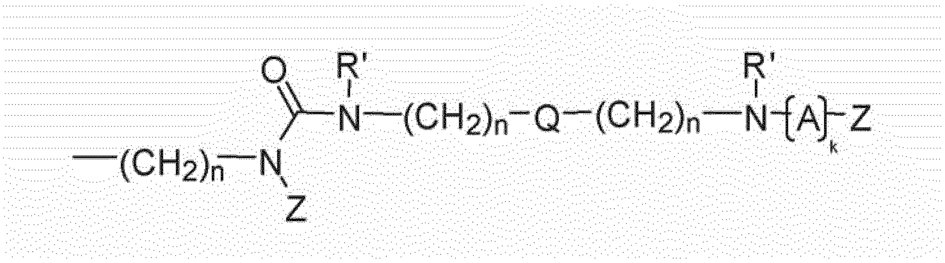

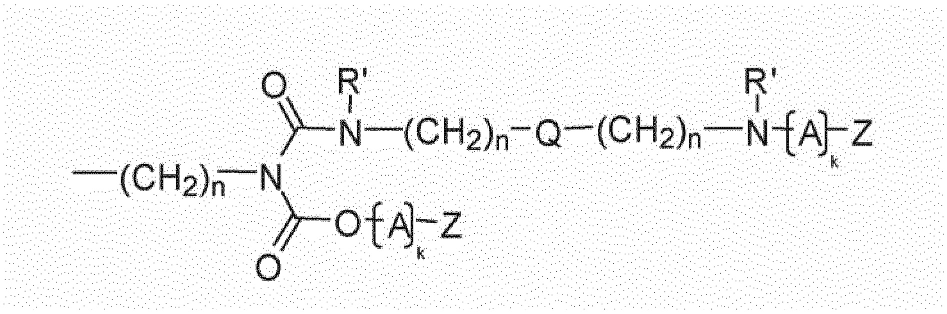

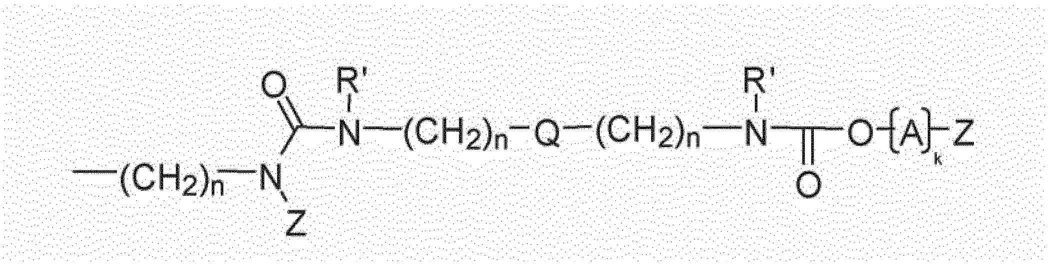

wobei jedes Q hat unabhängig von etwaigen weiteren Strukturelementen Q die obige Bedeutung hat und wobei Z, A, k und R' sowie n die oben genannte Bedeutung haben.

Bevorzugt ist eine erfindungsgemäß einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, wobei mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind, aus der Gruppe bestehend aus

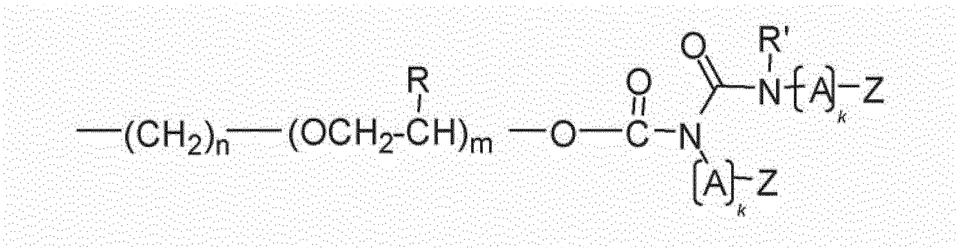

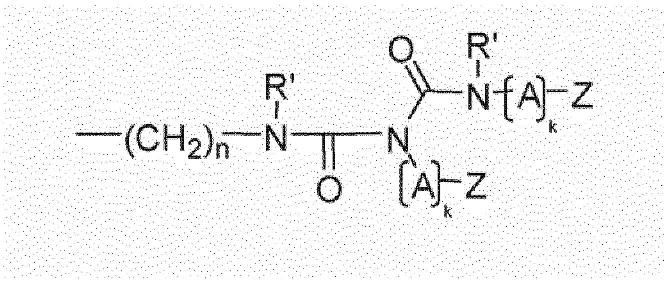

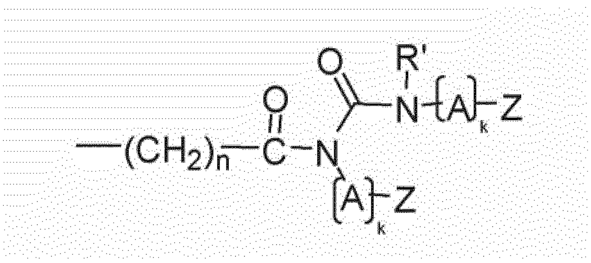

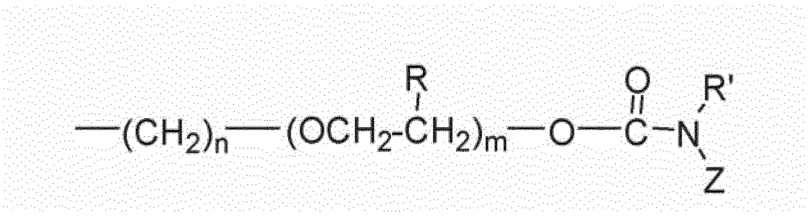

wobei Z, R, m sowie n die obige Bedeutung haben und worin ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

Bevorzugt ist eine erfindungsgemäß einzusetzende Verbindung $Q(Y_xZ_e)_b$ mit x = 1, vorzugsweise eine Verbindung $Q(Y_xZ_e)_b$ mit x = 1 wie sie vorstehend oder nachfolgend als bevorzugt bezeichnet ist, wobei
mindestens ein Strukturelement $YZ_e$ unabhängig von dem oder den weiteren Strukturelementen $YZ_e$ ausgewählt ist, und vorzugsweise sämtliche Strukturelemente $YZ_e$ ausgewählt sind,
aus der Gruppe bestehend aus

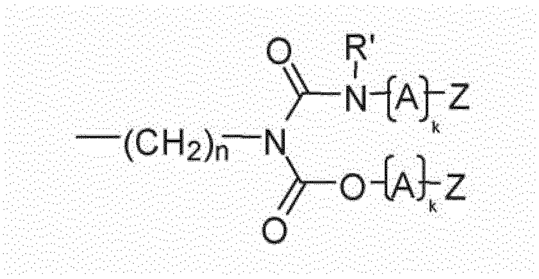

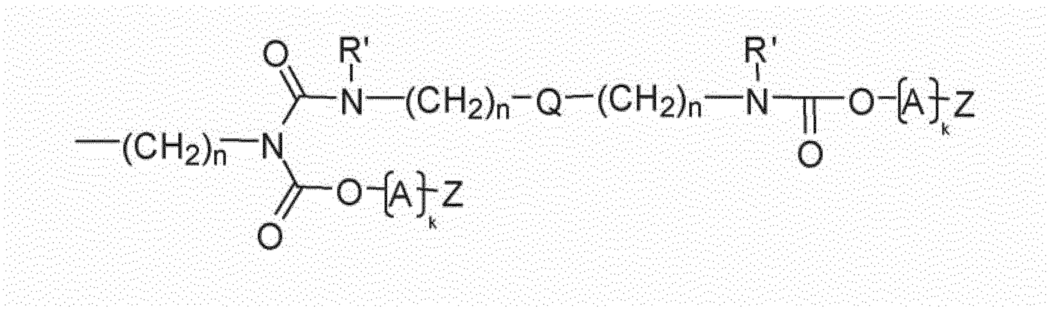

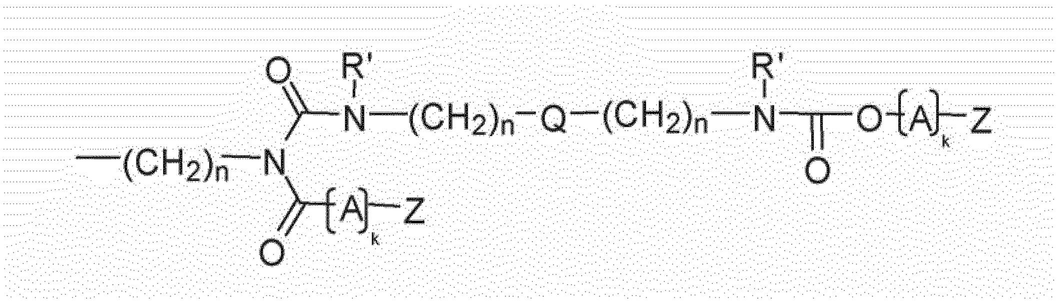

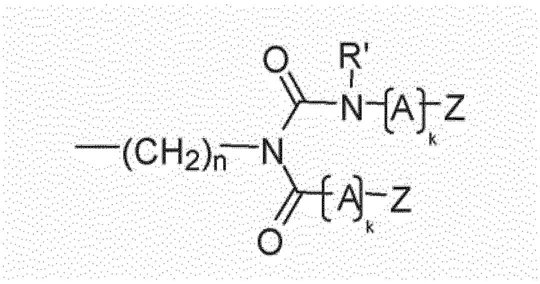

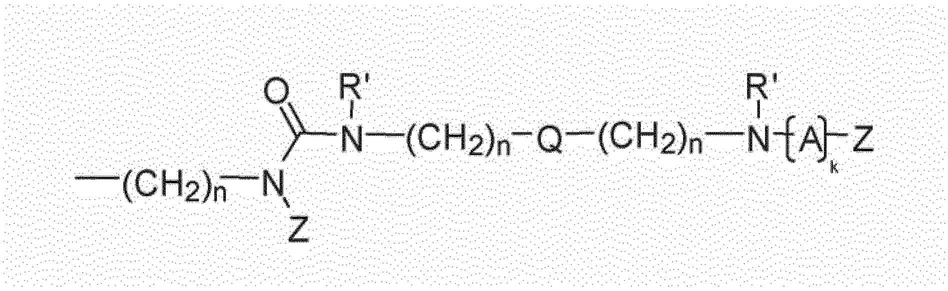

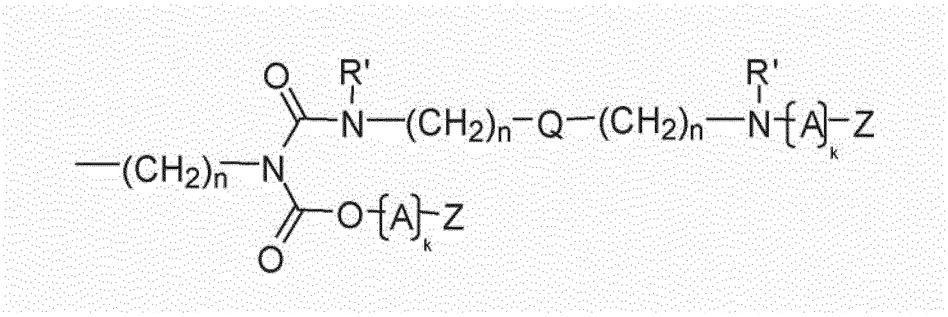

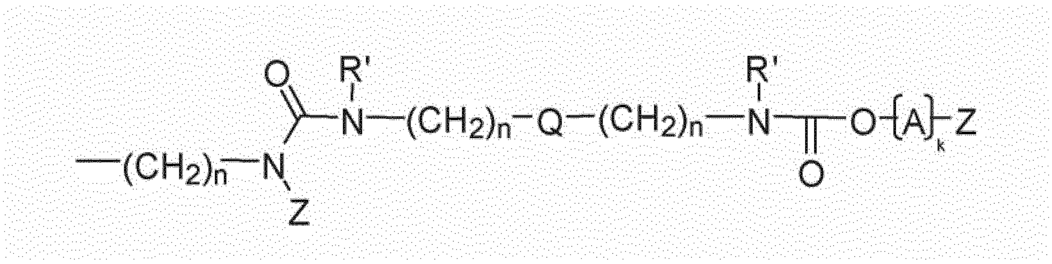

wobei jedes Q unabhängig von etwaigen weiteren Strukturelementen Q die obige Bedeutung hat, und wobei Z und n die oben genannte Bedeutung haben und wobei ferner gilt:

- jedes A bedeutet ein organisches Strukturelement,
- jeder Index k ist eine natürliche Zahl, die unabhängig von etwaigen weiteren Indizes k ausgewählt ist aus der Gruppe bestehend aus 0 und 1;
- jedes R' bedeutet ein Strukturelement, das unabhängig von etwaigen weiteren Strukturelementen R' ausgewählt ist aus der Gruppe bestehend aus H und einem Strukturelement (C=O)-NH-$(A)_k$-Z, wobei A, Z und k wiederum die obigen Bedeutungen haben.

Dabei wiederum bevorzugt sind erfindungsgemäße einzusetzende Verbindungen, in denen jedes Strukturelement A unabhängig von etwaigen weiteren Strukturelementen A ausgewählt ist aus der Gruppe bestehend aus linearen, verzweigten oder Ringe umfassenden zweibindigen organischen Brückengliedern mit 1 bis 25 C-Atomen und gegebenenfalls 1 bis 10, vorzugsweise 1 bis 5 Heteroatomen, wobei die gegebenenfalls vorhandenen Heteroatome vorzugsweise gewählt sind aus der Gruppe bestehend aus N und O.

Dabei wiederum bevorzugt ist eine Verbindung, in der jedes Strukturelement A unabhängig von etwaigen weiteren Strukturelementen A ausgewählt ist aus der Gruppe bestehend aus linearen, verzweigten oder Ringe umfassenden Strukturelementen mit 1 bis 25 C-Atomen und 0 bis 10 Heteroatomen, vorzugsweise mit 1 bis 5 Heteroatomen, wobei die gegebenenfalls vorhandenen Heteroatome vorzugsweise gewählt sind aus der Gruppe bestehend aus N und O.

Weiter bevorzugt sind Verbindungen, in denen jedes Strukturelement A unabhängig von etwaigen weiteren Strukturelementen A ausgewählt ist aus der Gruppe bestehend aus $C_1$-$C_{20}$-Alkylen, $C_1$-$C_{20}$-Heteroalkylen, $C_3$-$C_{20}$-Cycloalkylen, $C_4$-$C_{20}$-Cycloalkylalkylen, $C_2$-$C_{20}$-Alkenylen, $C_3$-$C_{20}$-Cycloalkenylen, $C_4$-$C_{20}$-Cycloalkenylalkylen, $C_4$-$C_{20}$-Cycloalkenylenalkylen, $C_3$-$C_{25}$-Arylen, $C_2$-$C_{25}$-Heteroarylen, $C_4$-$C_{25}$-Arylalkylen, $C_4$-$C_{25}$-Arylenalkylen, $C_4$-$C_{25}$-Arylheteroalkylen, $C_4$-$C_{25}$-Arylenheteroalkylen.

In bevorzugten Ausgestaltungen umfasst Strukturelement A eine oder mehrere der folgenden Atome oder Atomgruppen:

$$-O-, -O-Ar^1-CR^6R^7-Ar^2-O-, -NR^8-, -N-(C=O)-, -NH-(C=O)-O-, -NH-C(=O)-NH-$$

wobei gilt:

> $Ar^1$ und $Ar^2$ bedeuten unabhängig voneinander einen gegebenenfalls substituierten, dabei vorzugsweise einen ein oder mehrfach mit C1-C4-Alkylresten substituierten, aromatischen Ring, dabei wiederum bevorzugt einen Rhenylring,
>
> $R^6$, $R^7$ und $R^8$ bedeuten unabhängig voneinander Wasserstoff oder einen C1-C8-Rest, dabei vorzugsweise einen C1-C4-Alkylrest, dabei wiederum bevorzugt Methyl oder Ethyl.

Offenbart wird ferner ein Verfahren zur Herstellung einer Verbindung $Q(YZ_e)_b$ (wobei jedes Y unabhängig von etwaigen weiteren Strukturelementen Y gewählt ist) oder einer Mischung, umfassend zumindest solche eine erfindungsgemäße Verbindung $Q(YZ_e)_b$, mit folgenden Schritten:

In einer ersten Reaktion Umsetzen von

> A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$, vorzugsweise einer Verbindung der Struktur $QG_b$, worin jedes G eine reaktive Gruppe bedeutet, die unabhängig von weiteren Gruppen G ausgewählt ist aus der Gruppe bestehend aus $-NH_2$, $-CH_2NH_2$, -OH, $-CH_2OH$, -NCO, $-CH_2NCO$, und-COOH,
> mit
>
> B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH
> zu einem ersten Umsetzungsprodukt,

gegebenenfalls in einer zweiten Reaktion Umsetzen des ersten Umsetzungsproduktes mit

> C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,

zu einem zweiten Umsetzungsprodukt
und gegebenenfalls in einer dritten Reaktion Umsetzen des zweiten Umsetzungsproduktes mit

> D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion,

wobei Q, b, Y, Z, und e jeweils die oben genannte Bedeutung haben, und wobei gilt:

- R bedeutet jeweils unabhängig von etwaigen weiteren R ein Wasserstoffatom oder einen Alkylrest; bevorzugt bedeutet R ein Wasserstoffatom oder einen linearen oder verzweigten Alkylrest mit 1 bis 6 C-Atomen; weiter bevorzugt bedeutet R ein Wasserstoffatom oder einen Methylrest;

- m ist eine natürliche Zahl ausgewählt aus der Gruppe der natürlichen Zahlen von 0 bis 10,

- jeder Index n ist eine natürliche Zahl, die unabhängig von etwaigen weiteren Indizes n ausgewählt ist aus der Gruppe bestehend aus 0 und 1,
wobei das Verhältnis der Gesamtzahl von NCO-Gruppen zu der Gesamtzahl von-$NH_2$, -OH und -COOH in der Gesamtzahl von Verbindungen gemäß A) und B) in der ersten, gegebenenfalls zweiten und gegebenenfalls dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.

Ein bevorzugtes Verfahren zur Herstellung einer Verbindung $Q(Y_xZ_e)_b$ mit x = 1, vorzugsweise einer Verbindung $Q(Y_xZ_e)_b$ mit x = 1 wie sie vorstehend oder nachfolgend als bevorzugt bezeichnet ist, oder einer Mischung umfassend zumindest eine solche Verbindung $Q(Y_xZ_e)_b$, ist ein Verfahren mit folgenden Schritten:
In einer ersten Reaktion Umsetzen von

A) einer Verbindung der Struktur $QG_b$, worin jedes G eine reaktive Gruppe bedeutet, die unabhängig von weiteren Gruppen G ausgewählt ist aus der Gruppe bestehend aus $(-CH_2)_n-NH_2$, $(-CH_2)_n-(OCH_2-CHR)_m-OH$, $(-CH_2)_n-NCO$ und $(-CH_2)_n-COOH$
mit

B) zwei oder mehr gleichen oder verschiedenen Verbindungen $MZ_e$, wobei M ein Strukturelement bedeutet, das jeweils eine oder mehrere gegenüber den reaktiven Gruppen G reaktive Gruppierung(en) aufweist ausgewählt aus der Gruppe bestehend aus -NH, $-NH_2$, -OH, -NCO und -COOH

zu einem ersten Umsetzungsprodukt,
in einer zweiten Reaktion Umsetzen des ersten Umsetzungsproduktes mit

C) einer weiteren Verbindung gemäß A) oder B), wobei jede weitere Verbindung gemäß A) bzw. B) die obige Bedeutung hat, unabhängig von der Bedeutung von A) bzw. B) in der ersten Reaktion,

zu einem zweiten Umsetzungsprodukt
und gegebenenfalls in einer dritten Reaktion Umsetzen des zweiten Umsetzungsproduktes mit

D) einer weiteren Verbindung gemäß A) oder B), wobei jede weitere Verbindung gemäß A) bzw. B) die obige Bedeutung hat, unabhängig von der Bedeutung von A) bzw. B) in der ersten bzw. zweiten Reaktion,

wobei Q, b, Y, Z, e, M, R, m und n jeweils die oben genannte Bedeutung haben, und
wobei das Verhältnis der Gesamtzahl von NCO-Gruppen zu der Gesamtzahl von $-NH_2$,-OH und -COOH in der Gesamtzahl von Verbindungen gemäß A) und B) in der ersten, der zweiten und gegebenenfalls der dritten Reaktion größer als oder gleich 1 ist, bevorzugt im Bereich von 1,1 : 1 bis 5 : 1 liegt, weiter bevorzugt im Bereich von 1,25 : 1 bis 4 : 1 liegt, besonders bevorzugt im Bereich von 1,5 : 1 bis 3 : 1 liegt, und am meisten bevorzugt im Bereich von 2 : 1 bis 2,5 : 1 liegt.
In einem Verfahren zur Herstellung einer Verbindung $Q(Y_xZ_e)_b$ mit x = 1, vorzugsweise einer Verbindung $Q(Y_xZ_e)_b$ mit x = 1 wie sie vorstehend oder nachfolgend als bevorzugt bezeichnet ist, oder einer Mischung umfassend zumindest eine solche Verbindung $Q(Y_xZ_e)_b$, liegt das Verhältnis der Gesamtzahl von umgesetzten NCO-Gruppen zu der Gesamtzahl von umgesetzten-$NH_2$, -OH und -COOH in der Gesamtzahl von Verbindungen gemäß A) und B) in der ersten, gegebenenfalls zweiten und gegebenenfalls dritten Reaktion bevorzugt im Bereich von 1,1 : 1 bis 5 : 1, weiter bevorzugt im Bereich von 1,25 : 1 bis 4 : 1, besonders bevorzugt im Bereich von 1,5 : 1 bis 3 : 1, und am meisten bevorzugt im Bereich von 2 : 1 bis 2,5 : 1.
Vorzugsweise erfolgt die Reaktion zu dem ersten Umsetzungsprodukt, zu dem zweiten Umsetzungsprodukt und/oder zu dem dritten Umsetzungsprodukt in Gegenwart eines Katalysators.
Bevorzugte Katalysatoren sind dabei tertiäre Amine oder Lewis Säuren, dabei wiederum bevorzugt Metallsalze höherer Fettsäuren, insbesondere Dibutylzinndilaurat oder Zinn(II)octoat.
Die Menge des Katalysators liegt dabei vorzugsweise im Bereich von 0,01 bis 2 Gew.-%, bevorzugt von 0,08 bis 1 Ges.-%, bezogen auf die Gesamtmenge der Reaktanden gemäß A) und B) sowie gegebenenfalls C) und gegebenenfalls D).
Die Reaktion zu dem ersten Umsetzungsprodukt, zu dem zweiten Umsetzungsprodukt und/oder zu dem dritten Umsetzungsprodukt erfolgt vorzugsweise in einem Temperaturbereich von 0 bis 160°C, bevorzugt im Bereich von 30 bis 140°C und besonders bevorzugt im Bereich von 60 bis 120°C. Die Umsetzung wird vorzugsweise bei Normaldruck

(1013 mbar) ausgeführt.

Offenbart wird auch eine Mischung umfassend ein, zwei oder mehr unterschiedliche erfindungsgemäß einzusetzende Verbindungen, herstellbar nach einem hier offenbarten Verfahren.

Offenbart wird auch die Verwendung einer Lackzusammensetzung, vorzugsweise in einer der als bevorzugt oder besonders bevorzugt gekennzeichneten Ausgestaltungen, zur Beschichtung einer Oberfläche, insbesondere als Schutz- und/oder Glanzlack.

Offenbart wird auch die Verwendung einer erfindungsgemäß einzusetzende Verbindung, vorzugsweise in einer der als bevorzugt oder besonders bevorzugt gekennzeichneten Ausgestaltungen, zur Herstellung einer dentalen Lackzusammensetzung.

Offenbart wird auch die Verwendung einer erfindungsgemäß einzusetzenden Verbindung, vorzugsweise in einer der als bevorzugt oder besonders bevorzugt gekennzeichneten Ausgestaltungen, in einer Lackzusammensetzung, vorzugsweise in einer der als bevorzugt oder besonders bevorzugt gekennzeichneten Ausgestaltungen.

Offenbart wird auch die Verwendung einer erfindungsgemäß einzusetzenden Verbindung, vorzugsweise in einer der als bevorzugt oder besonders bevorzugt gekennzeichneten Ausgestaltungen, in einer dentalen Lackzusammensetzung.

Offenbart wird auch eine Lackzusammensetzung als bzw. zur Verwendung als Dentalmaterial, vorzugsweise als Kavitäten- und/oder Oberflächenlack, als Unterfüllung für Zahnfüllmaterialien, zum Schutz des Zahnschmelzes, zur Verhinderung von Karies, zum Schutz einer Restauration (insbesondere gegen vorzeitige Abnutzung), zur Verbesserung der Verschleissfestigkeit eines dentalen Füllungsmaterials, zur Festigung restaurierter Oberflächen, zum Schutz vor Abrasion und/oder Verfärbung eines Zahns oder einer Restauration, zum Schließen marginaler Spalten und/oder von Mikrorissen, zur Glättung von restaurativen Oberflächen, zum Verleihen von natürlichem Glanz auf einem Zahn oder einer Restauration und zum Reduzierung von Farbstoffablagerungen auf einem Zahn oder einer Restauration.

Offenbart wird auch ein Erzeugnis, erhältlich durch Härten einer erfindungsgemäß einzusetzenden Lackzusammensetzung, insbesondere in einer der als bevorzugt bzw. besonders bevorzugt gekennzeichneten Ausgestaltungen.

Die vorliegende Erfindung betrifft somit auch eine Lackzusammensetzung, vorzugsweise in einer der vorstehend bzw. nachstehend als bevorzugt bezeichneten Ausgestaltungen, zur Anwendung in einem therapeutischen Verfahren als Dentalmaterial, vorzugsweise als Kavitäten- und/oder Oberflächenlack, als Unterfüllung für Zahnfüllmaterialien, zum Schutz des Zahnschmelzes, zur Verhinderung von Karies, zum Schutz einer Restauration (insbesondere gegen vorzeitige Abnutzung), zur Verbesserung der Verschleissfestigkeit eines dentalen Füllungsmaterials, zur Festigung restaurierter Oberflächen, zum Schutz vor Abrasion und/oder Verfärbung eines Zahns oder einer Restauration, zum Schließen marginaler Spalten und/oder von Mikrorissen, zur Glättung von restaurativen Oberflächen, zum Verleihen von natürlichem Glanz auf einem Zahn oder einer Restauration und zum Reduzieren von Farbstoffablagerungen auf einem Zahn oder einer Restauration.

Offenbart wird auch die nicht-therapeutische Verwendung, vorzugsweise die kosmetische bzw. ästhetische Verwendung, einer Lackzusammensetzung, vorzugsweise in einer der vorstehend bzw. nachstehend als bevorzugt bezeichneten Ausgestaltungen, zur Glättung von restaurativen Oberflächen, zum Verleihen von natürlichem Glanz auf einem Zahn oder einer Restauration und zum Reduzieren von Farbstoffablagerungen auf einem Zahn oder einer Restauration.

Offenbart wird auch die Verwendung einer hier offenbarten Lackzusammensetzung, vorzugsweise in einer der vorstehend bzw. nachstehend als bevorzugt bezeichneten Ausgestaltungen, in einem nicht-therapeutischen Verfahren, vorzugsweise einem kosmetischen bzw. ästhetischen Verfahren, zur Glättung von restaurativen Oberflächen, zum Verleihen von natürlichem Glanz auf einem Zahn oder einer Restauration und zum Reduzieren von Farbstoffablagerungen auf einem Zahn oder einer Restauration.

Zur Herstellung der erfindungsgemäß einzusetzenden Verbindungen können vorzugsweise Hydroxylverbindungen von (Meth)acrylaten eingesetzt werden, wobei auch Gemische von Acrylaten und Methacrylaten verwendet werden können. Bevorzugt sind (zum Einsatz als Reaktionspartner gemäß Komponente B), C) und/oder D)): Alkylenoxidmono(meth)acrylate wie beispielsweise Ethylenglykolmono(meth)acrylat, Diethylenglykomono(meth)acrylat, Triethylenglykolmono(meth)acrylat, Tetraethylenglykolmono(meth)acrylat, etc., Polyalkylenoxidmono(meth)acrylate wie beispielsweise Polyethylenglykolmono(meth)acrylat, Polypropylenglykolmono(meth)acrylat, Polybutylenglykolmono(meth)acrylat, etc., Hydroxyalkylmono(meth)acrylate wie beispielsweise Hydroxyethyl(meth)acrylat, Hydroxypropyl(meth)acrylat, 2-Hydroxypropyl(meth)acrylat, Hydroxybutyl(meth)acrylat, 4-Hydroxybutyl(meth)acrylat, Hydroxypentyl(meth)acrylat, 3-Hydroxy-2,2-dimethylpropyl(meth)acrylat, Hydroxyhexyl(meth)acrylat, Hydroxyheptyl(meth)acrylat, Hydroxyoctyl(meth)acrylat, Hydroxynonyl(meth)acrylat, Hydroxydecyl(meth)acrylat, Hydroxyundecyl(meth)acrylat, Hydroxydodecyl(meth)acrylat, etc., Poly(ε-caprolacton)mono(meth)acrylat, Poly(γ-caprolacton)mono(meth)acrylat, etc., die Mono-, Di-, Tetra-, oder Penta(meth)acrylate mehrwertiger Alkohole, wie Glycerin, wie beispielsweise Glycerindi(meth)acrylat (2-Hydroxypropyl-1,3-di(meth)acrylat, 3-Hydroxypropyl-1,2-di(meth)acrylat), wie Trimethylolpropan, wie beispielsweise Trimethylolpropandi(meth)acrylat, wie Pentaerythrit, wie beispielsweise Pentaerythritoltri(meth)acrylat,

wie Dipentaerythrit, wie beispielsweise Dipentaerythritolpenta(meth)acrylat, wie Ditrimethylolpropantri(meth)acrylat, wie Neopentylglykol(meth)acrylat, die (Meth)acrylate von alkoxyliertem oder phenoxyliertem Glycerin, dabei vorzugsweise die (Meth)acrylate von ethoxyliertem, propoxyliertem, etc. Glycerin, Trimethylolpropan, Pentaerythrit, Dipentaerythrit, Ditrimethylolpropan etc. sowie deren technische Gemische, Bisphenol-A-Glycidyl-(Meth)acrylat (Bis-GMA), Bisphenol-B-Glycidyl-(Meth)acrylat, Bisphenol-C-Glycidyl-(Meth)acrylat, Bisphenol-F-Glycidyl-(Meth)acrylat, alkoxyliertes Bisphenol-A-Glycidyl-(Meth)acrylat (z.B. ethoxyliertes Bisphenol-A-Glycidyl-(Meth)acrylat), etc..

Zur Herstellung der erfindungsgemäß einzusetzenden Verbindungen können als Komponente B) auch Isocyanate eingesetzt werden. Bevorzugt sind dabei Mono- und Diisocyanate.

Bevorzugte Diisocyanate sind gewählt aus der Gruppe bestehend aus Cyclohexandiisocyanat, Methylcyclohexandiisocyanat, Ethylcyclohexandiisocyanat, Propylcyclohexandiisocyanat, Methyldiethylcyclohexandiisocyanat, Phenylendiisocyanat, Toluylendiisocyanat, Bis(isocyanatophenyl)methan, Propandiisocyanat, Butandiisocyanat, Pentandiisocyanat, Hexandiisocyanat, wie Hexamethylendiisocyanat oder 1,5-Diisocyanato-2-methylpentan, Heptandiisocyanat, Octandiisocyanat, Nonandiisocyanat, wie 1,6-Diisocyanato-2,4,4-trimethylhexan oder 1,6-Diisocyanato-2,2,4-trimethylhexan, Nonantriisocyanat, wie 4-Isocyanatomethyl-1,8-octandiisocyanat, Decandi- und triisocyanat, Undekandi- und -triisocyanat, Dodecandi- und -triisocyanate, Isophorondiisocyanat, Dicyclohexylmethan-4,4'-diisocyanat, Isocyanatomethylmethylcyclohexylisocyanat, 1,3-Bis(isocyanatomethyl)cyclohexan oder 1,4-Bis(isocyanatomethyl)cyclohexan.

Bevorzugte Monoisocyanate sind (Meth)acryloylisocyanat und (Meth)acryl-C2-C8-alkylisocyanate (d.h. (Meth)acrylalkylisocyanate mit Alkylspacem, die über 2 bis 8, besonders bevorzugt 2 bis 6 Kohlenstoffatome verfügen), dabei wiederum bevorzugt ist (Meth)acrylethylisocyanat (2-Isocyanatoethyl(meth)acrylat).

Außerdem haben sich als Komponente B) Monoisocyanate vorteilhaft erwiesen, die Umsetzungsprodukte sind aus Amino- bzw. Hydroxyalkyl(meth)acrylaten, deren Alkylspacer über 1 bis 12, bevorzugt 2 bis 8, besonders bevorzugt 2 bis 6 Kohlenstoffatome verfügen, und Diisocyanaten.

Vorzugsweise wird dazu ein vorstehend genanntes Diisocyanat äquimolar mit einer (oben als bevorzugt gekennzeichneten) Amino- oder Hydroxylalkylverbindung eines (Meth)acrylats umgesetzt, wobei die Hydroxylalkylverbindungen wiederum vorzugsweise gewählt sind aus der Gruppe bestehend aus Hydroxyethyl(meth)acrylat, Hydroxypropyl(meth)acrylat, Hydroxybutyl(meth)acrylat, Hydroxypentyl(meth)acrylat, Hydroxyhexyl(meth)acrylat.

Als Beispiele seien die Umsetzungsprodukte im molaren Verhältnis von 1 : 1 von Hydroxyethylmethacrylat mit Isophorondiisocyanat, Dicyclohexylmethan-4,4'-diisocyanat oder Hexamethylendiisocyanat genannt.

Im Folgenden wird die Erfindung zunächst für Monomere umfassend tricyclische Strukturelemente Q am Beispiel von Tricyclo[5.2.1.0$^{2,6}$]decan (TCD) - Derivaten im Detail erläutert.

1.) Ausgehend vom Bis(hydroxymethyl)tricyclo[5.2.1.0$^{2,6}$]decan (TCD-Diol)

Bis(hydroxymethyl)tricyclo[5.2.1.0$^{2,6}$]decan ist kommerziell erhältlich, beispielsweise als

Dicidolgemisch der isomeren Verbindungen 3,8-Bis(hydroxymethyl)tricyclo[5.2.1.0$^{2,6}$]decan und 4,8-Bis(hydroxymethyl)tricyclo[5.2.1.0$^{2,6}$]decan sowie 3,9-Bis(hydroxymethyl)tricyclo[5.2.1.0$^{2,6}$]decan und 4,9-Bis(hydroxymethyl)tricyclo[5.2.1.0$^{2,6}$]decan.

Die Bis(hydroxymethyl)tricyclo[5.2.1.0$^{2,6}$]decane können, ausgehend von Dicyclopentadien (Tricyclo[5.2.1.0$^{2,6}$]deca-3,8-dien), synthetisiert werden. Dicyclopentadien ist präparativ leicht in einer Diels-Alder Reaktion durch Dimerisierung von Cyclopentadien zugänglich. Hydroformylierung von Dicyclopentadien ergibt dann das Bis(hydroxymethyl)tricyclo[5.2.1.0$^{2,6}$]decan. Je nach Synthesepfad können gezielt an unterschiedlichen Positionen substituierte Bis(hydroxymethyl)tricyclo[5.2.1.0$^{2,6}$]decane erhalten werden. So werden in den Druckschriften JP 7-206740, EP 1 112 995 B1 oder EP 0 049 631 B1 Vorschriften angegeben, wie beispielsweise das 8,9-Bis(hydroxymethyl)tricyclo[5.2.1.0$^{2,6}$]decan herstellbar ist. Die DE 103 52 260 B3 beschreibt dagegen Verfahren zur Herstellung von 3(4), 8(9)-Bis(hydroxymethyl)tricyclo[5.2.1.0$^{2,6}$]decan. Die Notation der Positionen der Hydroxymethylgruppen 3(4), 8(9) bedeutet 3 oder 4, 8 oder 9.

Das im Handel erhältliche und als Ausgangsverbindung zur Herstellung erfindungsgemäß einzusetzender Monomere einsetzbare Bis(hydroxymethyl)tricyclo[5.2.1.0$^{2,6}$]decan enthält somit Hydroxymethylgruppen sowohl an den Positionen 3 oder 4 als auch in den Stellungen 8 oder 9. Es ist nun möglich, durch Anlagerung von Alkylenoxiden, im Allgemeinen in Mengen von 1 bis 10 mol, insbesondere von Ethylenoxid, Propylenoxid, Butylenoxid, etc. in Gegenwart basischer Katalysatoren nach bekannten Verfahren die entsprechenden Polyetherpolyole zu synthetisieren. Die EP 0 023 686 B1 enthält hierzu genaue Herstellvorschriften.

Die Umsetzung der 3(4), 8(9)-Bis(hydroxymethyl)tricyclo[5.2.1.0$^{2,6}$]decane mit Isocyanaten zu den entsprechenden Urethanen ist ebenfalls bekannt. So beschreibt die DE 35 22 006 A1 die Reaktion des 3(4), 8(9)-Bis(hydroxymethyl)tricyclo[5.2.1.0$^{2,6}$]decans mit 2-Isocyanatoethylmethacrylat. 2-Isocyanatoethylmethacrylat ist kommerziell erhältlich oder kann gemäß der Herstellvorschrift aus der DE 33 38 077 A1 durch Phosgenierung von Dihydrooxazinen synthetisiert werden.

Das erhaltene Reaktionsprodukt (Formel (1)) von 2-Isocyanatoethylmethacrylat mit 3(4), 8(9)-Bis(hydroxymethyl)tricyclo[5.2.1.0$^{2,6}$]decan weist in einer Formulierung nach Aushärtung einen niedrigen Reaktionsschrumpf und eine hohe mechanische Festigkeit auf.

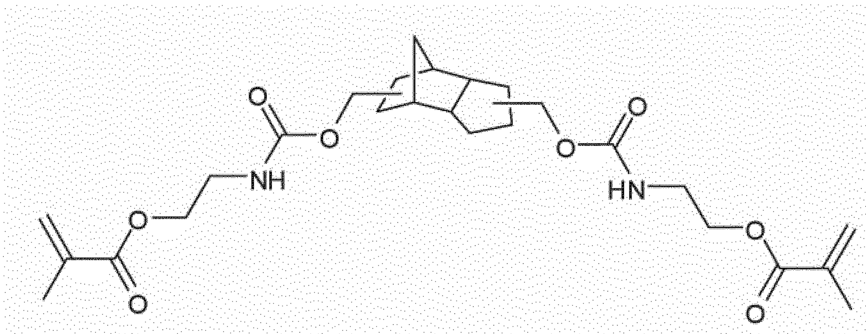

Formel (1)

Das Urethan der Formel (1) besitzt noch zwei reaktionsfähige Wasserstoffatome am Stickstoff, die nun in einer zweiten Umsetzungsstufe mit überschüssigem Isocyanat weiter abreagiert werden unter Bildung einer erfindungsgemäß einzusetzenden Verbindung. Es bildet sich hierbei zunächst das Allophanat der Formel (2) als tetrafunktitinalisierte, radikalisch vernetzbare Verbindung. Auch dieses Monomer weist wiederum noch umsetzungsfähige Wasserstoffatome am Stickstoff auf, die bei Reaktion mit weiterem Isocyanat das hexafunktionalisierte, radikalisch härtbare Allophanate der Formel (3) bilden.

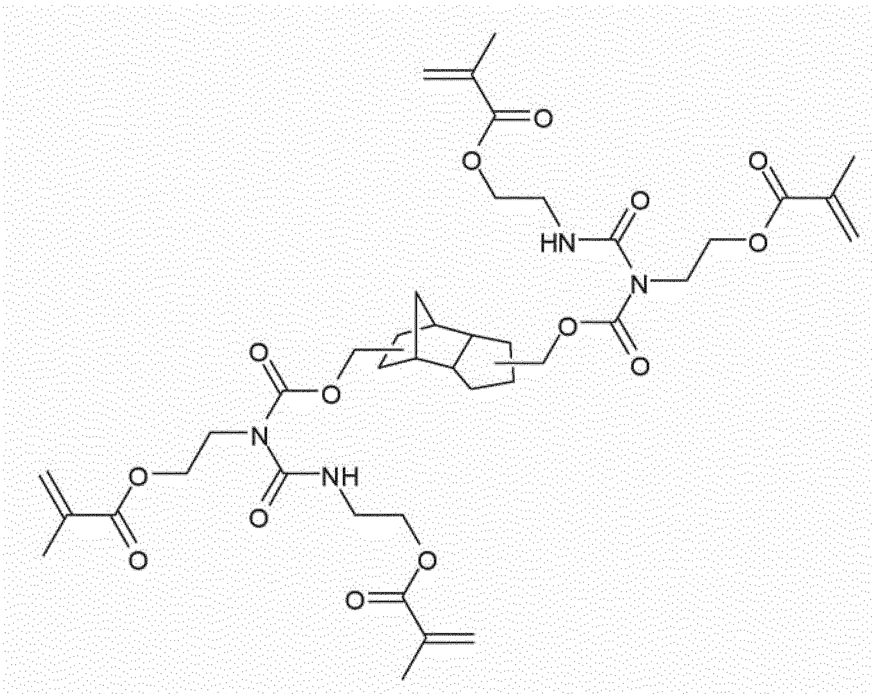

Formel (2)

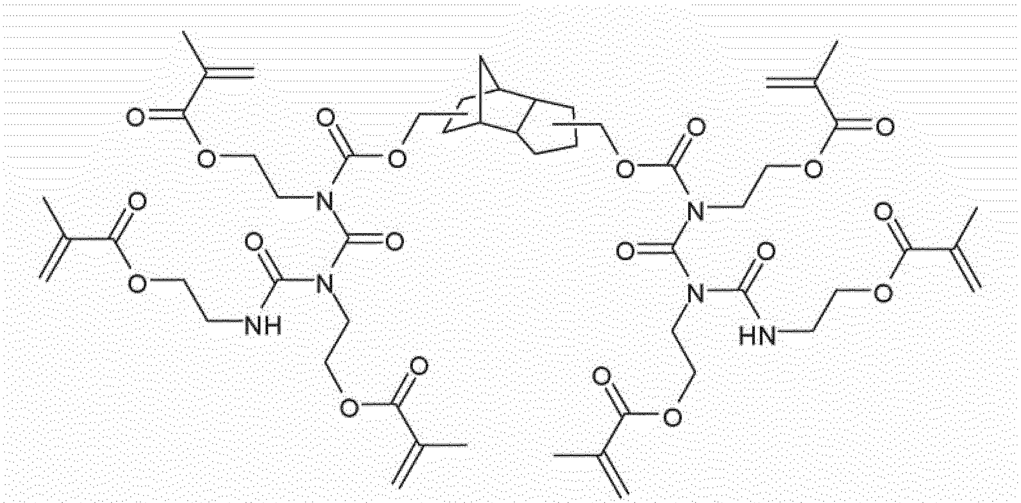

Formel (3)

Alternativ kann das 3(4), 8(9)-Bis(hydroxymethyl)tricyclo[5.2.1.0$^{2,6}$]decan auch mit Methacryloylisocyanat zur Reaktion gebracht werden. Methacryloylisocyanat ist kommerziell oder durch Umsetzung von Methacrylamid mit Oxalylchlorid erhältlich, wie in der EP 0 143 613 B1 beschrieben. Durch Umsetzung von 3(4), 8(9)-Bis(hydroxymethyl)tricyclo[5.2.1.0$^{2,6}$]decan mit Methacryloylisocyanat wird eine Verbindung der Formel (4) erhalten:

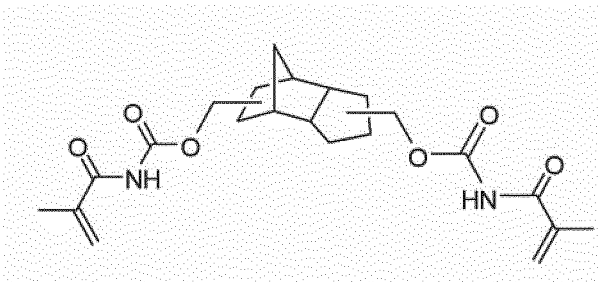

Formel (4)

Die verbleibenden reaktiven Wasserstoffatome am Stickstoff der Verbindung der Formel (4) können anschließend wiederum in Isocyanatreaktionen zu Allophanaten umgesetzt werden. Beispielhaft sei hier das Reaktionsprodukt mit 2-Isocyanatoethylmethacrylat (Formel (5)) gezeigt.

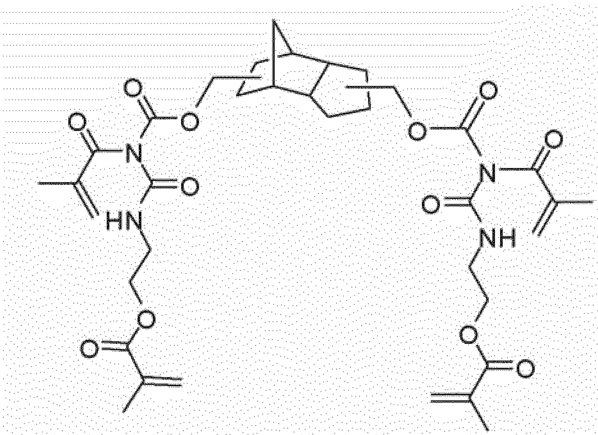

Formel (5)

2.) Ausgehend von 3(4), 8(9)-Bis(Carbonsäure)tricyclo[5.2.1,0$^{2,6}$]decan

Das 3(4), 8(9)-Bis(Carbonsäure)tricyclo[5.2.1.0$^{2,6}$]decan ist durch einfache Oxidation des kommerziell erhältlichen 3(4), 8(9)-Bis(formyl)tricyclo[5.2.1.0$^{2,6}$]decans herstellbar. Umsetzung der Dicarbonsäure mit 2-Isocyanatoethylmethacrylat ergibt das Amid der Formel (8):

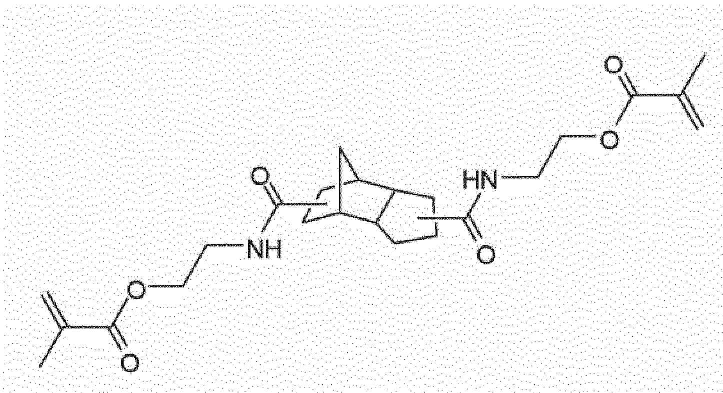

Formel (8)

Weitere Umsetzung der beiden reaktionsfähigen Amid-Wasserstoffatome des Amids der Formel (8) mit 2-Isocyanatoethylmethacrylat ergibt den Acylharnstoff der Formel (9).

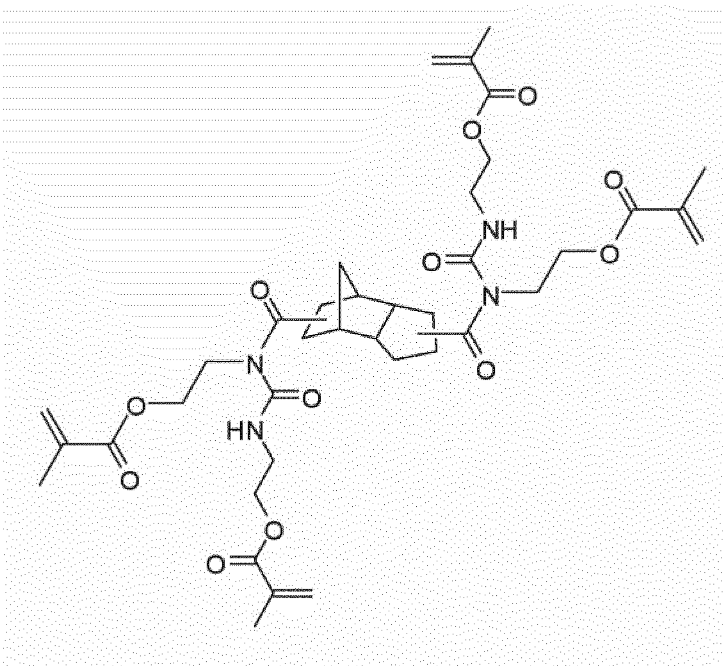

Formel (9)

Wird 3(4), 8(9)-Bis(Carbonsäure)tricyclo[5.2.1.0$^{2,6}$]decan mit Methacryloylisocyanat umgesetzt, ergibt sich das Imid der Formel (10). Die reaktiven Wasserstoffatome am Stickstoff können auch hier weiter in Isocyanatreaktionen umgesetzt werden.

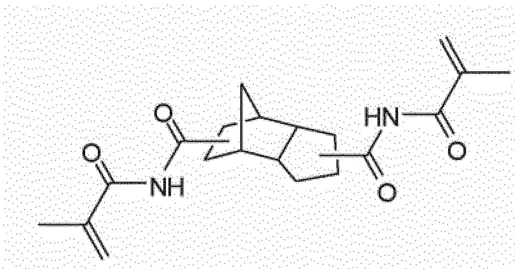

Formel (10)

3.) Ausgehend von 3(4), 8(9)-Bis(isocyanatomethyl)tricyclo[5.2.1,0$^{2,6}$]decan

Das 3(4), 8(9)-Bis(isocyanatomethyl)tricyclo[5.2.1.0$^{2,6}$]decan ist an sich bekannt und zählt zu den herkömmlichen, in industriellen Anwendungen eingesetzten Diisocyanatverbindungen (siehe hierzu die DE 37 03 120 A1 sowie die WO 2009/065873 A2). Die Durchführung der zweiten Umsetzungsstufe der Isocyanat-Alkoholreaktion kann nicht nur ausgehend vom Tricyclodecandiol mit dem Isocyanatoethylmethacrylat, sondem auch ausgehend vom Tricyclodecandiisocyanat und Hydroxyethylmethacrylat eingeleitet werden. Durch stöchiometrische Umsetzung der beiden Reaktanden erhält man das Urethan der Formel (11).

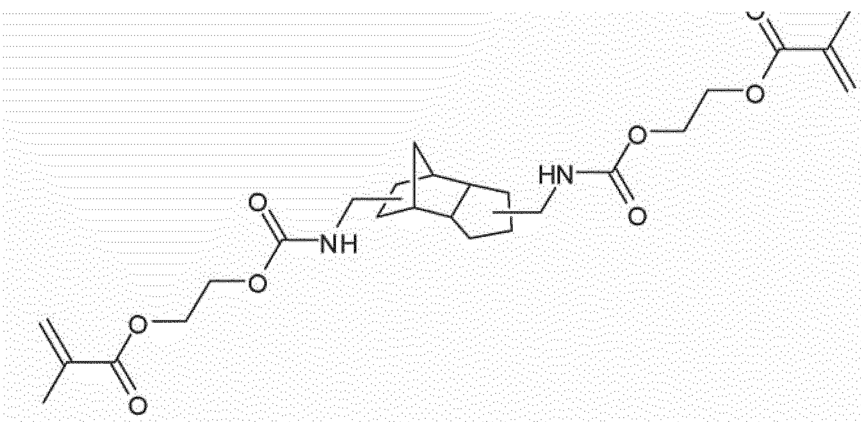

Formel (11)

Auch dieses Carbamat (Formel (11)) weist zwei reaktionsfähige Wasserstoffatome am Stickstoff auf, die mit einem Überschuss an Bis(isocyanatomethyl)tricyclo[5.2.1.0$^{2,6}$]decan zu dem Diisocyanat der Formel (12) weiter umgesetzt werden kann.

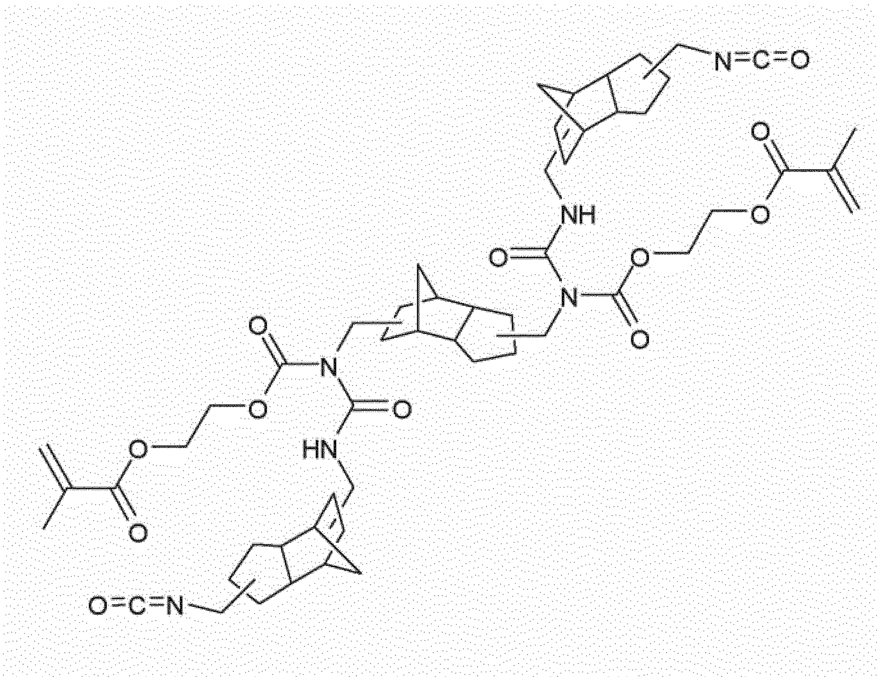

Formel (12)

Umsetzung des Allophanat-Diisocyanats (Formel (12)) mit Methacrylsäure liefert die Verbindung der Formel (13).

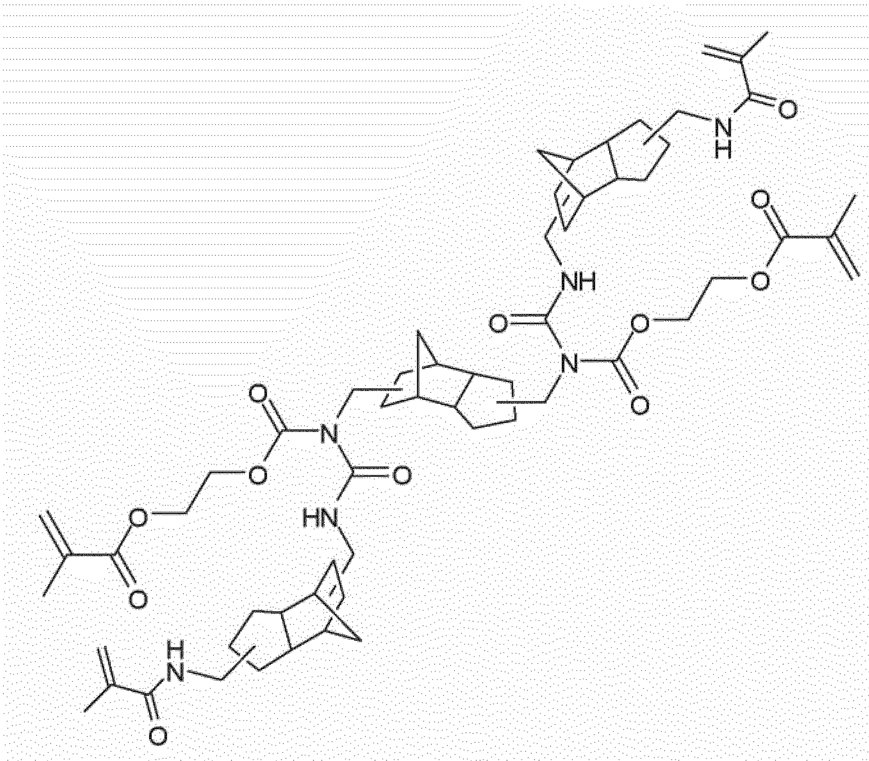

Formel (13)

Anstelle von Hydroxyethylmethacrylat können in den oben exemplarisch dargelegten Umsetzungen auch andere Hydroxylverbindungen von (Meth)acrylaten eingesetzt werden, wobei auch Gemische von Acrylaten und Methacrylaten verwendet werden können. So kann - analog dem obigen Beispiel - 3(4), 8(9)-Bis(isocyanatomethyl)-tricyclo[5.2.1.$0^{2,6}$]decan umgesetzt werden. Dabei bevorzugte Hydroxylverbindungen von (Meth)acrylaten sind die oben explizit genannten.

Diese Verbindungen weisen sowohl (Meth)acrylatgruppen als auch Hydroxygruppen auf. Letztere können mit Isocyanatgruppen in der oben für die Reaktion zwischen Hydroxyethylmethacrylat und 3(4), 8(9)-Bis(isocyanatomethyl)-tricyclo[5.2.1.$0^{2,6}$]decan beschriebenen Weise reagieren. So lässt sich in einem einzigen Reaktionsschritt ein hoher Funktionalisierungsgrad erreichen.

3(4), 8(9)-Bis(isocyanatomethyl)tricyclo[5.2.1.$0^{2,6}$]decan kann mit 2-Carbonsäureethylmethacrylat zum entsprechenden Amid der Formel (16) umgesetzt werden.

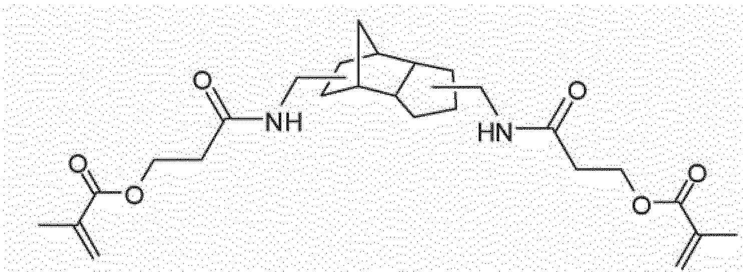

Formel (16)

Umsetzung des Amids der Formel (16) mit 2-Isocyanatoethylmethacrylat liefert den Acylharnstoff der Formel (17).

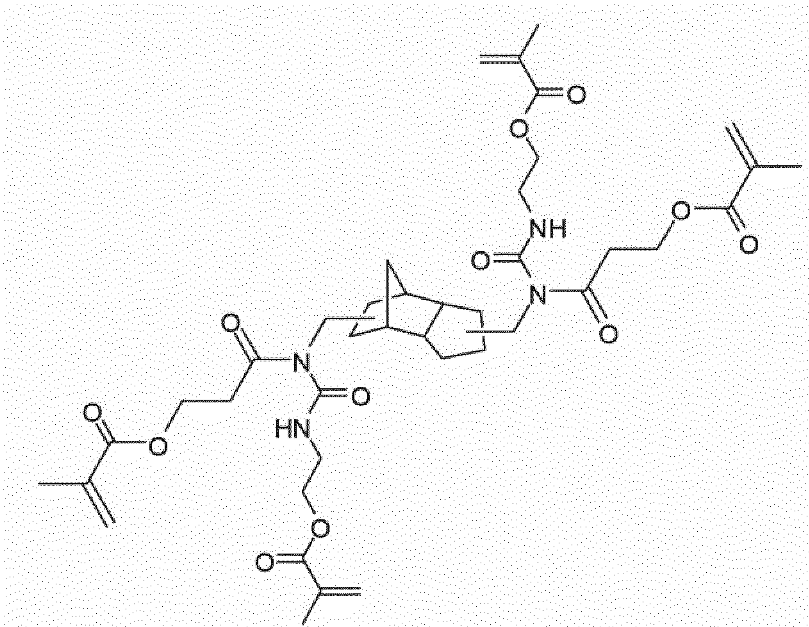

Formel (17)

Das Amid der Formel (16) kann auch mit einem Überschuss an 3(4), 8(9)-Bis(isocyanatomethyl)tricyclo[5.2.1.$0^{2,6}$]decan zum entsprechenden Isocyanat umgesetzt werden, wobei das gebildete Isocyanat mit Hydroxyethylmethacrylat weiter zum vernetzbaren Monomer der Formel (18) weiter umgesetzt wird.

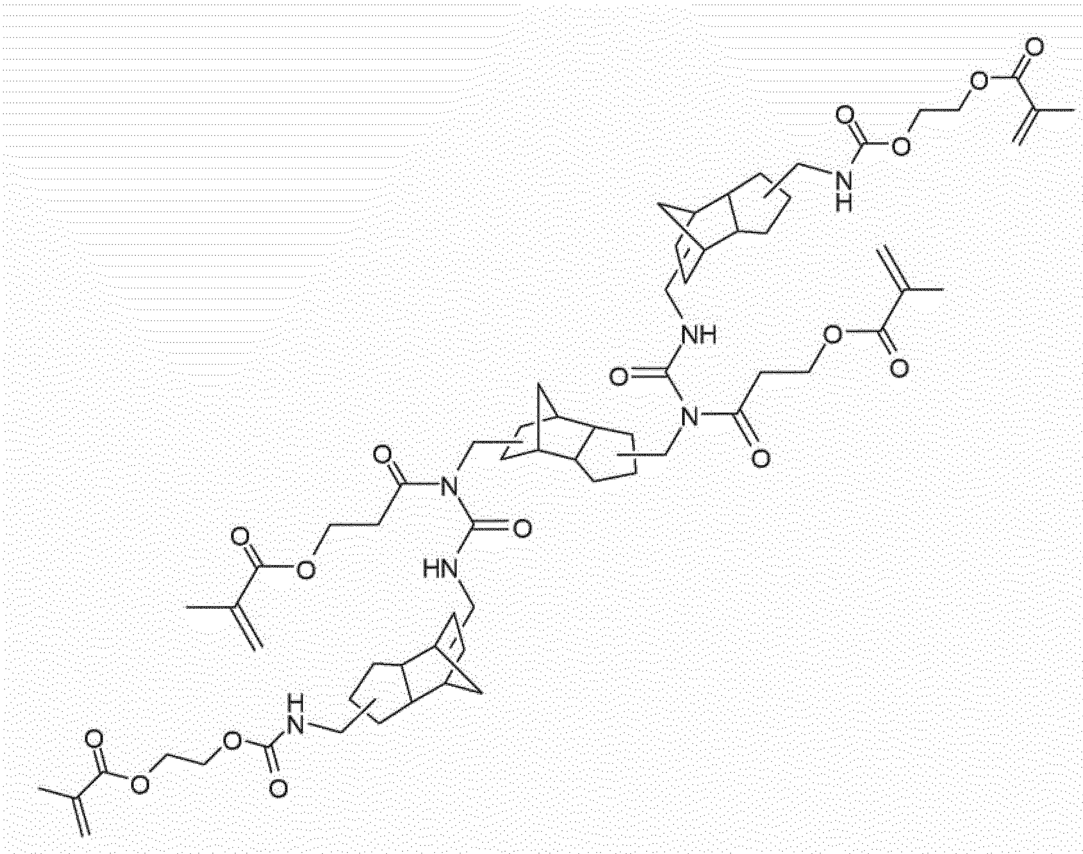

Formel (18)

Wird 3(4), 8(9)-Bis(isocyanatomethyl)tricyclo[5.2.1.0$^{2,6}$]decan mit 2-Methacryloylaxyethylhydrogensuccinat umgesetzt, so erhält man das Amid der Formel (19), das weiter mit 2-Isocyanatoethylmethacrylat zum Acylharnstoff der Formel (20) abreagiert wird.

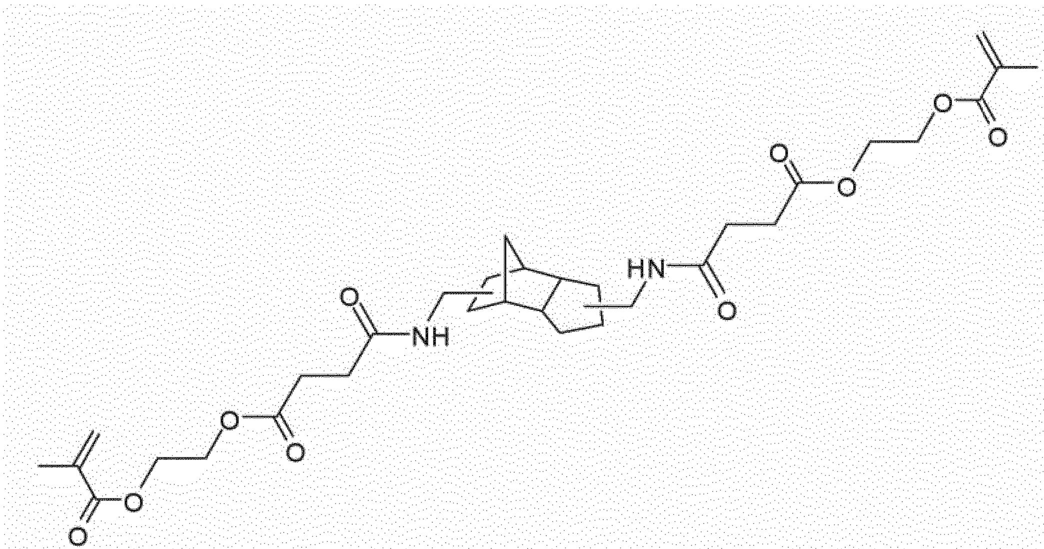

Formel (19)

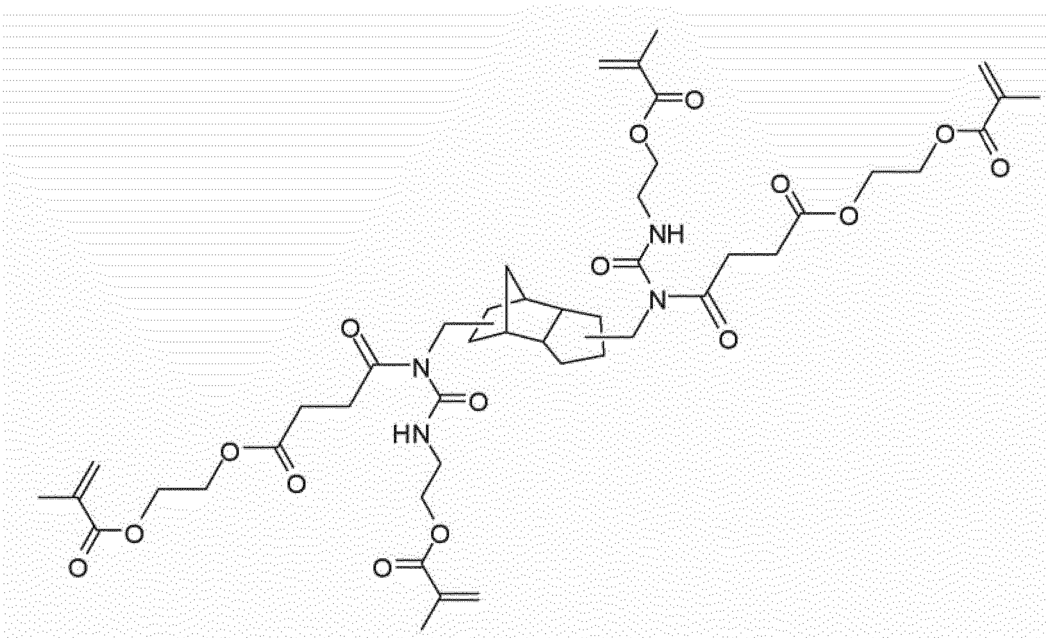

Formel (20)

Weitere geeignete Carbonsäuremethacrylate können durch Reaktionen von Di- oder Tetracarbonsäuremono- oder dianhydrid mit geeigneten OH-funktionalisierten, härtbaren Verbindungen wie beispielsweise 2-Hydroxyethylmethacrylat erhalten werden.

4.) Ausgehend von 3(4), 8(9)-Bis(Aminomethyl)tricyclo[5.2.1.$0^{2,6}$]decan

Das 3(4), 8(9)-Bis(Aminomethyl)tricyclo[5.2.1.$0^{2,6}$]decan ist an sich bekannt oder kann beispielsweise durch Umsetzung der entsprechenden Tosylate mit Ammoniak hergestellt werden. Reaktion des 3(4), 8(9)-Bis(Aminomethyl)tricyclo[5.2.1.$0^{2,6}$]decans mit 2-Isocyanatoethylmethacrylat ergibt die aus der EP 0 209 700 A2 bekannte Harnstoffverbindung der Formel (26).

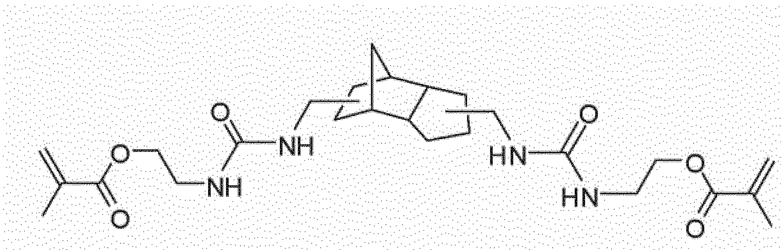

Formel (26)

Auch hier befinden sich noch aktive, reaktionsfähige Wasserstoffatome am Stickstoff, die mit einem Überschuss an Isocyanat beispielsweise zum Biuret der Formel (27) reagieren.

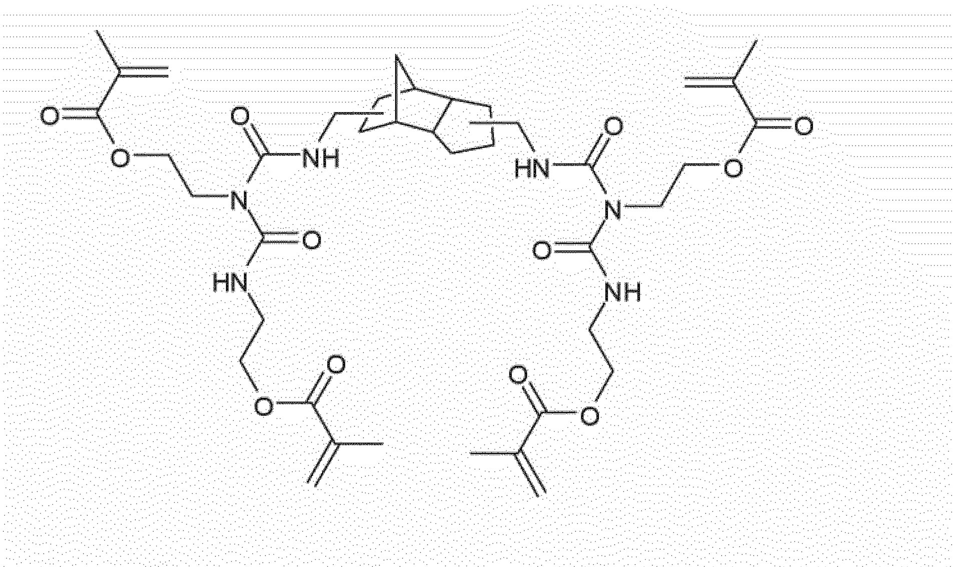

Formel (27)

Das 3(4), 8(9)-Bis(Aminomethyl)tricyclo[5.2.1.0$^{2,6}$]decan kann auch mit Methacryloylisocyanat zum entsprechenden Acylharnstoff umgesetzt werden. Die weitere Reaktion der verbleibenden reaktiven am Stickstoff befindlichen Wasserstoffatome mit Methacryloylisocyanat liefert das Biuret der Formel (28).

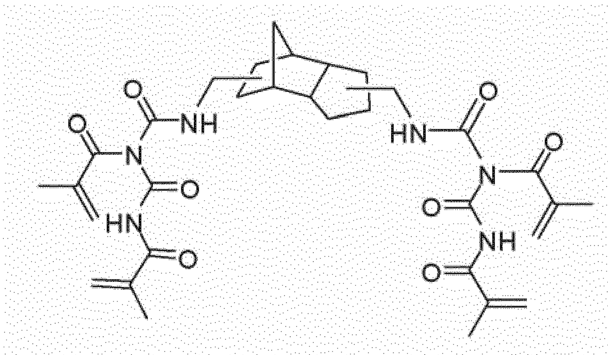

Formel (28)

Analog zu den vorstehend beschriebenen Monomeren, welche ein vom Tricyclo[5.2.1.0$^{2,6}$]decan abgeleitetes polyalicyclisches Strukturelement Q umfassen, können auch Monomere hergestellt werden, welche ein vom Tricyclo[3.3.1.1$^{3,7}$]decan (Adamantan) abgeleitetes polyalicyclisches Strukturelement Q umfassen. Als Beispiele seien folgende Reaktionsprodukte gezeigt:

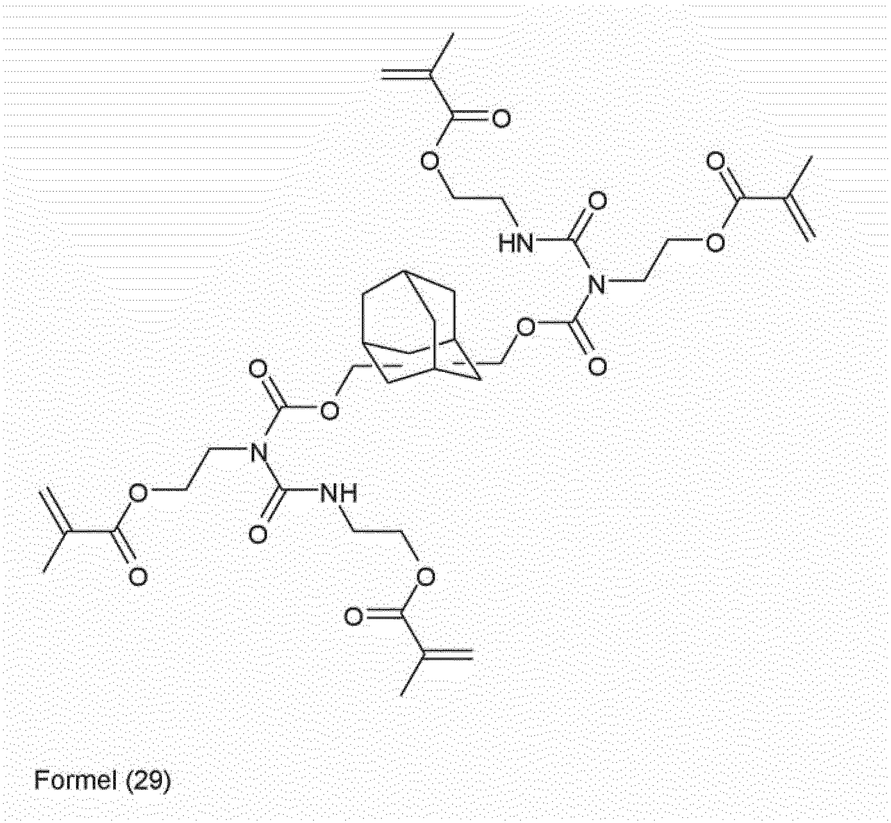

Formel (29)

Die Umsetzung der Verbindung der Formel (11) mit Diisocyanatoadamantan [(Bis(isocyanatomethyl)tricyclo[3.3.1.1$^{3,7}$]decan liefert ein erfindungsgemäß einzusetzendes Monomer, dessen Molekül zwei voneinander verschiedene polyalicyclische Strukturelemente Q umfasst, wie die nachfolgenden Strukturformel der Verbindung der Formel (69) zeigt.

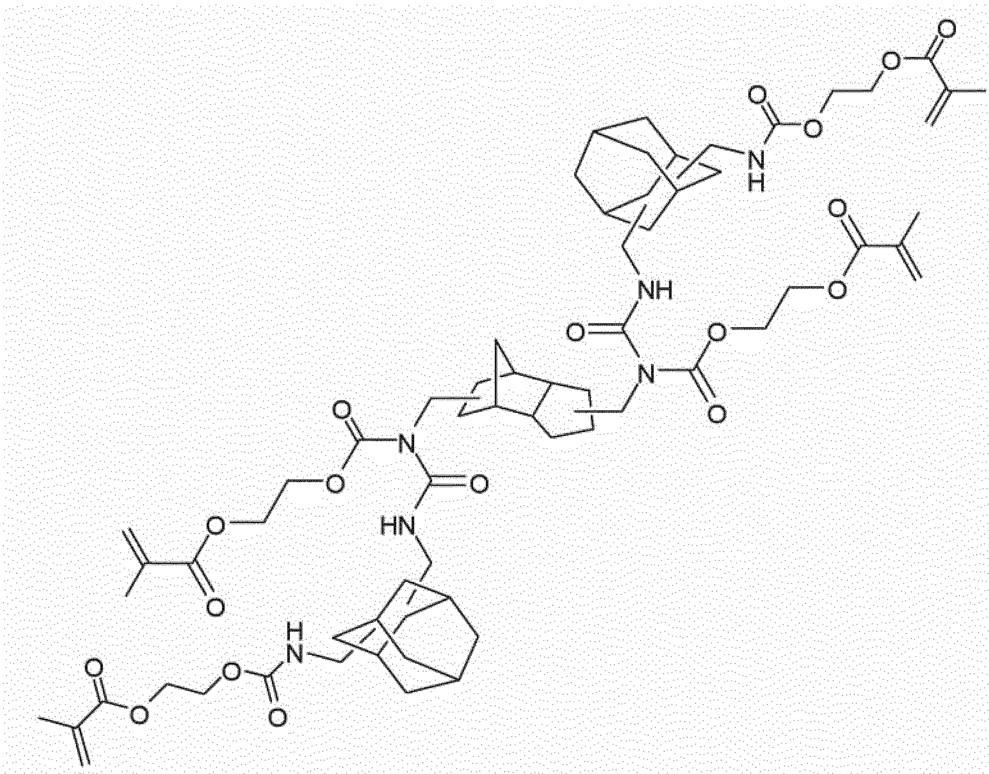

Formel (69)

Komponente (a2): Monofunktionalisierte C1-C5-Alkylmethacrylate

Komponente (a2) besteht aus ein, zwei oder mehr radikalisch polymerisierbaren monofunktionalisierten Monomeren aus der Gruppe bestehend aus Methylmethacrylat, Ethylmethacrylat, Propylmethacrylat, Butylmethacrylat und Pentylmethacrylat.

Besonders bevorzugt sind erfindungsgemäße Lackzusammensetzungen zur Anwendung in einem therapeutischen Verfahren als Dentalmaterial umfassend eine Komponente (a2), die Methylmethacrylat umfasst oder aus Methylmethacrylat besteht, da dann besonders gute Resultate im Sinne der vorliegenden Erfindung erzielt wurden.

Die Gesamtmenge der Komponente (a2) beträgt vorzugsweise zumindest 5 Gew.-%, bevorzugt zumindest 8 Gew.-%, jeweils bezogen auf die Gesamtmasse der erfindungsgemäßen Lackzusammensetzung.

Die Gesamtmenge der Komponente (a2), insbesondere an Methylmethacrylat, liegt vorzugsweise im Bereich von 5 bis 45 Gew.-%, bevorzugt im Bereich von 8 bis 40 Gew.-%, jeweils bezogen auf die Gesamtmasse der erfindungsgemäßen Lackzusammensetzung zur Anwendung in einem therapeutischen Verfahren als Dentalmaterial.

Komponente (a3): Monomere mit drei oder mehr (Meth)acrylatgruppen

Die optionale Komponente (a3) besteht aus ein, zwei oder mehr radikalisch polymerisierbaren Monomeren aus der Gruppe bestehend aus weiteren radikalisch polymerisierbaren Monomeren mit drei, vier, fünf, sechs oder mehr (Meth)acrylatgruppen, vorzugsweise mit drei bis sechs Acrylatgruppen.

Bevorzugt als Komponente (a3) sind Acrylat-Monomere, da diese deutlich schneller in der Polymerisation als die entsprechenden Methacrylate sind, d.h. einen raschen Polymerisationverlauf und eine raschere Härtung erlauben.

Besonders bevorzugt sind Lackzusammensetzungen zur Anwendung in einem therapeutischen Verfahren als Dentalmaterial umfassend eine Komponente (a3), die Dipentaerythritolpentaacrylat umfasst oder aus Dipentaerythritolpentaacrylat besteht, da damit besonders gute Resultate im Sinne der vorliegenden Erfindung erzielt wurden.

Erfindungsgemäß bevorzugte Lackzusammensetzungen enthalten daher sowohl Methylmethacrylat (Komponente (a2) als auch Dipentaerythritolpentaacrylat (Komponente (a3).

Mit solchen Lackzusammensetzungen wurden besonders gute Resultate im Sinne der vorliegenden Erfindung erzielt.

Die Gesamtmenge der Komponente (a3) beträgt vorzugsweise zumindest 5 Gew.-%, bevorzugt zumindest 8 Gew.-%, jeweils bezogen auf die Gesamtmasse der erfindungsgemäßen Lackzusammensetzung zur Anwendung in einem therapeutischen Verfahren als Dentalmaterial.

Die Gesamtmenge der Komponente (a3), insbesondere an Dipentaerythritolpentaacrylat, liegt vorzugsweise im Bereich von 5 bis 65 Gew.-%, bevorzugt im Bereich von 8 bis 60 Gew.-%, jeweils bezogen auf die Gesamtmasse der erfindungsgemäßen Lackzusammensetzung zur Anwendung in einem therapeutischen Verfahren als Dentalmaterial.

Komponente (a4): weitere radikalisch polymerisierbare Monomere aus der Gruppe bestehend aus Acrylaten und Methacrylaten, vorzugsweise der Gruppe der Methacrylate

Den nicht zu Komponenten (a1), (a2) und (a3) zählbaren optionalen Bestandteil der matrixbildenden Monomerenmischung bilden radikalisch polymerisierbare Monomere ausgewählt aus der Gruppe bestehend aus Acrylaten und Methacrylaten.

Die radikalisch polymerisierbaren Monomere der Komponente (a4) weisen vorzugsweise mindestens zwei ethylenische Gruppen auf.

In der Patentliteratur ist eine Vielzahl von Diacrylat- und Dimethacrylat-Monameren genannt (beispielsweise auch in der DE 39 41 629 A1, welche im Wege der Verweisung Bestandteil der vorliegenden Anmeldung ist, insbesondere die Offenbarung im Bereich von Spalte 6, Zeile 15 bis Spalte 8, Zeile 10), die sich zum Einsatz in einer erfindungsgemäßen Lackzusammensetzung zur Anwendung in einem therapeutischen Verfahren als Dentalmaterial eignen.

Erfindungsgemäße Lackzusammensetzungen zur Anwendung in einem therapeutischen Verfahren als Dentalmaterial können als Komponente (a4) auch ein oder mehrere Dimethacrylat-Monomere enthalten, vorzugsweise gewählt aus der Gruppe bestehend aus Ethylenglykoldimethacrylat (EGDMA), 1,6-Hexandioldimethacrylat (HEDMA), Triethylenglykoldimethacrylat (TEDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat und Glycerindimethacrylat.

Bisphenol-A-Glycidyl-Methacrylat (Bis-GMA) kann zwar eingesetzt werden, vorzugsweise enthält eine erfindungsgemäße Lackzusammensetzung zur Anwendung in therapeutischen Verfahren als Dentalmaterial jedoch nicht die Verbindung Bis-GMA. Vorzugsweise ist eine erfindungsgemäße Lackzusammensetzung zur Anwendung in einem therapeutischen Verfahren als Dentalmaterial frei von sämtlichen Verbindungen mit einem Bisphenol-A-Strukturelement.

Die radikalisch polymerisierbaren Monomere der Komponente (a4), die somit nicht zu Komponente (a1) zählen, können auch Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Als weiterer Bestandteil können auch lichthärtbare Acrylat- oder Methacrylat-Monomere auf Basis von Polysiloxanen verwendet werden, wie sie beispielsweise in der DE 199 03 177 oder in der DE 44 16 857 beschrieben sind.

Eine erfindungsgemäße Lackzusammensetzung zur Anwendung in einem therapeutischen Verfahren als Dentalmaterial kann ferner in Komponente (a4) ein oder mehrere säuregruppenhaltige Acrylate- und/oder Methacrylat-Monomere enthalten. Solche säuregruppenhaltigen Monomere können vorzugsweise eine Carbonsäure-, eine Phosphorsäure-, eine Phosphonsäure-, eine Sulfonsäure- und/oder eine Thiophosphorsäurefunktion aufweisen. Das Monomer kann, eine oder eine Vielzahl von Säurefunktionen in einem Molekül enthalten.

Geeignete eine Phosphorsäuregruppe enthaltende Monomere sind beispielsweise 2-(Meth)acryloyloxyethyldihydrogenphosphat, Bis[2-(meth)acryloyloxyethyl]hydrogenphosphat, 2-(Meth)acryloyloxyethylphenylhydrogenphosphat, 6-(Meth)-acryloyloxyhexyldihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)heptyl]hydrogenphosphat.

Geeignete eine Carbonsäuregruppe enthaltende Monomere sind beispielsweise 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Weitere geeignete säuregruppentragende Monomere sind beispielsweise in der EP 0 980 682 A1 (insbesondere Absätze [0059] bis [0065]) oder der EP 0 948 955 A1 (insbesonde- re Absätze [0031] bis [0034]) genannt

Ferner können auch die Phosphorsäureester mit Glycerindimethacrylat oder mit Hydroxyethylmethacrylat oder mit Hydraxypropylmethacrylat verwendet werden.

Die genannten Monomere können einzeln oder in Gemischen eingesetzt werden.

Bestandteil (b): Initiatoren und/oder Katalysatoren

Eine erfindungsgemäße Lackzusammensetzung zur Anwendung in einem therapeutischen Verfahren als Dentalmaterial ist vorzugsweise lichthärtbar und/oder chemisch härtbar. Bevorzugt ist eine erfindungsgemäße Lackzusammensetzung, wobei Bestandteil (b) einen oder mehrere Lichthärtungsinitiatoren und/oder einen oder mehrere Initiatoren zur chemischen Aushärtung umfasst oder aus diesen besteht.

Die Gesamtmenge der Komponente (b), insbesondere an Phosphinoxid-Initiatoren, liegt vorzugsweise im Bereich von 1 bis 6 Gew.-%, bevorzugt im Bereich von 1,5 bis 5 Gew.-%, jeweils bezogen auf die Gesamtmasse der erfindungsgemäßen Lackzusammensetzung zur Anwendung in einem therapeutischen Verfahren als Dentalmaterial.

Bevorzugte erfindungsgemäße Lackzusammensetzung zur Anwendung in einem therapeutischen Verfahren als Dentalmaterial sind lichthärtbar (photohärtbar) und umfassen Lichthärtungsinitiatoren. Beispiele für einen Lichthärtungsinitiator schließen Substanzen ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2

Weitere geeignete Initiatoren sowie Initiatorkombinationen sind in der DE 601 16 142 beschrieben

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen Lackzusammensetzung zur Anwendung in einem therapeutischen Verfahren als Dentalmaterial bewirken können.

Das Absorptionsmaximum von Campherchinon (CC) liegt bei ca. 470 nm und somit im Bereich des blauen Lichts. Campherchinon (CC) zählt zu den $PI_2$-Initiatoren und wird regelmäßig zusammen mit einem Coinitiator eingesetzt.

Vorzugsweise enthält eine erfindungsgemäße Lackzusammensetzung zur Anwendung in einem therapeutischen Verfahren als Dentalmaterial die Kombination eines alpha-Diketons und eines aromatischen tertiären Amins, bevorzugt ist die Kombination von Campherchinon (CC) und Ethyl-*p*-*N*,*N*-dimethylaminobenzoat (DABE).

Besonders bevorzugte Initiatoren als Komponente (b) einer erfindungsgemäßen Lackzusammensetzung zur Anwendung in einem therapeutischen Verfahren als Dentalmaterial sind Phosphinoxide, insbesondere Acylphosphinoxide.

Mit diesen Phosphinoxid-Initiatoren lassen sich erfindungsgemäße Lackzusammensetzungen zur Anwendung in einem therapeutischen Verfahren als Dentalmaterial erzielen, die schmierschichtfrei aushärten. Weiter bevorzugte Phosphinoxide sind Phenyl-bis(2,4,6-trimethylbenzoyl)phosphinoxid und/oder 2,4,6-Trimethylbenzoyldiphenylphosphinoxid.

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinaxid.

Bezüglich der Strukturen geeigneter Phosphinoxide zum Einsatz in einer erfindungsgemäßen Lackzusammensetzung zur Anwendung in einem therapeutischen Verfahren als Dentalmaterial wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen

Die in diesen Druckschriften angegebenen Phosphinoxide eigenen sich besonders allein oder in Kombination mit dem System "alpha-Diketon/Amin" als Photopolymerisationsinitiatorsystem in einer erfindungsgemäßen Lackzusammensetzung zur Anwendung in einem therapeutischen Verfahren als Dentalmaterial.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer

Science and Technology, Vol. II, Elsevier Applied Science, London, New York 1993 beschrieben

Dem Fachmann sind diverse Initiatoren für eine chemische Aushärtung bekannt. Es sei insoweit exemplarisch auf die EP 1 720 506 verwiesen.

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die Peroxide und die Amine werden dabei auf zwei unterschiedliche Komponenten des Dentalmaterials aufgeteilt. Beim Mischen der aminhaltigen Komponente (sogenannte Basispaste) mit der peroxidhaltigen Komponente (sogenannte Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung.

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Geeignete Malonylsulfamide sind in der EP 0 059 451 beschrieben welche im Wege der Verweisung Bestandteil der vorliegenden Anmeldung ist. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzalsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Bestandteil (c): Füllstoffkomponente

Eine erfindungsgemäße Lackzusammensetzung zur Anwendung in einem therapeutischen Verfahren als Dentalmaterial kann einen Anteil einer Füllstoffkomponente (c) von 0 bis zu 60 Gew.-% enthalten, bezogen auf die Gesamtmasse der Lackzusammensetzung, wobei die Füllstoffkomponente einen, zwei oder mehrere Füllstoffe umfasst.

Entsprechend den vorstehenden und nachfolgenden Ausführungen wird auch eine Lackzusammensetzung (wie oben definiert) offenbart, umfassend

(c) 0 bis 60 Gew.-% einer Füllstoffkomponente, umfassend einen, zwei oder mehrere Füllstoffe ausgewählt aus der Gruppe bestehend aus

(c1) eine Gesamtmenge im Bereich von 0 bis 60 Gew.-% an nicht agglomerierten, vorzugsweise oberflächenmodifizierten, Nanopartikeln mit einer mittleren Partikelgröße kleiner 200 nm (vorzugsweise kleiner 100 nm, besonders bevorzugt kleiner 60 nm),

(c2) eine Gesamtmenge im Bereich von 0 bis 10 Gew.-% an Mikropartikeln mit einer mittleren Partikelgröße von 0,4 µm bis 10 µm, und

(c3) eine Gesamtmenge im Bereich von 0 bis 15 Gew.-% an weiteren Füllstoffe, vorzugsweise 0 bis 10 Gew.-%, bevorzugt 0 bis 5 Gew.-%, weitere Füllstoffe,

wobei sich die Gewichtsprozentangaben jeweils auf die Gesamtmasse der Lackzusammensetzung beziehen.

Die mittlere Partikelgröße $d_{50}$ der erfindungsgemäß optional einzusetzenden Füllstoffpartikel der Füllstoffkomponente (c) einer efindungsgemäßen Lackzusammensetzung zur Anwendung in einem therapeutischen Verfahren als Dentalmaterial wird mittels Lichtstreuung (Laserbeugung) bestimmt, vorzugsweise mit einem Partikelgrößenmessgerät Beckman Coulter LS 13320.

Die Füllstoffkomponente (c) kann vorzugsweise einen Füllstoff (c1) in Form nicht agglomerierter Nanopartikel mit einer mittleren Partikelgröße kleiner 200 nm umfassen.

Die Füllstoffkomponente (c) kann vorzugsweise einen Füllstoff (c1) in Form nicht agglomerierter, organisch oberflächenmodifizierter Nanopartikel mit einer mittleren Partikelgröße kleiner 200 nm umfassen.

Die Füllstoffkomponente (c) kann vorzugsweise alternativ oder zusätzlich einen Füllstoff (c2) in Form von Mikropartikeln mit einer mittleren Partikelgröße im Bereich von 0,4 μm bis 10 μm umfassen.

Die Füllstoffkomponente (c) umfasst in einer bevorzugten Ausgestaltung eine Mischung eines ersten Füllstoffs (c1) in Form nicht agglomerierter, organisch oberflächenmodifizierter Nanopartikel mit einer mittleren Partikelgröße kleiner 200 nm und eines zweiten Füllstoffs (c2) in Form von Mikropartikeln mit einer mittleren Partikelgröße im Bereich von 0,4 μm bis 10 μm.

Durch die Kombination von (c1) Nanopartikeln und (c2) Mikropartikeln in einer erfindungsgemäß bevorzugten Lackzusammensetzung zur Anwendung in einem therapeutischen Verfahren als Dentalmaterial wird eine vollständige und gleichmäßige Volumenfüllung der Lackzusammensetzung erzielt. Dadurch wird sowohl die Schrumpfung der Lackzusammensetzung beim Aushärten der Polymermatrix als auch die Empfindlichkeit der Lackzusammensetzung gegen Abrasion vermindert.

Insbesondere zur Einstellung der Rheologie umfasst eine erfindungsgemäße Lackzusammensetzung zur Anwendung in einem therapeutischen Verfahren als Dentalmaterial vorzugsweise eine geringe Menge an pyrogener Kieselsäure, bevorzugt in einer Menge im Bereich von 0,1 bis 5 Gew.-%, weiter bevorzugt im Bereich von 0,5 bis 2,5 Gew.-%, bezogen auf die Gesamtmasse der Lackzusammensetzung.

Komponente (c1): nicht agglomerierte Nanopartikel

Unter Nanopartikeln werden in Zusammenhang mit der vorliegenden Erfindung Partikel mit einer mittleren Partikelgröße von weniger als 200 nm verstanden. Vorzugsweise ist die mittlere Partikelgröße kleiner als 100 nm und besonders bevorzugt kleiner 60 nm.

Die Füllstoffkomponente (c) einer erfindungsgemäßen Lackzusammensetzung zur Anwendung in einem therapeutischen Verfahren als Dentalmaterial kann einen Füllstoff (c1) in einer Menge von 0 bis 60 Gew.-% umfassen, bezogen auf die Gesamtmasse der Lackzusammensetzung.

Der Anteil an nicht agglomerierten, optional organisch oberflächenmodifizierten, Nanopartikeln mit einer mittleren Partikelgröße kleiner 200 nm sollte dabei nicht mehr als 60 Gew.-% betragen, da die Verarbeitbarkeit der Lackzusammensetzung dann nicht mehr ausreichend ist; wegen des hohen Feststoffgehalts kann dann ihre Viskosität zu hoch werden.

Die Materialien für die einzusetzenden Nanopartikel sind vorzugsweise Oxide oder Mischoxide und bevorzugt ausgewählt aus der Gruppe bestehend aus Oxiden und Mischoxiden der Elemente Silizium, Titan, Yttrium, Strontium, Barium, Zirkonium, Hafnium, Niob, Tantal, Wolfram, Wismut, Molybdän, Zinn, Zink, Ytterbium, Lanthan, Cer, Aluminium und deren Mischungen. Die bevorzugten oxidischen Nanopartikel sind dabei wie dargelegt nicht agglomeriert.

In einer bevorzugten Ausgestaltung liegen die nanoskaligen Teilchen in nicht agglomerierter Form vor, beispielsweise dispergiert in einem Medium, vorzugsweise in monodisperser Form.

Um eine gute Einbindung der Nanopartikel in die Polymermatrix einer erfindungsgemäßen Lackzusammensetzung zur Anwendung in einem therapeutischen Verfahren als Dentalmaterial zu ermöglichen, sind die Oberflächen der Nanopartikel (vorzugsweise der bevorzugten oxidischen Nanopartikel) organisch modifiziert, d.h. ihre Oberflächen weisen organische Strukturelemente auf. Beispielhaft genannt sei die Oberflächenbehandlung der Füllstoffe mit einem Silan. Als Haftvermittler eignet sich besonders das Methacryloxypropyltrimethoxysilan.

Wird als Füllstoffkomponente (c) ausschließlich ein nanoskaliger Füllstoff (c1) eingesetzt, so können transparente Versiegelungsmaterialien erhalten werden (siehe WO 01/30307 A1 und WO 2007/028159 A2). Es werden erfindungsgemäß dann sehr gute Werte zur Abrasion erzielt.

Auch die erfindungsgemäßen transparenten dentalen Lackzusammensetzungen zur Anwendung in einem therapeutischen Verfahren als Dentalmaterial zeichnen sich gegenüber den Produkten aus dem Stand der Technik durch ein stark verbessertes Anfließverhalten (im nicht-ausgehärteten Zustand) am Zahnschmelz, insbesondere dem trockenen Zahnschmelz, sowie durch eine viel geringere Wasseraufnahme aus (im ausgehärteten Zustand).

Daher umfasst in einer bevorzugten Ausführungsform die Füllstoffkomponente (c) einer erfindungsgemäßen Lackzusammensetzung zur Anwendung in einem therapeutischen Verfahren als Dentalmaterial ausschließlich (c1) nicht agglomerierte Nanopartikel mit einer mittleren Partikelgröße kleiner 200 nm.

In einer weiteren bevorzugten Ausführungsform besteht die Füllstoffkomponente (c) einer erfindungsgemäßen Lackzusammensetzung zur Anwendung in einem therapeutischen Verfahren als Dentalmaterial ausschließlich aus (c1) nicht agglomerierten, organisch oberflächenmodifizierten Nanopartikeln mit einer mittleren Partikelgröße kleiner 200 nm.

Komponente (c2): Mikropartikel mit einer mittleren Partikelgröße im Bereich von 0,4 μm bis 10 μm

Unter Mikropartikeln werden in Zusammenhang mit der vorliegenden Erfindung Partikel mit einer mittleren Partikelgröße von 400 nm bis 10 μm verstanden. Vorzugsweise ist die mittlere Partikelgröße kleiner als 5 μm. In eigenen Untersuchungen hat sich gezeigt, dass die mit den Mikropartikeln bereits erreichbare Volumenfüllung der Lackzusammensetzung umso vollständiger und gleichmäßiger ist, je kleiner die Mikropartikel sind.

Die Füllstoffkomponente (c) einer erfindungsgemäßen Lackzusammensetzung zur Anwendung in einem therapeutischen Verfahren als Dentalmaterial kann einen Füllstoff (c2) in einer Menge von 0 bis 10 Gew.-% umfassen, bezogen auf die Gesamtmasse der Lackzusammensetzung.

Die Mikropartikel der Komponente (c2) können eine monomodale oder polymodale, beispielsweise eine bimodale Partikelgrößenverteilung aufweisen. Mikropartikel mit einer bimodale oder multimodalen Partikelgrößenverteilung sind erfindungsgemäß bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bi- oder multimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Hohlräume zwischen den Partikeln der Fraktionen mit der größeren Partikelgröße ausfüllen werden.

Bevorzugt wird somit in einer erfindungsgemäßen Lackzusammensetzung zur Anwendung in einem therapeutischen Verfahren als Dentalmaterial eine Komponente (c2) eingesetzt, welche zwei oder mehr Fraktionen von Mikropartikeln enthält, wobei sich die mittleren Partikelgrößen der Fraktionen unterscheiden.

Vorzugsweise enthält Komponente (c2) zumindest zwei Mikropartikel-Fraktionen, wobei deren mittlere Partikelgrößen um mindestens 1 μm voneinander abweichen.

Die Mikropartikel verschiedener Fraktionen können aus dem gleichen oder aus verschiedenen Materialien bestehen; es können dabei auch mehrere Fraktionen von Mikropartikeln vorliegen, deren mittlere Partikelgröße annähernd gleich ist oder in einem bestimmten Bereich liegt, wobei die Materialien der Partikel sich zwischen den Fraktionen unterscheiden.

Bevorzugt umfasst eine erfindungsgemäße Lackzusammensetzung zur Anwendung in einem therapeutischen Verfahren als Dentalmaterial eine Komponente (c2), welche eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm (d.h. größer als 0,4 μm, aber kleiner als 1 μm), vorzugsweise 0,5 μm bis 0,8 μm, besitzen.

Bevorzugt liegt das Verhältnis der Gesamtmasse der ersten Mikropartikelfraktionen zur Gesamtmasse der zweiten Mikropartikelfraktionen im Bereich von 1 : 1 bis 10 : 1, vorzugsweise im Bereich von 1,5 : 1 bis 5 : 1.

Bevorzugt liegt das Verhältnis der mittleren Korngröße der oder einer ersten Mikropartikelfraktion zur mittleren Korngröße der oder einer zweiten Mikropartikelfraktion der Komponente (c2) im Bereich von 1,5 : 1 bis 10 : 1, vorzugsweise im Bereich von 2: 1 bis 5 : 1.

In einer besonders bevorzugten erfindungsgemäßen Lackzusammensetzung zur Anwendung in einem therapeutischen Verfahren als Dentalmaterial umfasst die Komponente (c2) eine oder mehrere erste Mikropartikelfraktionen, die jeweils eine mittlere Partikelgröße im Bereich von 1 μm bis 10 μm, vorzugsweise 1 μm bis 5 μm, besitzen, und eine oder mehrere zweite Mikropartikelfraktionen, die jeweils eine mittlere Partikelgröße im Bereich von > 0,4 μm bis < 1 μm, vorzugsweise 0,5 μm bis 0,8 μm, besitzen; wobei das Verhältnis der Gesamtmasse der ersten Mikropartikelfraktionen zur Gesamtmasse der zweiten Mikropartikelfraktionen im Bereich von 1 : 1 bis 10 : 1, vorzugsweise 1,5 : 1 bis 5 : 1 und/oder das Verhältnis der mittleren Korngröße der oder einer ersten Mikropartikelfraktion zur mittleren Korngröße der oder einer zweiten Mikropartikelfraktion der Komponente (c2) im Bereich von 1,5 : 1 bis 10 : 1, vorzugsweise 2 : 1 bis 5 : 1 liegt.

Die Basismaterialien für die in oberflächenmodifizierter Form einzusetzenden Mikropartikel sind bevorzugt ausgewählt aus der Gruppe bestehend aus amorphen Materialien auf der Basis von $SiO_2$ $ZrO_2$ und/oder $TiO_2$, sowie Mischoxiden, pyrogener Kieselsäure oder Fällungskieselsäure, wie Quarz-Glaskeramik oder Glaspulver (insbesondere Dentalglaspulver), Barium- oder Strontiumgläser, Fluoridionen abgebende Gläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

Zur besseren Einbindung in die Polymermatrix einer erfindungsgemäßen Lackzusammensetzung zur Anwendung in einem therapeutischen Verfahren als Dentalmaterial sind die Mikropartikel vorzugsweise organisch oberflächenmodifiziert. Beispielhaft genannt sei die Oberflächenbehandlung der Füllstoffe mit einem Silan, die zu silanisierten Mikropartikeln führt. Zur Oberflächenbehandlung (als Haftvermittler) eignet sich besonders Methacryloxypropyltrimethoxysilan.

In einer besonders bevorzugten erfindungsgemäßen Lackzusammensetzung zur Anwendung in einem therapeutischen Verfahren als Dentalmaterial wird zumindest ein Teil der Mikropartikel der Komponente (c2) durch organisch oberflächenmodifizierte Partikel, vorzugsweise silanisierte Partikel gebildet und/oder es wird zumindest ein Teil der

Mikropartikel der Komponente (c2) durch Dentalglas-Partikel gebildet; vorzugsweise sind zumindest ein Teil der Mikropartikel der Komponente (c2) organisch oberflächenmodifizierte Dentalglas-Partikel, vorzugsweise silanisierte Dentalglas-Partikel.

Bevorzugt zeichnet sich in diesen Fällen Komponente (c2) durch eine bi- oder multimodale Partikelgrößenverteilung aus, insbesondere eine bi- oder multimodale Partikelgrößenverteilung mit den oben beschriebenen bevorzugten Merkmalen.

Komponente (c3) - Weitere Füllstoffe

Die Füllstoffkomponente (c) einer erfindungsgemäßen Lackzusammensetzung zur Anwendung in einem therapeutischen Verfahren als Dentalmaterial kann ferner einen Füllstoff (c3) in einer Menge von 0 bis 15 Gew.-%, vorzugsweise in einer Menge von 0 bis 10 Gew.-%, bevorzugt in einer Menge von 0 bis 5 Gew.-%, bevorzugt in einer Menge von 0 bis 2,5 Gew.-%, umfassen, jeweils bezogen auf die Gesamtmasse der Lackzusammensetzung.

Diese oben angegebenen Mengenbereiche gelten insbesondere für manche Füllstoffe aus agglomerierten Partikeln, da solche Füllstoffe zu einer unerwünschten hohen Viskosität der Lackzusammensetzung führen können, so dass die Lackzusammensetzung deutlich schlechter oder gar nicht mehr für die genannten Einsatzzwecke geeignet ist.

So können z.B. verstärkend wirkende Füllstoff-Materialien, wie Glasfasern, Polyamidoder Kohlenstofffasern eingesetzt werden. Eine erfindungsgemäße Lackzusammensetzung zur Anwendung in einem therapeutischen Verfahren als Dentalmaterial kann auch feinteilige Splitter oder Perlpolymerisate enthalten, wobei die Perlpolymerisate Homo- oder Copolymere organischer härtbarer Monomerer sein können.

Bestandteil (d): optionale weitere Additive

Eine erfindungsgemäße Lackzusammensetzung zur Anwendung in einem therapeutischen Verfahren als Dentalmaterial umfasst in manchen Fällen ein oder mehrere weitere Additive.

Diese Additive können verschiedene Funktionen haben. Übliche Additive für den Einsatz in dentalen Lackzusammensetzungen sind dem Fachmann bekannt, je nach gewünschter Funktion wird er das oder die geeigneten Additive auswählen. Beispielhaft sind im folgenden typische Additive und ihre Funktionen beschrieben.

Lichthärtbare dentale Lackzusammensetzungen, wie sie erfindungsgemäß bevorzugt sind, enthalten vorzugsweise einen oder mehrere Inhibitoren, auch Stabilisatoren genannt. Diese werden üblicherweise zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie und tert.-Butylhydroxyanisol (BHA), 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Eine erfindungsgemäß bevorzugte dentale Lackzusammensetzung zur Anwendung in einem therapeutischen Verfahren als Dentalmaterial umfasst somit als Additiv ein oder mehrere Polymerisationsinhibitoren zur Erhöhung der Lagerstabilität der Lackzusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Eine erfindungsgemäß bevorzugte dentale Lackzusammensetzung zur Anwendung in einem therapeutischen Verfahren als Dentalmaterial umfasst als Additiv eine oder mehrere fluoridabgebende Substanzen, dabei vorzugsweise Natriumfluorid und/oder Aminfluoride.

Ferner können ein oder mehrere Tenside Bestandteil einer erfindungsgemäßen (vorzugsweise dentalen) Lackzusammensetzun zur Anwendung in einem therapeutischen Verfahren als Dentalmaterial sein.

UV-Absorber, die beispielsweise durch ihre konjugierten Doppelbindungssytemen und aromatischen Ringe befähigt sind, UV Strahlung zu absorbieren, sind in manchen Fällen Bestandteil einer erfindungsgemäßen (vorzugsweise dentalen) Lackzusammensetzung zur Anwendung in einem therapeutischen Verfahren als Dentalmaterial. Beispiele an UV-Absorbern sind 2-Hydroxy-4-methoxybenzophenon, Salicylsäurephenylester 3-(2'-Hydroxy-5'-methylphenyl)-benzotriazol oder Diethyl-2,5-dihydroxy-terephthalat.

Da die Zähne möglichst naturgetreu wiederherzustellen sind ist es notwendig, erfindungsgemäße dentale Lackzusammensetzungen zur Anwendung in einem therapeutischen Verfahren als Dentalmaterial in den unterschiedlichsten Farbtönen bereitzustellen. Man benutzt zu diesem Zweck in der Regel anorganische Farbstoffe sowie organische Pigmente in sehr geringen Mengen, die somit in bevorzugten Ausgestaltungen als Additiv eingesetzt werden.

Weitere optionale Additive sind Aromastoffe.

Offenbart wird auch ein Verfahren zur Herstellung einer erfindungsgemäßen Lackzusammensetzung zur Anwendung in einem therapeutischen Verfahren als Dentalmaterial mit folgendem Schritt:

- Mischen der Bestandteile (a) und (b) sowie gegebenenfalls (c) und gegebenenfalls (d).

Beispiele

Anhand der folgenden Beispiele wird die Erfindung weiter erläutert. Sofern nicht anders angegeben, beziehen sich alle Angaben auf das Gewicht. Es werden dabei folgende Abkürzungen verwendet:

TCD-Monomer = Bis(methacryloyloxymethyl)tricyclo[5.2.1.$0^{2,6}$]decan

BHT = 2,6-Di-tert-butyl-4-methylphenol (Stabilisator)

Nano-$SiO_2$: es wurde nanoskalige Kieselsäure mit einer mittleren Partikelgröße von 50 nm eingesetzt

Aerosil: Aerosil A200 (Evonik)

Tensid: BYK307 (Byk-Chemie Gmbh)

KW = Kontaktwinkel auf trockenem Zahnschmelz

Herstellung der Lackzusammensetzungen

Erfindungsgemäße Lackzusammensetzungen zur Anwendung in einem therapeutischen Verfahren als Dentalmaterial sowie eine nicht erfindungsgemäße Lackzusammensetzung wurden jeweils wie folgt hergestellt:

Die Monomere, sowie Initiatoren und Additive werden zunächst in einem Kunststoffbehälter mittels eines KPG-Rührers homogenisiert. Anschließend werden gegebenenfalls die Füllstoffe zugegeben und mit einem Doppelplaneten-Mischer vermischt.

Messmethoden:

Biegefestigkeit: 3-Punkt Biegefestigkeit

Die Bestimmung der Biegefestigkeit wurde in Anlehnung an die ISO 4049 durchgeführt. In einer Form aus PTFE wurden Probekörper mit den Maßen $2 \times 2 \times 50$ mm hergestellt. Diese wurden anschließend für 60 Minuten in einem Lichtkasten polymerisiert. Nach der Entformung wurden die Probekörper bis zur Messung 24 Stunden bei 37°C in destilliertem Wasser gelagert. Die Messung der Biegefestigkeit erfolgte mit einer Universalprüfmaschine bei einer Traversengeschwindigkeit von 0,75 mm/min und einer Einspannlänge von 25 mm bis zum Bruch.

E-Modul:

Der E-Modul wurde aus den Messungen der Biegefestigkeiten durch Berechnung der Steigung im linearen Bereich ermittelt.

Wasseraufnahme:

Die Wasseraufnahme wurde analog zu ISO 4049 bestimmt. Dazu wurden das zu untersuchende Material luftblasenfrei in entsprechende Teflonformen eingefüllt, mit Folien und Glasplatten bedeckt und mit einer Schraubzwinge wurden die Überschüsse herausgepresst. Die Probekörper mit einem Durchmesser von $15{,}0 \pm 0{,}1$ mm und einer Höhe von $1{,}0 \pm 0{,}1$ mm wurden segmentweise lichtgehärtet. Anschließend wurden die Probekörper in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_1$, erreicht war.

Nach dem vollständigen Trocknen wurde der Durchmesser zweimal rechtwinklig zueinander mit einer Messgenauigkeit von 0,01 mm gemessen und daraus der mittlere Durchmesser berechnet. Die Dicke des Probekörpers wurde in der Mitte und an vier in gleichem Abstand liegenden Stellen des Randes auf 0,01 mm gemessen. Aus dem

mittleren Durchmesser und der mittleren Dicke wurde das Volumen, V, berechnet.

Anschließend wurden die Probekörper für 7 Tage in Wasser bei 37°C gelagert Danach wurden die Probekörper herausgenommen, mit Wasser abgespült und abgetupft, bis auf der Oberfläche keine Feuchtigkeit mehr sichtbar war. Die Probekörper wurden 15 s in der Luft hin- und hergeschwenkt und 1 min nach dem Herausnehmen aus dem Wasser gewogen. Diese Masse wird als $m_2$ angegeben.

Anschließend wurden die Probekörper erneut in einen Exsikkator bei 37°C aufbewahrt. Nach 22 Stunden wurden die Probekörper entnommen, für 2 Stunden in einen zweiten Exsikkator auf 23°C gebracht und dann auf 0,1 mg gewogen. Dieser Zyklus wurde so lange wiederholt, bis eine konstante Masse, $m_3$, erreicht war.

Die Wasseraufnahme, $W_{sp}$, wurde nach folgender Gleichung berechnet:

$$W_{sp} = \frac{m_2 - m_3}{V}$$

Dabei ist

$m_2$ die Masse des Probekörpers nach Wasserlagerung für 7 Tage in µg;
$m_3$ die Masse des wieder getrockneten Probekörpers in µg;
$V$ das Volumen des Probekörpers in $mm^3$

Anfließverhalten: Kontaktwinkel auf trockenem Zahnschmelz

Das Anfließverhalten wurde ermittelt durch Messung des Kontaktwinkels auf trockenem Zahnschmelz.

Für die Kontaktwinkelmessungen auf trockenem Zahnschmelz wurde ein extrahierter humaner Molar verwendet. Dieser wurde vor der Messung mit einem Zellstofftuch durch Abwischen getrocknet. Anschließend wurde ein Tropfen des zu untersuchenden Materials auf den Schmelzbereich des Zahns aufgetragen. Anschließend wurde der Kontaktwinkel über einen Zeitraum von 30 Sekunden mit einem Kontaktwinkelmessgerät (DSA 100, Firma Krüss) bestimmt.

Die Zusammensetzungen der Mischungen (in Gewichtsteilen) sowie die Ergebnisse der Messungen sind in den nachfolgenden Tabellen aufgelistet.

Tabelle 1:

| Beispiel Nr. | 1 | 2 | 3 | Vergleich |
|---|---|---|---|---|
| Phosphinoxidinitiator | 3,970 | 3,970 | 3,970 | 4,130 |
| UV-Stabilisator | 0,029 | 0,029 | 0,029 | 0,029 |
| BHT | 0,016 | 0,016 | 0,016 | 0,017 |
| Aerosil | 0,760 | 0,760 | 0,760 | 0,840 |
| Tensid | 0,760 | 0,760 | 0,760 | 0,840 |
| Methylmethacrylat | 20,14 | 20,16 | 35,13 | 30,51 |
| Dipentaerythritolpentaacrylat | 44,32 | 59,30 | 44,33 | 63,63 |
| TCD-Monomer | 30,00 | 15,00 | 15,00 | 0,00 |
| Nano-$SiO_2$ | 0,00 | 0,00 | 0,00 | 0,00 |
| | | | | |
| Biegefestigkeit [MPa] | 85 | 65 | 94 | 54 |
| E-Modul [MPa] | 2846 | 2730 | 2656 | 2300 |
| KW [°] trockener Zahnschmelz | 31,9 | 30,2 | 19,8 | 30,8 |
| Wasseraufnahme [$\mu g/mm^3$] | 34,80 | 39,64 | 42,19 | 49,12 |

Tabelle 2:

| Beispiel Nr. | 4 Vergleich | 5 Vergleich | 6 | 7 |
|---|---|---|---|---|
| Phosphinoxidinitiator | 3,970 | 3,970 | 1,998 | 1,998 |
| UV-Stabilisator | 0,030 | 0,030 | 0,029 | 0,029 |
| BHT | 0,017 | 0,017 | 0,017 | 0,017 |
| Aerosil | 0,764 | 0,764 | 0,000 | 0,000 |
| Tensid | 0,764 | 0,769 | 0,000 | 0,000 |
| Methylmethacrylat | 42,30 | 20,00 | 9,52 | 9, 52 |
| Dipentaerythritolpentaacrylat | 0,00 | 0,00 | 9,52 | 9,52 |
| TCD-Monomer | 52,15 | 74,45 | 19,05 | 0,00 |
| Monomer der Formel (2) | 0,00 | 0,00 | 0,00 | 19,05 |
| Nano-$SiO_2$ | 0,00 | 0,00 | 59,87 | 59,87 |
| | | | | |
| Biegefestigkeit [MPa] | 86 | 70 | 109 | 112 |
| E-Modul [MPa] | 2917 | 3178 | 3670 | 3735 |
| KW [°] trockener Zahnschmelz | | | 27,4 | 25,6 |
| Wasseraufnahme [$\mu g/mm^3$] | | | 35,13 | 32,17 |

Synthese der Verbindung der Formel (2)

0,95 g (4,84 mmol) 3(4), 8(9)-Bis(hydroxymethyl)tricyclo[5.2.1.$0^{2,6}$]decan wurden in 10 mL Toluol gelöst und mit 0,04 g BHT und 0,103 g der Katalysatorlösung (0,50 g Dibutylzinn(II)dilaurat gelöst in 9,50 g Toluol) versetzt. Unter Rühren wurden 3,00 g (19,34 mmol, 4 Äquivalente) Isocyanatoethylmethacrylat, gelöst in 10 mL Toluol, zugetropft. Nach beendeter Zugabe wurde der Tropftrichter durch einen Rückflusskühler ausgetauscht und das Reaktionsgemisch auf 120 °C erwärmt und der Fortschritt der Reaktion mittels IR-Spektroskopie verfolgt. Nach 72 Stunden wurden weitere 0,102 g Katalysatorlösung zugesetzt und weiter erhitzt, bis keine Isocyanatbande mehr detektiert wurde. Das Lösungsmittel wurde am Rotationsverdampfer entfernt. Das Allophanat der Formel (2) wurde in einer Ausbeute von 3,83 g (4,69 mmol, 97%) als leicht gelbliches Öl erhalten.

Die Beispiele 1 bis 6 wurden unter Austausch des dort jeweils eingesetzten TCD-Monomeren gegen die Verbindung der Formel (2) wiederholt; diese weiteren Beispiele werden als Beispiele S1 bis S6 bezeichnet. Es wurden sämtliche in den Beispielen 1 bis 6 bestimmten Parameter auch für die Beispiele S1 bis S6 bestimmt. Die bestimmten Parameterwerte für die Beispiele S1 bis S6 sind ähnlich zu denen aus den Beispielen 1 bis 6 und übertreffen diese teilweise. Dies zeigt neben Beispiel 7, dass die Verbindung der Formel (2), welche repräsentativ für die erfindungsgemäß einzusetzenden Verbindungen steht, hervorragend für den Einsatz in effindungsgemäß definierten Kompositmaterialien geeignet ist.

## Patentansprüche

**1.** Lackzusammensetzung zur Anwendung in einem therapeutischen Verfahren als Dentalmaterial bestehend aus oder umfassend:

(a) 35 bis 99 Gew.-%, bezogen auf die Gesamtmasse der Lackzusammensetzung, einer Monomerenkomponente umfassend

(a1) ein, zwei oder mehr Monomere ausgewählt aus der Gruppe bestehend aus Verbindungen der Struktur $Q(Y_xZ_e)_b$, wobei gilt:

Q bedeutet ein gesättigtes oder olefinisch ungesättigtes polyalicyclisches Strukturelement ausgewählt

aus der Gruppe bestehend aus tricyclischen Kohlenwasserstoffresten, wobei optional ein, zwei oder mehr der nicht durch Substituenten $Y_xZ_e$ substituierten Wasserstoffatome dieses polyalicyclischen Strukturelements Q durch Alkylgruppen , Alkoxygruppen, Halogenatome oder Trifluormethylgruppen substituiert sind,

- b ist eine natürliche Zahl ausgewählt aus der Gruppe der natürlichen Zahlen 1, 2, 3 und 4,
- jedes Z bedeutet ein Strukturelement, das unabhängig von etwaigen weiteren Strukturelementen Z ausgewählt ist aus der Gruppe bestehend aus

  $-O-(C=O)-CH=CH_2$, $-O-(C=O)-C(CH_3)=CH_2$,
  $-(C=O)-CH=CH_2$, $-(C=O)-C(CH_3)=CH_2$,
  $-CH=CH_2$, $-C(CH_3)=CH_2$ und $-O-CH=CH_2$,

- jeder Index e ist eine natürliche Zahl, die unabhängig von etwaigen weiteren Indizes e ausgewählt ist aus der Gruppe der natürlichen Zahlen 1, 2, 3 und 4,
- jeder Index x bedeutet unabhängig von etwaigen weiteren Indizes x 0 oder 1,
- jedes Y bedeutet in der Struktur $Q(Y_xZ_e)_b$ bei x = 1 ein Strukturelement, welches das polyalicyclische Strukturelement Q mit e Strukturelementen Z verbindet, wobei jedes Y unabhängig von etwaigen weiteren Strukturelementen Y gewählt ist,

(a2) ein, zwei oder mehr radikalisch polymerisierbare Monomere ausgewählt aus der Gruppe bestehend aus Methylmethacrylat, Ethylmethacrylat, Propylmethacrylat, Butylmethacrylat und Pentylmethacrylat, wobei Komponente (a2) Methylmethacrylat umfasst oder aus Methylmethacrylat besteht, wobei die Gesamtmenge der Komponente (a2) im Bereich von 5 bis 45 Gew.-% liegt bezogen auf die Gesamtmasse der Lackzusammensetzung,
(a3) ein, zwei oder mehr weitere radikalisch polymerisierbare Monomere ausgewählt aus der Gruppe bestehend aus Monomeren mit drei oder mehr (Meth)acrylatgruppen, vorzugsweise mit drei bis sechs (Meth)acrylatgruppen, wobei die weiteren radikalisch polymerisierbaren Monomere der Komponente (a3) keine Monomere gemäß der Definition zu (a1) sind,
sowie
(a4) optional ein, zwei oder mehr weitere radikalisch polymerisierbare Monomere aus der Gruppe bestehend aus Acrylaten und Methacrylaten, vorzugsweise der Gruppe der Methacrylate, wobei die weiteren radikalisch polymerisierbaren Monomere der Komponente (a4) keine Monomere gemäß den Definitionen zu (a1), (a2) oder (a3) sind,

und
(b) ein oder mehrere Initiatoren und/oder Katalysatoren sowie
(c) gegebenenfalls eine Füllstoffkomponente umfassend einen, zwei oder mehrere Füllstoffe,
(d) gegebenenfalls ein oder mehrere sonstige Additive.

2. Lackzusammensetzung nach Anspruch 1, **dadurch gekennzeichnet, dass** das Strukturelement Q der Verbindungen der Struktur $Q(Y_xZ_e)_b$ der Komponente (a1) einen Tricyclo[5.2.1.0$^{2,6}$]decan-Rest oder einen Tricyclo[3.3.1.1$^{3,7}$]decan-Rest bedeutet.

3. Lackzusammensetzung nach Anspruch 1 oder 2, **dadurch gekennzeichnet, dass** ein, zwei oder mehr Verbindungen der Struktur $Q(Y_xZ_e)_b$ ein Tricyclo[5.2.1.0$^{2,6}$]-decan- Strukturelement aufweisen und Z vorzugsweise ausgewählt ist aus der Gruppe bestehend aus $-O-(C=O)-CH=CH_2$ und $-O-(C=O)-C(CH_3)=CH_2$, bevorzugt bedeutet Z die Gruppe $-O-(C=O)-C(CH_3)=CH_2$.

4. Lackzusammensetzung nach einem der vorangehenden Ansprüche, wobei die Gesamtmenge an Komponente (a1) und Komponente (a2) zumindest 25 Gew.-% beträgt, bezogen auf die Gesamtmasse der Lackzusammensetzung.

5. Lackzusammensetzung nach einem der vorangehenden Ansprüche, wobei das Verhältnis der Masse der Komponente (a1) zur Gesamtmasse der Komponenten (a2), (a3) und (a4) vorzugsweise im Bereich von 4 : 1 bis 1 : 6 liegt.

6. Lackzusammensetzung nach einem der vorangehenden Ansprüche, wobei Monomerenkomponente (a) umfasst

(a1) ein, zwei oder mehr Monomere der Struktur $Q(Y_xZ_e)_b$ der Komponente (a1), wobei vorzugsweise Z eine

Gruppe $-O-(C=O)-CH=CH_2$, $-O-(C=O)-C(CH_3)=CH_2$, $-(C=O)-CH=CH_2$ oder $-(C=O)-C(CH_3)=CH_2$ bedeutet,
(a2) ein, zwei oder mehr radikalisch polymerisierbare Monomere aus der Gruppe bestehend aus Methylmethacrylat, Ethylmethacrylat, Propylmethacrylat, Butylmethacrylat und Pentylmethacrylat, wobei Komponente (a2) Methylmethacrylat umfasst oder aus Methylmethacrylat besteht,
(a3) ein, zwei oder mehr weitere radikalisch polymerisierbare Monomere mit drei oder mehr (Meth)acrylatgruppen, vorzugsweise mit drei bis sechs (Meth)acrylatgruppen, wobei die weiteren radikalisch polymerisierbaren Monomere der Komponente (a3) keine Monomere gemäß der Definition zu (a1) sind,
sowie
(a4) optional ein, zwei oder mehr weitere radikalisch polymerisierbare Monomere aus der Gruppe bestehend aus Acrylaten und Methacrylaten, vorzugsweise der Gruppe der Methacrylate wobei die weiteren radikalisch polymerisierbaren Monomere der Komponente (a4) keine Monomere gemäß den Definitionen zu (a1), (a2) oder (a3) sind.

7. Lackzusammensetzung nach einem der vorangehenden Ansprüche, **dadurch gekennzeichnet, dass** Komponente (a3) Dipentaerythritolpentaacrylat umfasst oder aus Dipentaerythritolpentaacrylat besteht.

8. Lackzusammensetzung nach einem der vorangehenden Ansprüche, **dadurch gekennzeichnet, dass** Komponente (a1) umfasst oder besteht aus Bis(methacryloyloxymethyl)tricyclo[5.2.1.$0^{2,6}$]decan und/oder Bis(acryloyloxymethyl)tricyclo [5.2.1.$0^{2,6}$]decan.

9. Lackzusammensetzung nach einem der vorangehenden Ansprüche, **dadurch gekennzeichnet, dass** sie lichthärtbar und/oder chemisch härtbar ist.

10. Lackzusammensetzung nach einem der vorangehenden Ansprüche, **dadurch gekennzeichnet, dass** Komponente

(b) der Lackzusammensetzung einen oder mehrere Initiatoren aus der Gruppe der Phosphinoxide, insbesondere der Acylphosphinoxide, enthält,
und/oder
(c) 0 bis 60 Gew.-% einer Füllstoffkomponente, umfassend einen, zwei oder mehrere Füllstoffe enthält.

11. Lackzusammensetzung nach einem der vorangehenden Ansprüche, **dadurch gekennzeichnet, dass** die Lackzusammensetzung transparent ist.

12. Lackzusammensetzung nach einem der vorangehenden Ansprüche, **dadurch gekennzeichnet, dass** die Wasseraufnahme der Lackzusammensetzung im ausgehärteten Zustand weniger als 50 $\mu g/mm^3$ beträgt, vorzugsweise weniger als 45 $\mu g/mm^3$ und bevorzugt weniger als 40 $\mu g/mm^3$, besonders bevorzugt weniger als 35 $\mu g/mm^3$.

13. Lackzusammensetzung nach einem der Ansprüche 1 bis 12 zur Anwendung in einem therapeutischen Verfahren als Dentalmaterial als Kavitätenund/oder Oberflächenlack, als Unterfüllung für Zahnfüllmaterialien, zum Schutz des Zahnschmelzes, zur Verhinderung von Karies, zum Schutz einer Restauration (insbesondere gegen vorzeitige Abnutzung), zur Verbesserung der Verschleissfestigkeit eines dentalen Füllungsmaterials, zur Festigung restaurierter Oberflächen, zum Schutz vor Abrasion und/oder Verfärbung eines Zahns oder einer Restauration, zum Schließen marginaler Spalten und/oder von Mikrorissen, zur Glättung von restaurativen Oberflächen, zum Verleihen von natürlichem Glanz auf einem Zahn oder einer Restauration und zum Reduzieren von Farbstoffablagerungen auf einem Zahn oder einer Restauration.

## Claims

1. Lacquer composition for use in a therapeutic method as a dental material consisting of or comprising:

(a) 35 to 99 wt.%, based on the total weight of the lacquer composition, of a monomer component comprising

(a1) one, two or more monomers selected from the group consisting of compounds of the structure $Q(Y_xZ_e)_b$, wherein:

Q denotes a saturated or olefinically unsaturated polyalicyclic structure element selected from the group consisting of tricyclic hydrocarbon radicals, wherein optionally one, two or more of the hydrogen atoms

of this polyalicyclic structure element Q which are not substituted by substituents $Y_xZ_e$ are substituted by alkyl groups, alkoxy groups, halogen atoms or trifluoromethyl groups,

- b is a positive integer selected from the group of positive integers 1, 2, 3 and 4,
- each Z denotes a structure element which, independently of any further structure elements Z, is selected from the group consisting of

$-O-(C=O)-CH=CH_2$, $-O-(C=O)-C(CH_3)=CH_2$,
$-(C=O)-CH=CH_2$, $-(C=O)-C(CH_3)=CH_2$,
$-CH=CH_2$, $-C(CH_3)=CH_2$ and $-O-CH=CH_2$,

- each index e is a positive integer which, independently of any further indices e, is selected from the group of positive integers 1, 2, 3 and 4,
- each index x, independently of any further indices x, denotes 0 or 1,
- each Y in the structure $Q(Y_xZ_e)_b$ where x = 1 denotes a structure element which connects the polyalicyclic structure element Q to e structure elements Z, wherein each Y is selected independently of any further structure elements Y,

(a2) one, two or more monomers which can be polymerised by free radicals, selected from the group consisting of methyl methacrylate, ethyl methacrylate, propyl methacrylate, butyl methacrylate and pentyl methacrylate, wherein component (a2) comprises methyl methacrylate or consists of methyl methacrylate, wherein the total amount of component (a2) is in the range of from 5 to 45 wt.%, based on the total weight of the lacquer composition,
(a3) one, two or more further monomers which can be polymerised by free radicals, selected from the group consisting of monomers having three or more (meth)acrylate groups, preferably having three to six (meth)acrylate groups, wherein the further monomers of component (a3) which can be polymerised by free radicals are not monomers according to the definition of (a1),
and
(a4) optionally one, two or more further monomers which can be polymerised by free radicals, from the group consisting of acrylates and methacrylates, preferably the group of methacrylates, wherein the further monomers of component (a4) which can be polymerised by free radicals are not monomers according to the definition of (a1), (a2) or (a3),

and
(b) one or more initiators and/or catalysts
and
(c) optionally a filler component comprising one, two or more fillers,
(d) optionally one or more other additives.

2. Lacquer composition according to claim 1, **characterised in that** the structure element Q of the compounds of the structure $Q(Y_xZ_e)_b$ of component (a1) denotes a tricyclo[5.2.1.0$^{2,6}$]decane radical or a tricyclo[3.3.1.1$^{3,7}$]decane radical.

3. Lacquer composition according to claim 1 or 2, **characterised in that** one, two or more compounds of the structure $Q(Y_xZ_e)_b$ have a tricyclo[5.2.1.0$^{2,6}$]decane structure element and Z is preferably selected from the group consisting of $-O(C=O)-CH=CH_2$ and $-O-(C=O)-C(CH_3)=CH_2$, preferably Z denotes the group $-O-(C=O)-C(CH_3)=CH_2$.

4. Lacquer composition according to one of the preceding claims, wherein the total amount of component (a1) and component (a2) is at least 25 wt.%, based on the total weight of the lacquer composition.

5. Lacquer composition according to one of the preceding claims, wherein the ratio of the weight of component (a1) to the total weight of components (a2), (a3) and (a4) is preferably in the range of from 4 : 1 to 1 : 6.

6. Lacquer composition according to one of the preceding claims, wherein monomer component (a) comprises

(a1) one, two or more monomers of the structure $Q(Y_xZ_e)_b$ of component (a1), wherein Z preferably denotes a group $-O-(C=O)-CH=CH_2$, $-O-(C=O)-C(CH_3)=CH_2$, $-(C=O)-CH=CH_2$ or $-(C=O)-C(CH_3)=CH_2$,
(a2) one, two or more monomers which can be polymerised by free radicals, from the group consisting of methyl

methacrylate, ethyl methacrylate, propyl methacrylate, butyl methacrylate and pentyl methacrylate, wherein component (a2) comprises methyl methacrylate or consists of methyl methacrylate,

(a3) one, two or more further monomers which can be polymerised by free radicals and having three or more (meth)acrylate groups, preferably having three to six (meth)acrylate groups, wherein the further monomers of component (a3) which can be polymerised by free radicals are not monomers according to the definition of (a1), and

(a4) optionally one, two or more further monomers which can be polymerised by free radicals, from the group consisting of acrylates and methacrylates, preferably the group of methacrylates, wherein the further monomers of component (a4) which can be polymerised by free radicals are not monomers according to the definitions of (a1), (a2) or (a3).

**7.** Lacquer composition according to one of the preceding claims, **characterised in that** component (a3) comprises dipentaerythritol pentaacrylate or consists of dipentaerythritol pentaacrylate.

**8.** Lacquer composition according to one of the preceding claims, **characterised in that** component (a1) comprises or consists of bis(methacryloyloxymethyl)tricyclo[5.2.1.0$^{2,6}$]decane and/or bis(acryloyloxymethyl)tricyclo[5.2.1.0$^{2,6}$]decane.

**9.** Lacquer composition according to one of the preceding claims, **characterised in that** it is photocurable and/or chemically curable.

**10.** Lacquer composition according to one of the preceding claims, **characterised in that** component

(b) of the lacquer composition contains one or more initiators from the group of phosphine oxides, in particular acylphosphine oxides,
and/or
(c) contains 0 to 60 wt.% of a filler component comprising one, two or more fillers.

**11.** Lacquer composition according to one of the preceding claims, **characterised in that** the lacquer composition is transparent.

**12.** Lacquer composition according to one of the preceding claims, **characterised in that** the water uptake of the lacquer composition in the cured state is less than 50 $\mu$g/mm$^3$, by preference less than 45 $\mu$g/mm$^3$ and preferably less than 40 $\mu$g/mm$^3$, particularly preferably less than 35 $\mu$g/mm$^3$.

**13.** Lacquer composition according to one of claims 1 to 12 for use in a therapeutic method as a dental material as a cavity and/or surface lacquer, as an underfilling for dental filling materials, to protect the tooth enamel, to prevent caries, to protect a restoration (in particular against premature wear), to improve the wear resistance of a dental filling material, to strengthen restored surfaces, to protect against abrasion and/or discoloration of a tooth or a restoration, to close marginal gaps and/or microcracks, to smooth restorative surfaces, to impart natural gloss on a tooth or a restoration and to reduce colouring agent deposits on a tooth or a restoration.

## Revendications

**1.** Composition de laque pour une utilisation dans un procédé thérapeutique comme matériau dentaire consistant en ou comprenant :

(a) 35 à 99 % en poids par rapport à la masse totale de la composition de laque, d’un composant de monomères comprenant

(a1) un, deux ou plusieurs monomères choisis dans le groupe consistant en composés de structure Q $(Y_xZ_e)_b$, dans laquelle :

Q désigne un élément structurel polyalicyclique saturé ou oléfiniquement insaturé choisi dans le groupe consistant en radicaux hydrocarbure tricycliques, dans lequel éventuellement, un, deux ou plusieurs des atomes d’hydrogène non substitués par les substituants $Y_xZ_e$ de cet élément structurel polyalicyclique Q sont substitués par des groupes alkyle, des groupes alcoxy, des atomes d’halogène ou des

groupes trifluorométhyle,

- b est un nombre entier choisi dans le groupe des nombres entiers 1, 2, 3 et 4,
- chaque Z désigne un élément structurel qui est choisi indépendamment parmi d'autres éléments structurels Z éventuels dans le groupe consistant en

$-O-(C=O)-CH=CH_2$, $-O-(C=O)-C(CH_3)=CH_2$,
$-(C=O)-CH=CH_2$, $-(C=O)-C(CH_3)=CH_2$,
$-CH=CH_2$, $-C(CH_3)=CH_2$ et $-O-CH=CH_2$,

- chaque indice e est un nombre entier qui est choisi indépendamment parmi d'autres indices e éventuels dans le groupe des nombres entiers 1, 2, 3 et 4,
- chaque indice x désigne, indépendamment d'autres indices x éventuels, 0 ou 1,
- chaque Y désigne dans la structure $Q(Y_xZ_e)_b$, pour x = 1, un élément structurel qui relie l'élément structurel polyalicyclique Q avec e éléments structurels Z, dans laquelle chaque Y est choisi indépendamment parmi d'autres éléments structurels Y éventuels,

(a2) un, deux ou plusieurs monomères polymérisables au niveau radicalaire, choisis dans le groupe consistant en méthylméthacrylate, éthylméthacrylate, propylméthacrylate, butylméthacrylate et pentylméthacrylate, dans laquelle le composant (a2) comprend du méthylméthacrylate ou consiste en du méthylméthacrylate, dans laquelle la quantité totale du composant (a2) se situe dans la plage de 5 à 45 % en poids par rapport à la masse totale de la composition de laque,
(a3) un, deux ou plusieurs autres monomères polymérisables au niveau radicalaire, choisis dans le groupe consistant en monomères avec trois groupes (méth)acrylate ou plus, de préférence avec trois à six groupes (méth)acrylate, dans laquelle les autres monomères polymérisables au niveau radicalaire du composant (a3) ne sont pas des monomères selon la définition de (a1),
ainsi que
(a4) éventuellement, un, deux ou plusieurs autres monomères polymérisables au niveau radicalaire, du groupe consistant en acrylates et méthacrylates, de préférence du groupe des méthacrylates, dans laquelle les autres monomères polymérisables au niveau radicalaire du composant (a4) ne sont pas des monomères selon les définitions de (a1), (a2) ou (a3),

et
(b) un ou plusieurs initiateurs et/ou catalyseurs ainsi que
(c) éventuellement un composant de charge comprenant un, deux ou plusieurs agents de charge,
(d) éventuellement un ou plusieurs autres additifs.

2. Composition de laque selon la revendication 1, **caractérisée en ce que** l'élément structurel Q des composés de structure $Q(Y_xZ_e)_b$ du composant (a1) désigne un radical tricyclo[5.2.1.0$^{2,6}$]-décane ou un radical tricyclo [3.3.1.1$^{3,7}$]décane.

3. Composition de laque selon la revendication 1 ou 2, **caractérisé en ce qu'**un, deux ou plusieurs composants de structure $Q(Y_xZ_e)_b$ présentent un élément structurel tricyclo[5.2.1.0$^{2,6}$] et Z est choisi de préférence dans le groupe consistant en $-O-(C=O)-CH=CH_2$ et $-O-(C=O)-C(CH_3)=CH_2$, de préférence Z désigne le groupe $-O-(C=O)-C(CH_3)=CH_2$.

4. Composition de laque selon l'une des revendications précédentes, dans laquelle la quantité totale de composant (a1) et de composant (a2) est d'au moins 25 % en poids par rapport à la masse totale de la composition de laque.

5. Composition de laque selon l'une des revendications précédentes, dans laquelle le rapport de la masse du composant (a1) à la masse totale des composants (a2), (a3) et (a4) se situe de préférence dans la plage de 4:1 à 1:6.

6. Composition de laque selon l'une des revendications précédentes, dans laquelle le composant de monomères (a) comprend

(a1) un, deux ou plusieurs monomères de structure $Q(Y_xZ_e)_b$ du composant (a1), dans laquelle de préférence Z désigne un groupe $-O-(C=O)-CH=CH_2$, $-O-(C=O)-C(CH_3)=CH_2$, $-(C=O)-CH=CH_2$, ou $-(C=O)-C(CH_3)=CH_2$,
(a2) un, deux ou plusieurs monomères polymérisables au niveau radicalaire, du groupe consistant en méthylméthacrylate, éthylméthacrylate, propylméthacrylate, butylméthacrylate et pentylméthacrylate, dans laquelle le composant (a2) comprend du méthylméthacrylate ou consiste en méthylméthacrylate,
(a3) un, deux ou plusieurs autres monomères polymérisables au niveau radicalaire comportant trois ou plusieurs groupes (méth)acrylate, de préférence trois à six groupes (méth)acrylate, dans laquelle les autres monomères polymérisables au niveau radicalaire du composant (a3) ne sont pas des monomères selon la définition de (a1), ainsi que
(a4) éventuellement, un, deux ou plusieurs autres monomères polymérisables au niveau radicalaire, du groupe consistant en acrylates et méthacrylates, de préférence du groupe des méthacrylates, dans laquelle les autres monomères polymérisables au niveau radicalaire du composant (a4) ne sont pas des monomères selon les définitions de (a1), (a2) ou (a3).

**7.** Composition de laque selon l'une des revendications précédentes, **caractérisée en ce que** le composant (a3) comprend le dipenta-érythritolpenta-acrylate ou consiste en dipenta-érythritolpenta-acrylate.

**8.** Composition de laque selon l'une des revendications précédentes, **caractérisée en ce que** le composant (a1) comprend ou consiste en bis(méthacryloyloxyméthyl)tricyclo[5.2.1.0$^{2,6}$]décane et/ou en bis(acryloyloxyméthyl)tricyclo[5.2.1.0$^{2,6}$] décane.

**9.** Composition de laque selon l'une des revendications précédentes, **caractérisée en ce qu'**elle est photodurcissable et/ou durcissable chimiquement.

**10.** Composition de laque selon l'une des revendications précédentes, **caractérisée en ce que** le composant

(b) de la composition de laque contient un ou plusieurs initiateurs du groupe des oxydes de phosphine, en particulier des oxydes d'acylphosphine,
et/ou
(c) 0 à 60 % en poids d'un composant de charge comprenant un, deux ou plusieurs agents de charge.

**11.** Composition de laque selon l'une des revendications précédentes, **caractérisée en ce que** la composition de laque est transparente.

**12.** Composition de laque selon l'une des revendications précédentes, **caractérisée en ce que** l'absorption d'eau de la composition de laque à l'état durci est inférieure à 50 $\mu g/mm^3$, de préférence inférieure à 45 $\mu g/mm^3$ et de préférence, inférieure à 40 $\mu g/mm^3$, de manière particulièrement préférée, inférieure à 35 $\mu g/mm^3$.

**13.** Composition de laque selon l'une des revendications 1 à 12 pour l'utilisation dans un procédé thérapeutique en tant que matériau dentaire comme laque de cavités et/ou de surfaces, comme agent de comblement de base pour les matériaux de comblement dentaire, pour la protection de l'émail dentaire, pour empêcher des caries, pour la protection d'une restauration (en particulier contre une usure prématurée), pour l'amélioration de la résistance à l'usure d'un matériau de comblement dentaire, pour la fixation de surfaces restaurées, pour la protection contre l'abrasion et/ou la coloration d'une dent ou d'une restauration, pour la fermeture de fentes marginales et/ou de microfissures, pour le lissage de surfaces restaurées, pour conférer un brillant naturel à une dent ou une restauration et pour réduire les dépôts colorés sur une dent ou une restauration.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 4029910 A
- US 7081485 B2
- US 20050288387 A1
- US 20040034119 A1
- US 20060111465 A1
- EP 1532958 B1
- US 20070010597 A1
- DE 102007048925 A1
- DE 102008010464 A1
- DE 102009011537 A1
- DE 102009011536 A1
- DE 102008039129 A1
- US 6617413 B1
- EP 1307173 B1
- US 20060063853 A1
- EP 0023685 A2
- EP 1238993 A
- EP 0209700 A2
- DE 3522006
- DE 3522005
- EP 0000194 A1
- US 4160080 A
- EP 0682012 B1
- EP 1727846 B1
- EP 0712840 B1
- EP 0867457 B1
- DE 102007040240 A1
- EP 1645582 A1
- DE 102007040239 A1
- DE 102004060285 A1
- WO 2006063891 A1
- WO 03035013 A1
- DE 60216951 T2
- US 6670499 B1
- US 6670499 B
- JP 7206740 A
- EP 1112995 B1
- EP 0049631 B1
- DE 10352260 B3
- EP 0023686 B1
- DE 3522006 A1
- DE 3338077 A1
- EP 0143613 B1
- DE 3703120 A1
- WO 2009065873 A2
- DE 3941629 A1
- DE 19903177
- DE 4416857
- EP 0980682 A1
- EP 0948955 A1
- DE 102006019092 A1
- DE 3941629 C2
- DE 102006019092
- DE 60116142
- DE 3801511 C2
- DE 102006050153 A1
- EP 0184095 B1
- DE 4231579 C2
- EP 0366977 B1
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- US 4772530 A
- US 4954414 A
- US 4874450 A
- US 5055372 A
- US 5057393 A
- EP 1720506 A
- EP 1839640 A
- DE 1495520
- WO 02092021 A
- WO 02092023 A
- EP 0059451 A
- WO 0130307 A1
- WO 2007028159 A2
- EP 0783880 B1
- DE 10119831 A1
- EP 1563821 A1